# EUROPEAN PATENT APPLICATION

(11) **EP 2 960 247 A1**
(43) Date of publication of application: **30.12.2015**
(21) Application number: 15170848.4
(22) Date of filing: 13.12.2010
(51) Int. Cl.: C07H 15/26, A61K 31/7034, A61P 31/04

(54) **PHENYL-ALPHA-D-MANNOSIDES FOR USE IN THE TREATMENT OF BACTERIAL INFECTIONS CAUSED BY ESCHERICHIA COLI**

(30) Priority: 14.12.2009 EP 09179007
(62) Divisional of application: 13158420.3
(71) Applicant: University of Basel, 4003 Basel (CH)
(72) Inventor: Ernst, Beat, 4312 Magden (CH); Herold, Janno, 4310 Rheinfelden (CH)
(74) Representative: Latscha Schöllhorn Partner AG

(57) **Abstract**

Compounds of the formula **(I)** wherein n is 0, 1 or 2, R² and R³ are hydrogen or a substituent as described in the specification, are useful for the prevention and treatment of bacterial infections, in particular of urinary infections caused by *E. coli,* 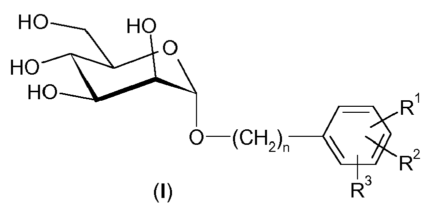 wherein R¹ is one of groups (B), (C), (D), (E) below:

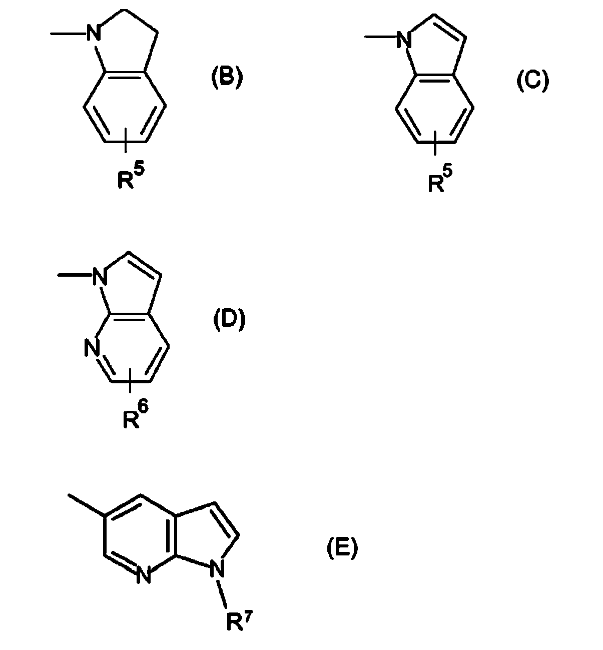

## Description

### Field of the invention

The invention relates to derivatives of α-D-mannopyranosides useful as antagonists of bacterial adhesion, and to their use in preventing and treating bacterial infections.

### Background of the invention

Urinary tract infection (UTI) is an inflammatory, pathogen-caused disease that occurs in any part of the urinary tract. UTI is characterized by a wide spectrum of symptoms ranging from mild irritative voiding (dysuria), frequent voiding (polakisuria) or suprapubic tenderness to invasion of bacteria into the kidney (acute pyelonephritis) or blood circulation (urosepsis) with potential local and distant bacterial seeding (abscess), multiorgan failure or even death (B. Foxman, Dis. Mon. 2003, 49, 53-70).

UTIs are among the most prevalent infectious diseases in general and of any organ system. Its magnitude can be estimated in the United States by the number of visits to physicians (about 8 million/year) or hospital discharge diagnoses (about 1.5 million/year). Particularly affected are women, who face a 40-50% risk experiencing a symptomatic UTI at some time during their life; more than half of them will experience consecutive infection within 6 months. In approximately 3-5% of women, multiple recurrences of UTI develop over the following years. Frequent sexual intercourse, diaphragm use and lack of urinating after sexual intercourse are risk factors for UTI, further increasing the prevalence of UTI in this subpopulation.

The predominant pathogen in UTIs is uropathogenic *Escherichia coli* (UPEC) causing >80% of all infections in otherwise healthy people with normal urinary tracts and no systemic predisposing factors (uncomplicated UTI). These strains express a number of well-studied virulence factors of UTI (e.g. fimbriae and toxins), which define tropism to and within the urinary tract, bacterial persistence and the degree of inflammation.

UTI can be described as an imbalance of "physiological inflammation", where both immune system and antimicrobial factors of the host are no longer able to control bacterial growth. In healthy individuals, most uropathogens originate from the rectal microbiota and enter the normally sterile urinary bladder via the urethra where they can trigger an infection (cystitis). If the bacterial invasion is not controlled by the immune system response or prompt treatment, bacteria may ascend the ureters to reach the kidneys and pyelonephritis occurs. Inadequate or delayed treatment of UTI may result in severe complications like life-threatening urosepsis, renal scarring or, rarely, end-stage renal disease and hypertension.

Once in the urinary tract, pathogens need to constantly avoid host defense mechanisms. Host defense consists mainly of the following three elements: First, the *unidirectional flow of urine* that supports the clearance of the urinary tract from bacteria. Second, the epithelial cells, which form a *physical barrier,* and third the local production of *inflammatory mediators and antimicrobial proteins* to recognize and trap bacteria or interfere with their ability to attach (P. Chowdhury, S.H. Sacks, N.S. Sheerin, Kidney Int. 2004, 66, 1334-1344). In order to overcome these protective elements, bacteria attach to the urinary tract epithelium via fimbrial adhesion molecules (H. Connell, M. Hedlund, W. Agace, C. Svanborg, Adv. Dent. Res. 1997, 11, 50-58). They are presumably internalized in an active process similar to phagocytosis once they are bound.

All symptomatic UTIs should be treated with antibiotics to prevent potential devastating complications. Uncomplicated UTI can be effectively treated with an oral antibiotic such as fluoroquinolones (e.g. ciprofloxacin or norfloxacin), cotrimoxazol or amoxicillin/clavanulate, depending on the susceptibility of the causing pathogen. However, recurrent infections with subsequent antibiotic exposure can lead to emergence of antimicrobial resistance, which often leads to treatment failure and reduces the range of therapeutic options.

Hence, there is an urgent need for public health to develop an efficient, cost-effective and safe non-antibiotic therapy to both prevent and treat UTIs without facilitating antimicrobial resistance. Inhibition of type 1 fimbriae-mediated bacterial attachment to the bladder epithelium is a very promising approach to achieve this aim.

The lectin FimH on the tip of type 1 fimbriae of *E. coli* binds to oligomannosides located on epithelial cells of the urinary tract. This specific binding plays an important role in the development of UTIs. *E. coli* adhere specifically to the terminal mannose moieties of uroplakin receptors on the surface of urinary tract epithelia.

More than two decades ago, Sharon and coworkers have investigated various mannosides and oligomannosides as antagonists for type 1 fimbriae-mediated specific bacterial adhesion (I. Ofek, D.L. Hasty, N. Sharon, FEMS Immunol Med Microbiol 2003, 38, 181-191). However, when binding affinities for various mannosides were tested in ELISA formats, only weak interactions with IC₅₀ values in the milli- to micromolar range were observed. Attempts to improve the affinity followed two different approaches: (i) the design of multivalent carbohydrate ligands and (ii) the rational design of ligands guided by information obtained from the crystal structure of FimH (A. Imberty, Y.M. Chabre, R. Roy, Chem. Eur J .2008, 14, 7490-7499).

Anti-adhesive α-D-mannopyranoside derivatives for prevention and treatment of bacterial infections are described in WO 2005/089733. Further anti-adhesive saccharide derivatives such as thio-α-L-fucopyranosides are described in WO 98/21220.

### Summary of the invention

The invention relates to compounds of the formula (I) 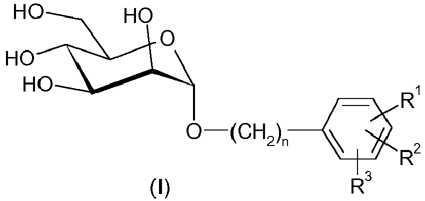 wherein
n is 0, 1 or 2;
R¹ is heteroaryl or heterocyclyl with 5 or more atoms, and
R² and R³ are, independent of each other, hydrogen, lower alkyl, halo-lower alkyl, hydroxy-lower alkyl, lower alkoxy-lower alkyl, optionally substituted alkenyl, optionally substituted alkinyl, cycloalkyl, hydroxy, lower alkoxy, halo-lower alkoxy, lower alkoxy-lower alkoxy, phenoxy, hydroxysulfonyloxy; mercapto, alkylmercapto, hydroxysulfinyl, alkylsulfinyl, halo-lower alkylsulfinyl, hydroxysulfonyl, alkylsulfonyl, arylsulfonyl, heteroaryl-sulfonyl, aminosulfonyl, amino optionally substituted by one or two substitutents selected from lower alkyl, hydroxy-lower alkyl, lower alkoxy-lower alkyl; lower alkylcarbonylamino, alkoxycarbonylamino, benzoylamino, pyridinylcarbonylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; carboxy, lower alkylcarbonyl, benzoyl, pyridinecarbonyl, pyrimidinecarbonyl, lower alkoxycarbonyl, aminocarbonyl, wherein amino is unsubstituted or substituted by one hydroxy or amino group or one or two substitutents selected from lower alkyl, hydroxy-lower alkyl or lower alkoxy-lower alkyl; tetrazolyl, cyano, halogen, or nitro; or wherein two substituents in ortho-position to each other form a 5- or 6-membered heterocyclic ring containing one or two oxygen atoms and/or one or two nitrogen atoms, wherein the nitrogen atoms are optionally substituted by lower alkyl, lower alkoxy-lower alkyl or lower alkylcarbonyl; and prodrugs and salts thereof.

Furthermore the invention relates to compounds of formula (I), wherein
n is 0, 1 or 2;
R¹ is heteroaryl or heterocyclyl; and
R² and R³ are, independent of each other, hydrogen, lower alkyl, halo-lower alkyl, hydroxy-lower alkyl, lower alkoxy-lower alkyl, optionally substituted alkenyl, optionally substituted alkinyl, cycloalkyl, hydroxy, lower alkoxy, halo-lower alkoxy, lower alkoxy-lower alkoxy, phenoxy, hydroxysulfonyloxy; mercapto, alkylmercapto, hydroxysulfinyl, alkyl-sulfinyl, halo-lower alkylsulfinyl, hydroxysulfonyl, alkylsulfonyl, arylsulfonyl, heteroaryl-sulfonyl, aminosulfonyl, amino optionally substituted by one or two substitutents selected from lower alkyl, hydroxy-lower alkyl, lower alkoxy-lower alkyl; lower alkylcarbonylamino, alkoxycarbonylamino, benzoylamino, pyridinylcarbonylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; carboxy, lower alkylcarbonyl, benzoyl, pyridinecarbonyl, pyrimidinecarbonyl, lower alkoxycarbonyl, aminocarbonyl, wherein amino is unsubstituted or substituted by one hydroxy or amino group or one or two substitutents selected from lower alkyl, hydroxy-lower alkyl or lower alkoxy-lower alkyl; tetrazolyl, cyano, halogen, or nitro; or wherein two substituents in ortho-position to each other form a 5- or 6-membered heterocyclic ring containing one or two oxygen atoms and/or one or two nitrogen atoms, wherein the nitrogen atoms are optionally substituted by lower alkyl, lower alkoxy-lower alkyl or lower alkylcarbonyl; and prodrugs and salts thereof
for use in the prevention and treatment of infectious diseases, such as infectious diseases caused by virulent strains of *E.coli,* in particular urinary tract infections.

Furthermore the invention relates to pharmaceutical compositions comprising these compounds, to a method of manufacture of these compounds, to the use of the compounds for the prevention and treatment of bacterial infections, in particular urinary tract infections, and to a method of prevention and treatment of such bacterial infections.

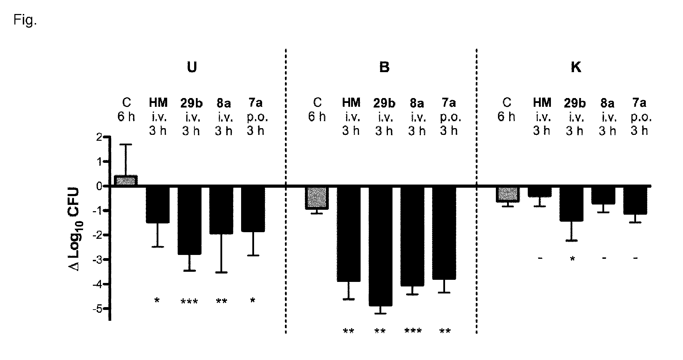

### Brief Description of the Figure

Treatment efficacy of the reference compound (**HM,** heptyl α-D-mannopyranoside) and three FimH antagonists (**8f, 8a** and **7a,** FimH = receptor binding domain of a fimbrial tip adhesin) at a dosage of 50 mg/kg in the UTI mouse model after 3 h of infection, compared to a 6 h infection study (n = 6, control). **HM, 8f** and **8a** were applied i.v. into the tail vein, whereas **7a** was applied orally. As baseline (reference), the mean counts of the 3 h infection were subtracted from the results of the tested antagonists and the 6 h control group. In all treated animals, bacterial counts were only marginally reduced in the kidneys. This lower response to the treatment with FimH antagonists is probably due to different bacterial adhesion mechanisms in bladder and kidney. Whereas in the bladder adhesion is mediated by type I pili (via the CRD of FimH), P pili-dependent interactions are crucial for the adhesion in the kidneys. *P -* values were calculated by comparing the treatment groups with the 3 h control group. (*) *P* < 0.05, (**) *P* < 0.01, (***) *P* < 0.001, (-) not significant (determined by Mann Whitney test).
C = control; U = urine; B = bladder; K= kidney;
Δ Log10 CFU = ΔLog10 CFU/ml (urine) or ΔLog10 CFU/organ (bladder, 2 kidneys).

### Detailed description of the invention

The invention relates to compounds of the formula **(I)** 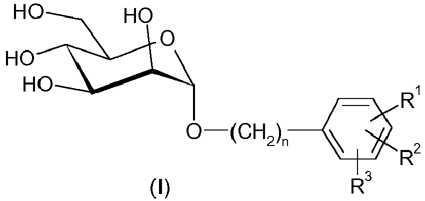 wherein
n is 0, 1 or 2;
R¹ is heteroaryl or heterocyclyl with 5 or more atoms, and
R² and R³ are, independent of each other, hydrogen, lower alkyl, halo-lower alkyl, hydroxy-lower alkyl, lower alkoxy-lower alkyl, optionally substituted alkenyl, optionally substituted alkinyl, cycloalkyl, hydroxy, lower alkoxy, halo-lower alkoxy, lower alkoxy-lower alkoxy, phenoxy, hydroxysulfonyloxy; mercapto, alkylmercapto, hydroxysulfinyl, alkyl-sulfinyl, halo-lower alkylsulfinyl, hydroxysulfonyl, alkylsulfonyl, arylsulfonyl, heteroaryl-sulfonyl, aminosulfonyl, amino optionally substituted by one or two substitutents selected from lower alkyl, hydroxy-lower alkyl, lower alkoxy-lower alkyl; lower alkylcarbonylamino, alkoxycarbonylamino, benzoylamino, pyridinylcarbonylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; carboxy, lower alkylcarbonyl, benzoyl, pyridinecarbonyl, pyrimidinecarbonyl, lower alkoxycarbonyl, aminocarbonyl, wherein amino is unsubstituted or substituted by one hydroxy or amino group or one or two substitutents selected from lower alkyl, hydroxy-lower alkyl or lower alkoxy-lower alkyl; tetrazolyl, cyano, halogen, or nitro; or wherein two substituents in ortho-position to each other form a 5- or 6-membered heterocyclic ring containing one or two oxygen atoms and/or one or two nitrogen atoms, wherein the nitrogen atoms are optionally substituted by lower alkyl, lower alkoxy-lower alkyl or lower alkylcarbonyl; and prodrugs and salts thereof.

Furthermore the invention relates to compounds of formula **(I),** wherein
n is 0, 1 or 2;
R¹ is heteroaryl or heterocyclyl; and
R² and R³ have the meanings indicated above; and
prodrugs and salts thereof
for use in the prevention and treatment of infectious diseases, such as infectious diseases caused by virulent strains of *E.coli,* in particular urinary tract infections.

Mannose-specific (type 1) fimbriae are among the most commonly found lectins in enterobacteriae. The adhesion of pathogenic organisms to host tissue mediated by such lectins is considered an important initial event in bacterial infection. Soluble carbohydrates recognized by the bacterial surface lectins inhibit the adhesion to complementary tissue resulting in the lack of the ability to initiate infection. The present invention relates to a particularly active group of mannoside derivatives, which can be successfully applied as FimH antagonists (FimH = receptor binding domain of a fimbrial tip adhesin). The compounds of the invention show a substantially higher activity than currently known mannosides.

A further aspect of the invention is the use of the compounds of the invention as drugs for the prevention and treatment of infectious diseases, in particular urinary tract infections. The advantage of the mannoside derivatives of the invention over state-of-the-art antibiotics is the fact that formation of resistance to carbohydrates leads to mutated lectins rendering themselves ineffective with respect to adhesion to host tissue.

The general terms used hereinbefore and hereinafter preferably have within the context of this disclosure the following meanings, unless otherwise indicated:

The prefix "lower" denotes a radical having up to and including a maximum of 7, especially up to and including a maximum of 4 carbon atoms, the radicals in question being either linear or branched with single or multiple branching.

Where the plural form is used for compounds, salts, and the like, this is taken to mean also a single compound, salt, or the like.

Double bonds in principle can have E- or Z-configuration. The compounds of this invention may therefore exist as isomeric mixtures or single isomers. If not specified both isomeric forms are intended.

Any asymmetric carbon atoms may be present in the (R)-, (S)- or (R,S)-configuration, preferably in the (R)- or (S)-configuration. The compounds may thus be present as mixtures of isomers or as pure isomers, preferably as enantiomer-pure diastereomers.

The invention relates also to possible tautomers of the compounds of formula **(I).**

Alkyl has from 1 to 12, preferably from 1 to 7 carbon atoms, and is linear or branched. Alkyl is preferably lower alkyl.

Lower alkyl has 1 to 7, preferably 1 to 4 carbon atoms and is butyl, such as n-butyl, sec-butyl, isobutyl, tert-butyl, propyl, such as n-propyl or isopropyl, ethyl or methyl. Preferably lower alkyl is methyl or ethyl. C₂-C₇-alkyl is lower alkyl with at least two carbon atoms, for example ethyl, propyl or butyl.

Cycloalkyl has preferably 3 to 7 ring carbon atoms, and may be unsubstitued or substituted, e.g. by lower alkyl or lower alkoxy. Cycloalkyl is, for example, cyclohexyl, cyclopentyl, methylcyclopentyl, or cyclopropyl, in particular cyclopropyl.

Aryl stands for a mono- or bicyclic fused ring aromatic group with 5 to 10 carbon atoms optionally carrying substituents, such as phenyl, 1-naphthyl or 2-naphthyl, or also a partially saturated bicyclic fused ring comprising a phenyl group, such as indanyl, dihydro-or tetrahydronaphthyl, all optionally substitued. Preferably, aryl is phenyl or indanyl or tetrahydronaphthyl, in particular phenyl.

The term "aryl carrying substituents" stands for aryl substituted by up to four substituents independently selected from lower alkyl, halo-lower alkyl, cycloalkyl-lower alkyl, carboxy-lower alkyl, lower alkoxycarbonyl-lower alkyl; arylalkyl or heteroarylalkyl, wherein aryl or heteroaryl are unsubstituted or substituted by up to three substituents selected from lower alkyl, cyclopropyl, halo-lower alkyl, lower alkoxy, hydroxysulfonyl, aminosulfonyl, tetrazolyl, carboxy, halogen, amino, cyano and nitro; hydroxy-lower alkyl, lower alkoxy-lower alkyl, aryloxy-lower alkyl, heteroaryloxy-lower alkyl, aryl-lower alkoxy-lower alkyl, heteroaryl-lower alkoxy-lower alkyl, lower alkoxy-lower alkoxy-lower alkyl; aminoalkyl wherein amino is unsubstituted or substituted by one or two substituents selected from lower alkyl, hydroxy-lower alkyl, alkoxy-lower alkyl and amino-lower alkyl, or by one substituent alkylcarbonyl, alkoxycarbonyl, amino-lower alkoxycarbonyl, lower alkoxy-lower alkoxycarbonyl and aminocarbonyl, or wherein the two substituents on nitrogen form together with the nitrogen heterocyclyl; optionally substituted alkenyl, optionally substituted alkinyl, cycloalkyl, aryl, heteroaryl, heterocyclyl, hydroxy, lower alkoxy, halo-lower alkoxy, lower alkoxy-lower alkoxy, cycloalkyl-lower alkoxy, aryloxy, aryl-lower alkoxy, aryloxy-lower alkoxy, heteroaryloxy, heteroaryl-lower alkoxy, heteroaryloxy-lower alkoxy, optionally substituted alkenyloxy, optionally substituted alkinyloxy, cycloalkyloxy, heterocyclyloxy, hydroxysulfonyloxy; alkylmercapto, hydroxysulfinyl, alkylsulfinyl, halo-lower alkylsulfinyl, hydroxysulfonyl, alkylsulfonyl, arylsulfonyl, heteroarylsulfonyl; aminosulfonyl wherein amino is unsubstituted or substituted by one or two substitutents selected from lower alkyl, cycloalkyl-lower alkyl, hydroxy-lower alkyl, lower alkoxy-lower alkyl, cycloalkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heterocyclyl; amino optionally substituted by one or two substitutents selected from lower alkyl, cycloalkyl-lower alkyl, hydroxy-lower alkyl, lower alkoxy-lower alkyl, di-lower alkylamino-lower alkyl, cycloalkyl, optionally substituted phenyl-lower alkyl and optionally substituted heteroaryl-lower alkyl, or by one substituent optionally substituted phenyl, optionally substituted heteroaryl, alkylcarbonyl, optionally substituted phenylcarbonyl, optionally substituted pyridylcarbonyl, alkoxycarbonyl or aminocarbonyl, and wherein alkyl or lower alkyl in each case may be substituted by halogen, lower alkoxy, aryl, heteroaryl or optionally substituted amino, or wherein the two substituents on nitrogen form together with the nitrogen heterocyclyl; carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; lower alkylcarbonyl, halo-lower alkylcarbonyl, optionally substituted phenylcarbonyl, optionally substituted heteroarylcarbonyl, carboxy, lower alkoxycarbonyl, lower alkoxy-lower alkoxycarbonyl; aminocarbonyl wherein amino is unsubstituted or substituted by one hydroxy or amino group or one or two substitutents selected from lower alkyl, cycloalkyl-lower alkyl, hydroxy-lower alkyl, lower alkoxy-lower alkyl, cycloalkyl, optionally substituted phenyl-lower alkyl and optionally substituted heteroaryl-lower alkyl, or wherein the two substituents on nitrogen form together with the nitrogen heterocyclyl; cyano, halogen, and nitro; and wherein two substituents in ortho-position to each other can form a 5-, 6- or 7-membered carbocyclic or heterocyclic ring containing one, two or three oxygen atoms, one or two nitrogen atoms and/or one sulfur atom, wherein the nitrogen atoms are optionally substituted by lower alkyl, lower alkoxy-lower alkyl or lower alkylcarbonyl.

In particular, the substituents may be independently selected from lower alkyl, halo-lower alkyl, hydroxy-lower alkyl, lower alkoxy-lower alkyl, optionally substituted alkenyl, optionally substituted alkinyl, cyclohexyl, cyclopropyl, aryl, heteroaryl, heterocyclyl, hydroxy, lower alkoxy, halo-lower alkoxy, lower alkoxy-lower alkoxy, cycloalkyloxy, phenoxy, hydroxysulfonyloxy; alkylmercapto, hydroxysulfinyl, alkylsulfinyl, halo-lower alkylsulfinyl, hydroxysulfonyl, alkylsulfonyl, arylsulfonyl, heteroarylsulfonyl; aminosulfonyl wherein amino is unsubstituted or substituted by one or two substitutents selected from lower alkyl, cycloalkyl-lower alkyl, hydroxy-lower alkyl, lower alkoxy-lower alkyl and optionally substituted phenyl-lower alkyl, or wherein the two substituents on nitrogen form together with the nitrogen heterocyclyl; amino optionally substituted by one or two substitutents selected from lower alkyl, cycloalkyl-lower alkyl, hydroxy-lower alkyl, lower alkoxy-lower alkyl, di-lower alkylamino-lower alkyl, cycloalkyl, or by one substituent optionally substituted phenyl, optionally substituted heteroaryl, alkylcarbonyl, optionally substituted phenylcarbonyl, optionally substituted pyridylcarbonyl, alkoxycarbonyl or aminocarbonyl, or wherein the two substituents on nitrogen form together with the nitrogen heterocyclyl; carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; lower alkylcarbonyl, halo-lower alkylcarbonyl, carboxy, lower alkoxycarbonyl, lower alkoxy-lower alkoxycarbonyl; aminocarbonyl wherein amino is unsubstituted or substituted by one hydroxy or amino group or one or two substitutents selected from lower alkyl, hydroxy-lower alkyl, lower alkoxy-lower alkyl, optionally substituted phenyl-lower alkyl and optionally substituted heteroaryl-lower alkyl, or wherein the two substituents on nitrogen form together with the nitrogen heterocyclyl; cyano, halogen, and nitro; and wherein two substituents in ortho-position to each other can form a 5- or 6-membered heterocyclic ring containing one or two oxygen atoms and/or one or two nitrogen atoms, wherein the nitrogen atoms are optionally substituted by lower alkyl, lower alkoxy-lower alkyl or lower alkylcarbonyl.

In optionally substituted phenyl, substituents are preferably lower alkyl, halo-lower alkyl, lower alkoxy-lower alkyl, cyclopropyl, hydroxy, lower alkoxy, halo-lower alkoxy, lower alkoxy-lower alkoxy, methylenedioxy, hydroxysulfonyloxy, carboxy, lower alkoxycarbonyl, aminocarbonyl, hydroxylaminocarbonyl, tetrazolyl, hydroxysulfonyl, aminosulfonyl, halo, cyano or nitro, in particular carboxy, lower alkoxycarbonyl, aminocarbonyl, hydroxylamino-carbonyl, tetrazolyl, or aminosulfonyl.

Heteroaryl represents an aromatic group containing at least one heteroatom selected from nitrogen, oxygen and sulfur, and is mono- or bicyclic, optionally carrying substituents. Monocyclic heteroaryl includes 5 or 6 membered heteroaryl groups containing 1, 2, 3 or 4 heteroatoms selected from nitrogen, sulfur and oxygen. Bicyclic heteroaryl includes 9 or 10 membered fused-ring heteroaryl groups. Examples of heteroaryl include pyrrolyl, thienyl, furyl, pyrazolyl, imidazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, and benzo or pyridazo fused derivatives of such monocyclic heteroaryl groups, such as indolyl, benzimidazolyl, benzofuryl, quinolinyl, isoquinolinyl, quinazolinyl, pyrrolopyridine, imidazopyridine, or purinyl, all optionally substituted. Preferably, heteroaryl is pyridyl, pyrimdinyl, pyrazinyl, pyridazinyl, thienyl, pyrazolyl, imidazolyl, thiazolyl, oxadiazolyl, triazolyl, oxazolyl, isoxazolyl, isothiazolyl, pyrrolyl, indolyl, pyrrolopyridine or imidazopyridine; in particular pyridyl, pyrimidinyl, pyrazinyl, pyridazinyl, pyrazolyl, imidazolyl, thiazolyl, oxadiazolyl, triazolyl, indolyl, pyrrolopyridine or imidazopyridine.

The term "heteroaryl carrying substituents" stands for heteroaryl substituted by up to three substituents independently selected from lower alkyl, halo-lower alkyl, cycloalkyl-lower alkyl, hydroxy-lower alkyl, lower alkoxy-lower alkyl, aryloxy-lower alkyl, heteroaryloxy-lower alkyl, lower alkoxy-lower alkoxy-lower alkyl; aminoalkyl, wherein amino is unsubstituted or substituted by one or two substituents selected from lower alkyl, hydroxy-lower alkyl, alkoxy-lower alkyl, amino-lower alkyl, alkylcarbonyl, alkoxycarbonyl, amino-lower alkoxycarbonyl, lower alkoxy-lower alkoxycarbonyl and aminocarbonyl; optionally substituted alkenyl, optionally substituted alkinyl, cycloalkyl; aryl, heteroaryl, arylalkyl or heteroarylalkyl, wherein aryl or heteroaryl are unsubstituted or substituted by up to three substituents selected from lower alkyl, halo-lower alkyl, lower alkoxy, halogen, amino, cyano and nitro; hydroxy, lower alkoxy, halo-lower alkoxy, lower alkoxy-lower alkoxy, cycloalkyloxy, cycloalkyl-lower alkoxy, aryloxy, aryl-lower alkoxy, heteroaryloxy, heteroaryl-lower alkoxy, alkenyloxy, alkinyloxy, alkylmercapto, alkylsulfinyl, halo-lower alkylsulfinyl, alkylsulfonyl, arylsulfonyl, heteroarylsulfonyl, aminosulfonyl wherein amino is unsubstituted or substituted by one or two substitutents selected from lower alkyl, cycloalkyl-lower alkyl, hydroxy-lower alkyl, lower alkoxy-lower alkyl, cycloalkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heterocyclyl; amino optionally substituted by one or two substitutents selected from lower alkyl, cycloalkyl-lower alkyl, hydroxy-lower alkyl, lower alkoxy-lower alkyl, di-lower alkylamino-lower alkyl, cycloalkyl, optionally substituted phenyl, optionally substituted phenyl-lower alkyl, optionally substituted heteroaryl, optionally substituted heteroaryl-lower alkyl, alkylcarbonyl, alkoxycarbonyl or aminocarbonyl, and wherein alkyl or lower alkyl in each case may be substituted by halogen, lower alkoxy, aryl, heteroaryl or optionally substituted amino, or wherein the two substituents on nitrogen form together with the nitrogen heterocyclyl; lower alkylcarbonyl, halo-lower alkylcarbonyl, optionally substituted phenylcarbonyl, carboxy, lower alkoxycarbonyl, lower alkoxy-lower alkoxycarbonyl; aminocarbonyl wherein amino is unsubstituted or substituted by one hydroxy or amino group or one or two substitutents selected from lower alkyl, cycloalkyl-lower alkyl, hydroxy-lower alkyl, lower alkoxy-lower alkyl, cycloalkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heterocyclyl; cyano, halogen, and nitro.

In particular, the substituents on heteroaryl may be independently selected from lower alkyl, halo-lower alkyl, cycloalkyl-lower alkyl, lower alkoxy-lower alkyl, lower alkoxy-lower alkoxy-lower alkyl, optionally substituted alkenyl, optionally substituted alkinyl, cycloalkyl, aryl, heteroaryl, hydroxy, lower alkoxy, cycloalkyloxy, alkenyloxy, alkinyloxy, alkyl-mercapto, alkylsulfinyl, halo-lower alkylsulfinyl, alkylsulfonyl, arylsulfonyl, aminosulfonyl wherein amino is unsubstituted or substituted by one or two substitutents selected from lower alkyl, cycloalkyl-lower alkyl, hydroxy-lower alkyl, lower alkoxy-lower alkyl, cycloalkyl, optionally substituted phenyl, optionally substituted phenyl-lower alkyl, optionally substituted heteroaryl and optionally substituted heteroaryl-lower alkyl, or wherein the two substituents on nitrogen form together with the nitrogen heterocyclyl; amino optionally substituted by one or two substitutents selected from lower alkyl, cycloalkyl-lower alkyl, hydroxy-lower alkyl, lower alkoxy-lower alkyl, di-lower alkylamino-lower alkyl, cycloalkyl, alkylcarbonyl, alkoxycarbonyl or aminocarbonyl, and wherein alkyl or lower alkyl in each case may be substituted by lower alkoxy or optionally substituted amino, or wherein the two substituents on nitrogen form together with the nitrogen heterocyclyl; lower alkyl-carbonyl, halo-lower alkylcarbonyl, carboxy, lower alkoxycarbonyl, lower alkoxy-lower alkoxycarbonyl; aminocarbonyl wherein amino is unsubstituted or substituted by one hydroxy or amino group or one or two substitutents selected from lower alkyl, cycloalkyl-lower alkyl, hydroxy-lower alkyl, lower alkoxy-lower alkyl or cycloalkyl, or wherein the two substituents on nitrogen form together with the nitrogen heterocyclyl; cyano, halogen, and nitro.

In optionally substituted heteroaryl, substituents are preferably lower alkyl, halo-lower alkyl, lower alkoxy-lower alkyl, hydroxy, lower alkoxy, halo-lower alkoxy, lower alkoxy-lower alkoxy, methylenedioxy, carboxy, lower alkoxycarbonyl, aminocarbonyl, hydroxylaminocarbonyl, tetrazolyl, aminosulfonyl, halo, cyano or nitro.

Alkenyl contains one or more, e.g. two or three, double bonds, and is preferably lower alkenyl, such as 1- or 2-butenyl, 1-propenyl, allyl or vinyl.

Alkinyl is preferably lower alkinyl, such as propargyl or acetylenyl.

In optionally substituted alkenyl or alkinyl, substituents are preferably lower alkyl, lower alkoxy, halo, optionally substituted aryl or optionally substituted heteroaryl, and are connected with a saturated or unsaturated carbon atom of alkenyl or alkinyl.

Heterocyclyl designates preferably a saturated, partially saturated or unsaturated, mono-or bicyclic ring containg 4-10 atoms comprising one, two or three heteroatoms selected from nitrogen, oxygen and sulfur, which may, unless otherwise specified, be carbon or nitrogen linked, wherein a ring nitrogen atom may optionally be substituted by a group selected from lower alkyl, amino-lower alkyl, aryl, aryl-lower alkyl and acyl, and a ring carbon atom may be substituted by lower alkyl, amino-lower alkyl, aryl, aryl-lower alkyl, heteroaryl, lower alkoxy, hydroxy or oxo, or which may be fused with an optionally substituted benzo ring. Substituents considered for substituted benzo are those mentioned above for optionally substituted aryl. Examples of heterocyclyl are pyrrolidinyl, oxazolidinyl, thiazolidinyl, piperidinyl, morpholinyl, piperazinyl, dioxolanyl, tetrahydro-furanyl and tetrahydropyranyl, and optionally substituted benzo fused derivatives of such monocyclic heterocyclyl, for example indolinyl, benzoxazolidinyl, benzothiazolidinyl, tetrahydroquinolinyl, and benzodihydrofuryl.

Acyl designates, for example, alkylcarbonyl, cycloalkylcarbonyl, arylcarbonyl, aryl-lower alkylcarbonyl, or heteroarylcarbonyl. Lower acyl is preferably lower alkylcarbonyl, in particular propionyl or acetyl.

Hydroxyalkyl is especially hydroxy-lower alkyl, preferably hydroxymethyl, 2-hydroxyethyl or 2-hydroxy-2-propyl.

Cyanoalkyl designates preferably cyanomethyl and cyanoethyl.

Haloalkyl is preferably fluoroalkyl, especially trifluoromethyl, 3,3,3-trifluoroethyl or pentafluoroethyl.

Halogen is fluorine, chlorine, bromine, or iodine.

Lower alkoxy is especially methoxy, ethoxy, isopropyloxy, or tert-butyloxy.

Arylalkyl includes aryl and alkyl as defined hereinbefore, and is e.g. benzyl, 1-phenethyl or 2-phenethyl.

Heteroarylalkyl includes heteroaryl and alkyl as defined hereinbefore, and is e.g. 2-, 3- or 4-pyridylmethyl, 1- or 2-pyrrolylmethyl, 1-pyrazolylmethyl, 1-imidazolylmethyl, 2-(1-imidazolyl)ethyl or 3-(1-imidazolyl)propyl.

In substituted amino, the substituents are preferably those mentioned as substituents hereinbefore. In particular, substituted amino is alkylamino, dialkylamino, optionally substituted arylamino, optionally substituted arylalkylamino, lower alkylcarbonylamino, benzoylamino, pyridylcarbonylamino, lower alkoxycarbonylamino or optionally substituted aminocarbonylamino.

Prodrugs are especially compounds wherein a -COOH, -S(O)OH, -S(O)₂OH or -P(O)(OH)₂ group of a compound of formula **(I)** is derivatized as linear or branched alkyl, hydroxyalkyl, methoxyalkyl, aminoalkyl, alkenyl, alkinyl, phenyl, benzyl and phenethyl ester. Most typically, the alkyl, hydroxyalkyl, methoxyalkyl, aminoalkyl, alkenyl and alkinyl group contains 1 to 12 carbon atoms, preferably 1 to 7 or more preferably 1 to 4 carbon atoms.

Further prodrugs according to this invention are compounds wherein one or more, for example one, two, three or four hydroxy groups of the mannose ring and/or a hydroxy group in one of the residues R¹, R² or R³ are derivatized by conversion into a group such as, but not limited to, a phosphate ester, acetate, fluoroacetate, chloroacetate, hemisuccinate, dimethylaminoacetate, or phosphoryloxy-methoxycarbonyl group. Carbamate prodrugs of hydroxy groups are also included, as are carbonate prodrugs, sulfonate esters and sulfate esters of hydroxy groups. Hydroxy groups are derivatized as (acyloxy)methyl and (acyloxy)ethyl ethers, wherein the acyl group is lower alkylcarbonyl optionally substituted by halogen, hydroxyl, lower alkoxy, amino and/or carboxy. More specific examples include replacement of the hydrogen atom of the hydroxy group with a group such as lower alkanoyloxymethyl, 1-(lower alkanoyloxy)ethyl, 1-methyl-1-(lower alkanoyloxy)ethyl, lower alkoxycarbonyloxymethyl, lower alkoxycarbonylaminomethyl, succinoyl, lower alkanoyl, halo-lower alkanoyl, α-amino-lower alkanoyl, arylcarbonyl, substituted α-aminoacetyl or α-(a-aminoacetylamino)acetyl, wherein each substituted α-aminoacetyl group is independently derived from a naturally occurring L-amino acid, -P(O)(OH)₂, -P(O)(lower alkoxy)₂, or glycosyl (the radical resulting from the removal of a hydroxy group of the hemiacetal form of a carbohydrate).

Particular prodrugs are compounds of formula **(I)** wherein all four hydroxy groups of the mannose ring are acetylated.

Salts are especially the pharmaceutically acceptable salts of compounds of formula **(I).**

Such salts are formed, for example, as acid addition salts, preferably with organic or inorganic acids, from compounds of formula **(I)** with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantane-carboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methyl-benzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

From compounds of formula **(I)** with acid functional groups, e.g. substituted by carboxy, salts may be formed with suitable cations, especially with pharmaceutically acceptable cations. Suitable cations are, e.g., sodium, potassium, calcium, magnesium or ammonium cations, or also cations derived by protonation from primary, secondary or tertiary amines containing, for example, lower alkyl, hydroxy-lower alkyl or hydroxy-lower alkoxy-lower alkyl groups, e.g., 2-hydroxyethylammonium, 2-(2-hydroxyethoxy)ethyldimethyl-ammonium, diethylammonium, di(2-hydroxyethyl)ammonium, trimethylammonium, triethylammonium, 2-hydroxyethyldimethylammonium, or di(2-hydroxyethyl)methyl-ammonium, also from correspondingly substituted cyclic secondary and tertiary amines, e.g., N-methylpyrrolidinium, N-methylpiperidinium, N-methylmorpholinium, N-2-hydroxy-ethylpyrrolidinium, N-2-hydroxyethylpiperidinium, or N-2-hydroxyethylmorpholinium, and the like.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient.

The compounds of formula **(I)** have valuable pharmacological properties. The invention also relates to compounds of formula **(I),** their prodrugs and salts as defined hereinbefore for use as medicaments. A compound of formula **(I)** according to the invention shows prophylactic and therapeutic efficacy especially against bacterial infections, in particular against infective diseases caused by *Escherichia coli* (*E. coli*), a Gram negative bacterium commonly found in the lower intestine of warm-blooded organisms. Most *E. coli* strains are harmless and part of the normal flora of the gut, however, the compounds of the invention are useful in the treatment of infective diseases caused by virulent strains of *E. coli,* in particular in the treatment of gastroenteritis, diarrhea, food poisoning, urinary tract infections, pyelonephritis, and neonatal meningitis caused by *E. coli* strains, also in the treatment of unusual infective diseases caused by virulent *E. coli* strains, in particular in the treatment of haemolytic-uremic syndrome (HUS), peritonitis, mastitis, sepsis, and pneumonia caused by *E. coli.*

Particularly preferred is the use of a compound of formula **(I),** a prodrug or a salt thereof according to the invention as a medicament for the prevention and treatment of urinary infections caused by *E. coli.*

A compound of formula **(I)** can be administered alone or in combination with one or more other therapeutic agents, possible combination therapy taking the form of fixed combinations, or the administration of a compound of the invention and one or more other therapeutic agents being staggered or given independently of one another, or the combined administration of fixed combinations and one or more other therapeutic agents. Therapeutic agents for possible combination are especially trimethoprim/sulfamethoxazol (co-trimoxazol), fluoroquinolone (e.g. ciprofloxacin, levofloxacin or norfloxacin), amoxicilin/clavulanic acid, and nitrofurantoin.

With the groups of preferred compounds of formula **(I)** mentioned hereinafter, definitions of substituents from the general definitions mentioned hereinbefore may reasonably be used, for example, to replace more general definitions with more specific definitions or especially with definitions characterized as being preferred.

In particular, the invention refers to compounds of formula **(I),** wherein n is 0 or 1, preferably 0.

Heteroaryl R¹ is preferably pyrrolyl, thienyl, furyl, pyrazolyl, imidazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolyl, benzimidazolyl, benzofuryl, pyridopyrrolyl, pyridoimidazolyl, quinolinyl, isoquinolinyl, quinazolinyl, or purinyl, all optionally substituted. Such groups R¹ are usually carbon-linked, but, in the case where the nitrogen of the heteroaryl group carries hydrogen, may also be nitrogen-linked. Preferably, R¹ is pyridyl, pyrimidinyl, pyrazinyl, pyridazinyl, thienyl, pyrazolyl, imidazolyl, thiazolyl, oxadiazolyl, triazolyl, tetrazolyl, oxazolyl, isoxazolyl, isothiazolyl, pyrrolyl, indolyl, benzimidazolyl, pyridopyrrolyl, or pyridoimidazolyl, all optionally substituted, in particular pyridyl, pyrimidinyl, pyrazinyl, triazolyl, tetrazolyl, oxazolyl, isoxazolyl, isothiazolyl, pyrrolyl, indolyl, benzimidazolyl, pyridopyrrolyl, or pyridoimidazolyl, all optionally subtituted. Particularly preferred is pyridyl, pyrimidinyl, pyrazinyl, triazolyl, tetrazolyl, pyrrolyl, indolyl, benzimidazolyl, pyridopyrrolyl, or pyridoimidazolyl, all optionally substituted.

Preferred substituents considered for R¹ with the meaning of the mentioned heteroaryl groups are alkyl, halo-lower alkyl, cycloalkyl-lower alkyl, lower alkoxy-lower alkyl, lower alkoxy-lower alkoxy-lower alkyl, optionally substituted alkenyl, optionally substituted alkinyl, cycloalkyl, aryl, heteroaryl, hydroxy, lower alkoxy, cycloalkyloxy, alkenyloxy, alkinyloxy, hydroxysulfonyloxy, lower alkylmercapto, hydroxysulfinyl, lower alkylsulfinyl, halo-lower alkylsulfinyl, hydroxysulfonyl, lower alkylsulfonyl, arylsulfonyl; amino optionally substituted by one or two substitutents selected from lower alkyl, cycloalkyl-lower alkyl, hydroxy-lower alkyl, lower alkoxy-lower alkyl and di-lower alkylamino-lower alkyl; or one substituent cycloalkyl, lower alkylcarbonyl, phenylcarbonyl, pyrimidinylcarbonyl, alkoxycarbonyl or aminocarbonyl, and wherein alkyl or lower alkyl in each case may be substituted by lower alkoxy or optionally substituted amino; carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; or wherein the two substituents on nitrogen form together with the nitrogen heterocyclyl; lower alkylcarbonyl, halo-lower alkylcarbonyl, carboxy, lower alkoxycarbonyl, lower alkoxy-lower alkoxy-carbonyl; aminocarbonyl wherein amino is unsubstituted or substituted by one hydroxy or amino group or one or two substitutents selected from lower alkyl, cycloalkyl-lower alkyl, hydroxy-lower alkyl, lower alkoxy-lower alkyl or cycloalkyl, or wherein the two substituents on nitrogen form together with the nitrogen heterocyclyl; cyano, halogen, and nitro.

Preferred substituents considered for R¹ with the meaning of the mentioned preferred heteroaryl groups are lower alkyl, halo-lower alkyl, lower alkoxy-lower alkyl, cyclopropyl, hydroxy, lower alkoxy, halo-lower alkoxy, lower alkoxy-lower alkoxy, phenoxy, methylene-dioxy, hydroxysulfonyloxy, hydroxysulfonyl, aminosulfonyl, lower alkylsulfonyl, amino, lower alkylcarbonylamino, benzoylamino, pyridylcarbonylamino, carboxymethylamino or lower alkoxycarbonylmethylamino substituted at the methyl group such that the resulting substituent corresponds to one of the 20 naturally occurring standard amino acids, amino-methylcarbonylamino substituted at the methyl group such that the resulting acyl group corresponds to one of the 20 naturally occurring standard amino acids; carboxy, lower alkoxycarbonyl, aminocarbonyl, hydroxylaminocarbonyl, tetrazolyl, halo, cyano or nitro. Most preferred substituents are halo-lower alkyl, lower alkoxy, carboxy, lower alkoxy-carbonyl, tetrazolyl, cyano and nitro.

Heterocyclyl R¹ with 5 or more atoms is preferably pyrrolidinyl, oxazolidinyl, thiazolidinyl, piperidinyl, morpholinyl, piperazinyl, dioxolanyl, tetrahydrofuranyl, tetrahydropyranyl, indolinyl, isoindolinyl, benzoxazolidinyl, benzothiazolidinyl, tetrahydroquinolinyl, or benzodihydrofuryl, wherein such group R¹ may be carbon-linked or, if possible, nitrogen-linked, wherein a ring nitrogen atom may optionally be substituted by a group selected from lower alkyl, amino-lower alkyl, aryl, aryl-lower alkyl and acyl, and a ring carbon atom may be substituted by lower alkyl, amino-lower alkyl, aryl, aryl-lower alkyl, heteroaryl, lower alkoxy, hydroxy or oxo, or wherein the benzo ring, if present, is optionally substituted by lower alkyl, halo-lower alkyl, lower alkoxy-lower alkyl, hydroxy, lower alkoxy, halo-lower alkoxy, lower alkoxy-lower alkoxy, methylenedioxy, carboxy, lower alkoxycarbonyl, aminocarbonyl, hydroxylaminocarbonyl, tetrazolyl, aminosulfonyl, halo, cyano or nitro.

More preferably, R¹ is pyrrolidinyl, oxazolidinyl, indolinyl, isoindolinyl, tetrahydroquinolinyl, or benzodihydrofuryl, in particular indolinyl, wherein such group R¹ may by carbon- or, if possible, nitrogen-linked, wherein a ring nitrogen atom may optionally be substituted by lower alkyl, aryl-lower alkyl or acyl, and a ring carbon atom may be substituted by lower alkyl, amino-lower alkyl, aryl, aryl-lower alkyl, heteroaryl, lower alkoxy, hydroxy or oxo, or wherein the benzo ring, if present, is optionally substituted by lower alkyl, halo-lower alkyl, lower alkoxy-lower alkyl, hydroxy, lower alkoxy, halo-lower alkoxy, lower alkoxy-lower alkoxy, methylenedioxy, carboxy, lower alkoxycarbonyl, aminocarbonyl, hydroxylaminocarbonyl, tetrazolyl, aminosulfonyl, halo, cyano or nitro, more preferably by halo-lower alkyl, lower alkoxy, carboxy, lower alkoxycarbonyl, tetrazolyl, cyano or nitro.

Preferred as R² and R³ are, independent of each other, hydrogen, lower alkyl, halo-lower alkyl, lower alkoxy-lower alkyl, cyclopropyl, hydroxy, lower alkoxy, halo-lower alkoxy, lower alkoxy-lower alkoxy, phenoxy, hydroxysulfonyloxy, methylenedioxy, hydroxysulfinyl, hydroxysulfonyl, lower alkylsulfonyl, arylsulfonyl, aminosulfonyl, amino optionally substituted by one or two substitutents selected from lower alkyl, hydroxy-lower alkyl, lower alkoxy-lower alkyl; lower alkylcarbonylamino, alkoxycarbonylamino, benzoylamino, pyridinylcarbonylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; carboxy, lower alkoxycarbonyl, aminocarbonyl, hydroxylaminocarbonyl, tetrazolyl, aminosulfonyl, halo, cyano or nitro.

Particularly preferred substituents R² and R³ are hydrogen, lower alkyl, halo-lower alkyl, cyclopropyl, lower alkoxy, lower alkoxy-lower alkoxy, phenoxy, hydroxysulfonyl, aminosulfonyl, amino, lower alkylcarbonylamino, benzoylaminoamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; carboxy, lower alkoxycarbonyl, aminocarbonyl, hydroxylaminocarbonyl, tetrazolyl, halo, cyano or nitro. Most preferred substituents R² and R³ are hydrogen, lower alkoxy, such as methoxy, and halo, such as chloro and fluoro.

Preferably, the invention refers to compounds of formula (I), wherein R¹ is a residue of formula (B) or (C) 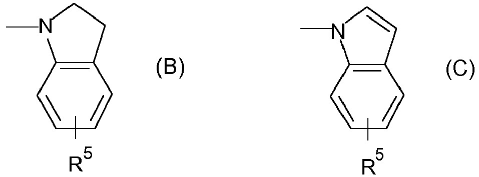 wherein R⁵ is hydrogen, trifluoromethyl, cylcopropyl, lower alkoxy, lower alkoxy-lower alkoxy, phenyl-lower alkoxy, phenoxy, hydroxysulfonyl, aminosulfonyl, lower alkylsulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids, carboxy, lower alkoxycarbonyl, aminocarbonyl, hydroxylaminocarbonyl, tetrazolyl, nitro, cyano, or halo;
preferably hydrogen, trifluoromethyl, lower alkoxy, such as methoxy, benzyloxy, amino, carboxy, lower alkoxycarbonyl, tetrazolyl, nitro, cyano, or halo;
or of formula (D) 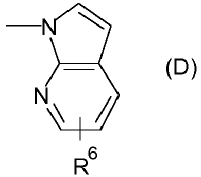 wherein R⁶ is hydrogen, trifluoromethyl, cylcopropyl, lower alkoxy, lower alkoxy-lower alkoxy, phenoxy, hydroxysulfonyl, aminosulfonyl, lower alkylsulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids, carboxy, lower alkoxycarbonyl, aminocarbonyl, hydroxylaminocarbonyl, tetrazolyl, nitro, cyano, or halo; preferably hydrogen, trifluoromethyl, lower alkoxy, such as methoxy, carboxy, lower alkoxycarbonyl, tetrazolyl, nitro, cyano, or halo;
or of formula (E) 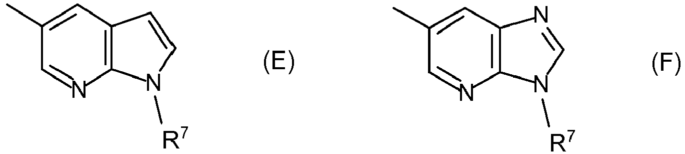 wherein R⁷ is hydrogen, lower alkyl, lower alkoxy-lower alkyl, lower alkylcarbonyl, optionally substituted phenylcarbonyl, or aminomethylcarbonyl substituted at the methyl group such that the resulting acyl group corresponds to one of the 20 naturally occurring standard amino acids;
preferably hydrogen or lower alkyl, such as methyl;
or of formula (G) 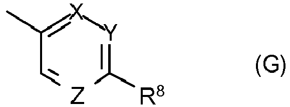 wherein R⁸ is carboxy or lower alkoxycarbonyl; X or Y or Z, or X and Z, or Y and Z are nitrogen atoms and the other atoms X, Y and Z are carbon atoms;
or of formula (H) 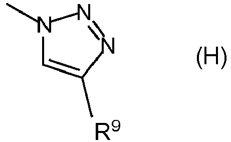 wherein R⁹ is carboxy or lower alkoxycarbonyl; and
prodrugs and salts thereof.

Also preferred are compounds of formula (I), wherein R¹ is a residue of formula (B) or (C) 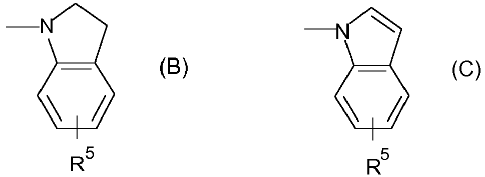 wherein R⁵ is hydrogen, trifluoromethyl, cylcopropyl, lower alkoxy, lower alkoxy-lower alkoxy, phenoxy, hydroxysulfonyl, aminosulfonyl, lower alkylsulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids, carboxy, lower alkoxycarbonyl, aminocarbonyl, hydroxylaminocarbonyl, tetrazolyl, nitro, cyano, or halo;
or of formula (D) 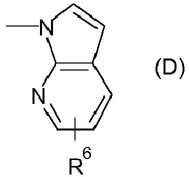 wherein R⁶ is hydrogen, trifluoromethyl, cylcopropyl, lower alkoxy, lower alkoxy-lower alkoxy, phenoxy, hydroxysulfonyl, aminosulfonyl, lower alkylsulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids, carboxy, lower alkoxycarbonyl, aminocarbonyl, hydroxylaminocarbonyl, tetrazolyl, nitro, cyano, or halo;
or of formula (E) 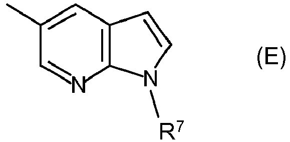 wherein R⁷ is hydrogen, lower alkyl, lower alkoxy-lower alkyl, lower alkylcarbonyl, optionally substituted phenylcarbonyl, or aminomethylcarbonyl substituted at the methyl group such that the resulting acyl group corresponds to one of the 20 naturally occurring standard amino acids; and
prodrugs and salts thereof
for use in the prevention and treatment of infectious diseases, such as infectious diseases caused by virulent strains of *E.coli,* in particular urinary tract infections.

Preferred prodrugs are the tetraacetates.

Most preferred are the compounds of the examples, in particular the examples 1-71.

A compound of the invention may be prepared by processes that, though not applied hitherto for the new compounds of the present invention, are known *per se,* in particular a process, wherein a compound of formula **(I),** wherein the hydroxy functions of the α-D-mannopyranoside are protected and wherein R¹ is halogen, is condensed with a reagent replacing halogen by heteroaryl or heterocyclyl, the protective groups are removed, and, if so desired, an obtainable compound of formula **(I)** is converted into another compound of formula **(I),** a compound of formula **(I)** is converted into a prodrug, a free compound of formula **(I)** is converted into a salt, an obtainable salt of a compound of formula **(I)** is converted into the free compound or another salt, and/or a mixture of isomeric compounds of formula **(I)** is separated into the individual isomers.

Suitable reagents for replacing halogen R¹ by carbon-linked heteroaryl or carbon-linked heterocyclyl are, e.g., boronic acids in the presence of a palladium catalyst, a reaction known under the name of Suzuki reaction. Other reagents that can be used are described, for example, in M. Rubens, S.L. Buchwald, Accounts Chem. Res. 2008, 41, 1461-1473.

Alternatively, halogen R¹ may be replaced by cyano and the heteroaryl or heterocyclyl group constructed by addition and further ring elaboration starting by addition reactions to the cyano function, see, for example, N.A. Bokach, V.Y. Kukushkin, Russ. Chem. Bull. 2006, 55, 1869-1882.

Suitable reagents for replacing halogen R¹ by nitrogen-linked heteroaryl or nitrogen-linked heterocyclyl are, e.g., the corresponding heteroaryl or heterocyclyl compound in the presence of strong base and optionally a catalyst, whereby halogen R¹ is preferably iodine.

The protecting groups may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end products. The specialist knows, or can easily establish, which protecting groups are suitable with the reactions mentioned.

The protection of such functional groups by such protecting groups, the protecting groups themselves, and their removal reactions are described for example in standard reference books for peptide synthesis and in special books on protective groups such as T.W. Greene & P.G.M. Wuts, "Protective Groups in Organic Synthesis", Wiley, 3rd edition 1999.

In the additional process steps, carried out as desired, functional groups of the starting compounds which should not take part in the reaction may be present in unprotected form or may be protected for example by one or more of the protecting groups mentioned hereinabove under "protecting groups". The protecting groups are then wholly or partly removed according to one of the methods described there.

In the conversion of an obtainable compound of formula (I) into another compound of formula (I), an amino group may be alkylated or acylated to give the correspondingly substituted compounds. Alkylation may be performed with an alkyl halide or an activated alkyl ester. For methylation, diazomethane may be used. Alkylation may also be performed with an aldehyde under reducing conditions. For acylation the corresponding acyl chloride is preferred. Alternatively, an acid anhydride may be used, or acylation may be accomplished with the free acid under conditions used for amide formation known *per* se in peptide chemistry, e.g. with activating agents for the carboxy group, such as 1-hydroxybenzotriazole, optionally in the presence of suitable catalysts or co-reagents. Furthermore amine may be transformed into heteroaryl and heterocyclyl under reaction conditions typical for such cyclizations.

A hydroxy group may be alkylated (etherified) or acylated (esterified) to give the correspondingly substituted compounds in a procedure related to the one described for an amino group. Alkylation may be performed with an alkyl halide or an activated alkyl ester. For methylation, diazomethane may be used. For acylation the corresponding acyl chloride or acid anhydride may be used, or acylation may be accomplished with the free acid and a suitable activating agent.

Reduction of a nitro group in a nitro-substituted aryl or heteroaryl group to give the corresponding amino group is done, e.g., with iron powder in alcohol or with other reducing agents.

A carboxy group in a carboxy-substituted aryl or heteroaryl group may be amidated under conditions used for amide formation known *per se* in peptide chemistry, e.g. with the corresponding amine and an activating agent for the carboxy group, such as 1-hydroxy-benzotriazole, optionally in the presence of suitable catalysts or co-reagents.

A chloro, bromo or iodo substitutent in an aryl or heteroaryl group may be replaced by phenyl or a phenyl derivative by reaction with a suitable phenylboronic acid in a Suzuki reaction as described above.

Prodrugs of a compound of formula **(I)** are prepared in a manner known *per se,* in particular by a standard esterification reaction. Tetraacetates are usually formed already at the stage of an intermediate, since the acetyl group is also a customary protecting group in sugar chemistry. In this case, benzyl esters are used in the aglycone to allow their selective deprotection.

Salts of a compound of formula (**I**) with a salt-forming group may be prepared in a manner known *per se.* Acid addition salts of compounds of formula **(I)** may thus be obtained by treatment with an acid or with a suitable anion exchange reagent.

Salts can usually be converted to free compounds, e.g. by treating with suitable basic agents, for example with alkali metal carbonates, alkali metal hydrogencarbonates, or alkali metal hydroxides, typically potassium carbonate or sodium hydroxide.

It should be emphasized that reactions analogous to the conversions mentioned in this chapter may also take place at the level of appropriate intermediates.

All process steps described here can be carried out under known reaction conditions, preferably under those specifically mentioned, in the absence of or usually in the presence of solvents or diluents, preferably such as are inert to the reagents used and able to dissolve these, in the absence or presence of catalysts, condensing agents or neutralising agents, for example ion exchangers, typically cation exchangers, for example in the H⁺ form, depending on the type of reaction and/or reactants at reduced, normal, or elevated temperature, for example in the range from -100°C to about 190°C, preferably from about -80°C to about 150°C, for example at -80 to +60°C, at -20 to +40°C, at r.t., or at the boiling point of the solvent used, under atmospheric pressure or in a closed vessel, where appropriate under pressure, and/or in an inert atmosphere, for example under argon or nitrogen.

Salts may be present in all starting compounds and transients, if these contain salt-forming groups. Salts may also be present during the reaction of such compounds, provided the reaction is not thereby disturbed.

At all reaction stages, isomeric mixtures that occur can be separated into their individual isomers, e.g. diastereomers or enantiomers, or into any mixtures of isomers, e.g. racemates or diastereomeric mixtures.

The invention relates also to those forms of the process in which one starts from a compound obtainable at any stage as a transient and carries out the missing steps, or breaks off the process at any stage, or forms a starting material under the reaction conditions, or uses said starting material in the form of a reactive derivative or salt, or produces a compound obtainable by means of the process according to the invention and further processes the said compound *in situ.* In the preferred embodiment, one starts from those starting materials which lead to the compounds described hereinabove as preferred, particularly as especially preferred, primarily preferred, and/or preferred above all.

In the preferred embodiment, a compound of formula **(I)** is prepared according to or in analogy to the processes and process steps defined in the Examples.

The compounds of formula **(I),** including their salts, are also obtainable in the form of hydrates, or their crystals can include for example the solvent used for crystallization, i.e. be present as solvates.

New starting materials and/or intermediates, as well as processes for the preparation thereof, are likewise the subject of this invention. In the preferred embodiment, such starting materials are used and reaction conditions so selected as to enable the preferred compounds to be obtained.

Starting materials of formula **(I)** are known, commercially available, or can be synthesized in analogy to or according to methods that are known in the art. In particular, compounds of formula **(I)** wherein R¹ is halogen are obtained in a reaction of a suitably activated and protected α-D-mannopyranoside, for example the corresponding trichloroacetimidate, or also 1-haloglycosides or thioglycosides, with a phenol, benzyl alcohol or phenylethanol, respectively, bearing the proper substituents R² and R³, and R¹ as halogen.

The present invention relates also to pharmaceutical compositions that comprise a compound of formula **(I)** as active ingredient and that can be used especially in the treatment of infective diseases mentioned at the beginning. Compositions for enteral administration, such as nasal, buccal, rectal, uretal or, especially, oral administration, and for parenteral administration, such as intravenous, intramuscular or subcutaneous administration, to warm-blooded animals, especially humans, are especially preferred. The compositions comprise the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

The present invention relates especially to pharmaceutical compositions that comprise a compound of formula **(I),** a tautomer, a prodrug or a pharmaceutically acceptable salt, or a hydrate or solvate thereof, and at least one pharmaceutically acceptable carrier.

The invention relates also to pharmaceutical compositions for use in a method for the prophylactic or especially therapeutic management of the human or animal body, in particular in a method of treating infective disease, especially those mentioned hereinabove.

The invention relates also to processes and to the use of compounds of formula **(I)** thereof for the preparation of pharmaceutical preparations which comprise compounds of formula **(I)** as active component (active ingredient).

A pharmaceutical composition for the prophylactic or especially therapeutic management of an infective disease, of a warm-blooded animal, especially a human, comprising a novel compound of formula **(I)** as active ingredient in a quantity that is prophylactically or especially therapeutically active against the said diseases, is likewise preferred.

The pharmaceutical compositions comprise from approximately 1 % to approximately 95% active ingredient, single-dose administration forms comprising in the preferred embodiment from approximately 20% to approximately 90% active ingredient and forms that are not of single-dose type comprising in the preferred embodiment from approximately 5% to approximately 20% active ingredient. Unit dose forms are, for example, coated and uncoated tablets, ampoules, vials, suppositories, or capsules. Further dosage forms are, for example, ointments, creams, pastes, foams, tinctures, lip-sticks, drops, sprays, dispersions, etc. Examples are capsules containing from about 0.05 g to about 1.0 g active ingredient.

The pharmaceutical compositions of the present invention are prepared in a manner known *per se,* for example by means of conventional mixing, granulating, coating, dissolving or lyophilizing processes.

Preference is given to the use of solutions of the active ingredient, and also suspensions or dispersions, especially isotonic aqueous solutions, dispersions or suspensions which, for example in the case of lyophilized compositions comprising the active ingredient alone or together with a carrier, for example mannitol, can be made up before use. The pharmaceutical compositions may be sterilized and/or may comprise excipients, for example preservatives, stabilizers, wetting agents and/or emulsifiers, solubilizers, salts for regulating osmotic pressure and/or buffers and are prepared in a manner known *per se,* for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, e.g. Tween 80^{®} (polyoxyethylene(20)sorbitan mono-oleate).

Suspensions in oil comprise as the oil component the vegetable, synthetic, or semisynthetic oils customary for injection purposes. In respect of such, special mention may be made of liquid fatty acid esters that contain as the acid component a long-chained fatty acid having from 8 to 22, especially from 12 to 22, carbon atoms. The alcohol component of these fatty acid esters has a maximum of 6 carbon atoms and is a monovalent or polyvalent, for example a mono-, di- or trivalent, alcohol, especially glycol and glycerol. As mixtures of fatty acid esters, vegetable oils such as cottonseed oil, almond oil, olive oil, castor oil, sesame oil, soybean oil and groundnut oil are especially useful.

The manufacture of injectable preparations is usually carried out under sterile conditions, as is the filling, for example, into ampoules or vials, and the sealing of the containers.

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, crosslinked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Tablet cores can be provided with suitable, optionally enteric, coatings through the use of, inter alia, concentrated sugar solutions which may comprise gum arabic, talc, polyvinyl-pyrrolidone, polyethylene glycol and/or titanium dioxide, or coating solutions in suitable organic solvents or solvent mixtures, or, for the preparation of enteric coatings, solutions of suitable cellulose preparations, such as acetylcellulose phthalate or hydroxypropyl-methylcellulose phthalate. Dyes or pigments may be added to the tablets or tablet coatings, for example for identification purposes or to indicate different doses of active ingredient.

Pharmaceutical compositions for oral administration also include hard capsules consisting of gelatin, and also soft, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

Pharmaceutical compositions suitable for rectal administration are, for example, suppositories that consist of a combination of the active ingredient and a suppository base. Suitable suppository bases are, for example, natural or synthetic triglycerides, paraffin hydrocarbons, polyethylene glycols or higher alkanols.

For parenteral administration, aqueous solutions of an active ingredient in water-soluble form, for example of a water-soluble salt, or aqueous injection suspensions that contain viscosity-increasing substances, for example sodium carboxymethylcellulose, sorbitol and/or dextran, and, if desired, stabilizers, are especially suitable. The active ingredient, optionally together with excipients, can also be in the form of a lyophilizate and can be made into a solution before parenteral administration by the addition of suitable solvents.

Solutions such as are used, for example, for parenteral administration can also be employed as infusion solutions.

Preferred preservatives are, for example, antioxidants, such as ascorbic acid, or microbicides, such as sorbic acid or benzoic acid.

The present invention relates furthermore to a method for the prevention and treatment of an infective disease, which comprises administering a compound of formula **(I)** or a pharmaceutically acceptable salt thereof, wherein the radicals and symbols have the meanings as defined above for formula **(I),** in a quantity effective against said disease, to a warm-blooded animal requiring such treatment. The compounds of formula **(I)** can be administered as such or especially in the form of pharmaceutical compositions, prophylactically or therapeutically, preferably in an amount effective against the said diseases, to a warm-blooded animal, for example a human, requiring such treatment. In the case of an individual having a bodyweight of about 70 kg the daily dose administered is from approximately 0.01 g to approximately 1 g, preferably from approximately 0.05 g to approximately 0.1 g, of a compound of the present invention.

The following Examples serve to illustrate the invention without limiting the invention in its scope.

### Examples

### General methods

Commercially available reagents were purchased from Fluka, Aldrich, Merck, AKSci, ASDI or Alfa Aesar. Methanol was dried by distillation from sodium methoxide. Dichloromethane (CH₂Cl₂) was dried by filtration through Al₂O₃ (Fluka, basic; 0.05-0.15 mm). Toluene was dried by distillation from sodium/benzophenone.

Optical rotations were measured at 20°C on a Perkin Elmer 341 polarimeter with a path length of 1 dm. Concentrations are given in g/100 mL.

NMR spectra were obtained on a Bruker Avance 500 UltraShield spectrometer at 500.13 MHz (¹H) or 125.76 MHz (¹³C). Chemical shifts are given in ppm and were calibrated on residual solvent peaks or to tetramethyl silane as internal standard. Multiplicities are specified as s (singulet), d (doublet), dd (doublet of a doublet), t (triplet), q (quartet) or m (multiplet). Assignment of the ¹H and ¹³C NMR spectra was achieved using 2D methods (COSY, HSQC).

Microanalyses were performed at the Department of Chemistry, University of Basel, Switzerland. ESI mass spectra were recorded on a Waters micromass ZQ instrument. High resolution mass spectra were obtained on an ESI Bruker Daltonics micrOTOF spectrometer equipped with a TOF hexapole detector.

Microwave-assisted reactions were carried out with CEM Discover and Explorer. Reactions were monitored by TLC using glass plates coated with silica gel 60 F₂₅₄ and visualized by using UV light and/or by charring with a molybdate solution (a 0.02 M solution of ammonium cerium sulfate dihydrate and ammonium molybdate tetrahydrate in aqueous 10% H₂SO₄) with heating to 140°C for 5 min.

Column chromatography was performed on a CombiFlash Companion (ISCO, Inc.) using RediSep normal phase disposable flash columns (silica gel). Reversed phase chromatography was performed on LiChroprep^{®}RP-18 (Merck, 40- 63 µm).

For the preparation of compounds of formula **(I)** wherein R¹ is 4-methoxycarbonylphenyl or 4-carboxyphenyl (Examples 1 to 21) the following procedure is used: Glycosylation of different halogenated phenols **4a-f** with trichloracetimidate **3** (obtainable from acetylated α-D-mannopyranoside **1** by selective deacetylation followed by condensation of **2** with trichloracetonitrile) under Lewis acid catalysis gives the mannosylated phenylhalides **5a-d** (Scheme 1) and **5e-f** (Scheme 2). In a following palladium catalyzed Suzuki coupling, the mannosylated phenylhalides **5a-f** and 4-methoxycarbonylphenylboronic acid are converted under microwave conditions to the para-substituted biphenyls **6a-d** (Scheme 1) and to the meta-substituted biphenyls **6e-f** (Scheme 2). Compounds **7a-d** (Scheme1) and **7e-f** (Scheme 2) are obtained by Zemplen deprotection. Saponification of the methyl esters finally gives the sodium salts **8a-b** (Scheme 1) and **8e-f** (Scheme 2).

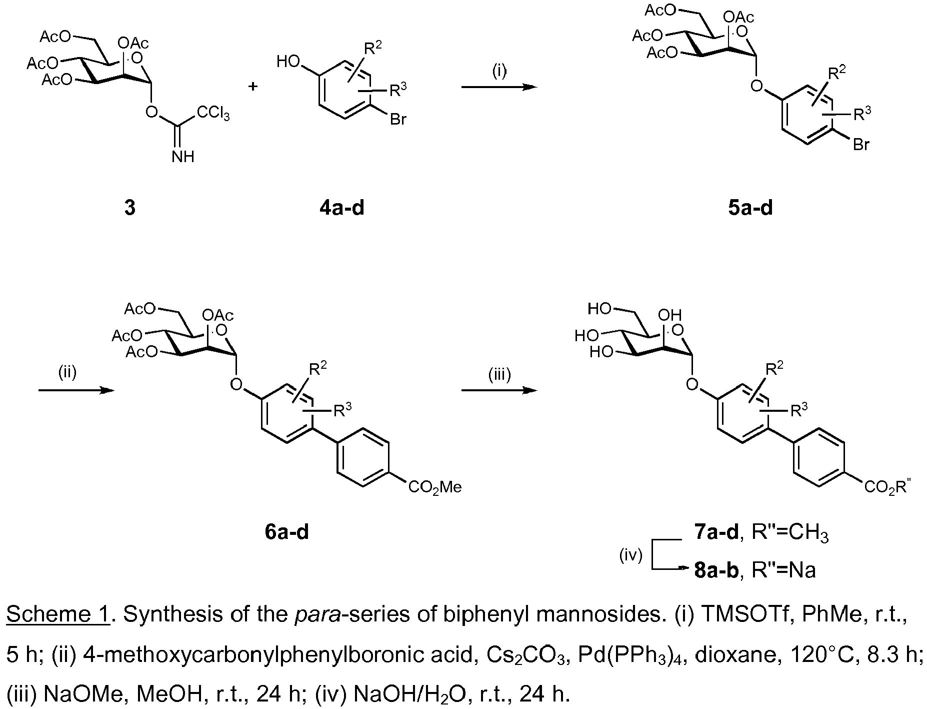

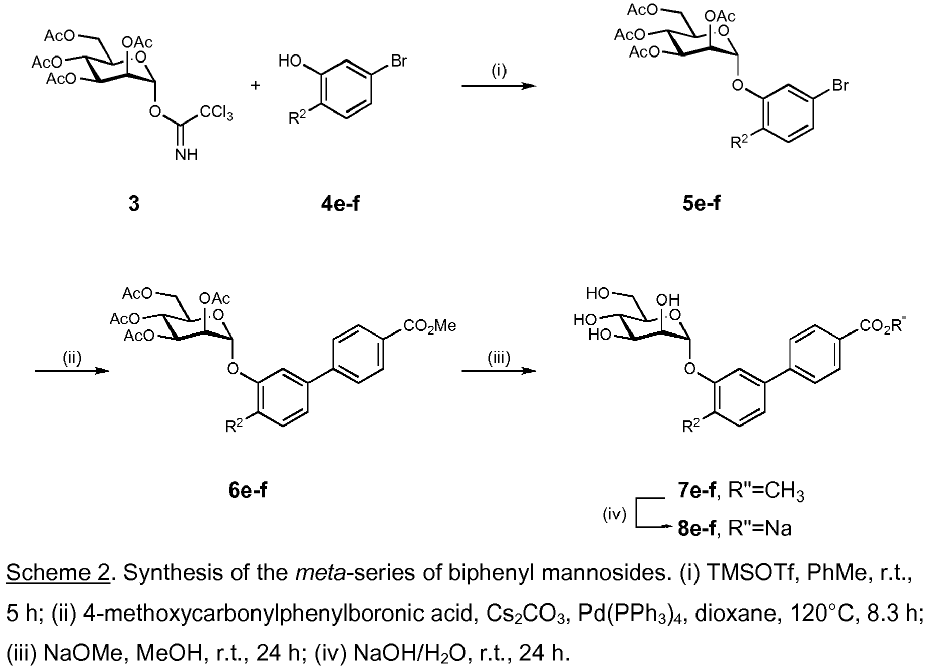

### Reference Example 1: 2,3,4,6-Tetra-O-acetyl-α-D-mannopyranose (2)

1,2,3,4,6-Penta-O-acetyl-α-D-mannopyranoside (**1,** 10 g, 25.6 mmol) is dissolved in DMF (55 mL). Hydrazine acetate (3.54 mg, 38.5 mmol) is added and the mixture is stirred at r.t. under argon for 3 h. Subsequently, the reaction mixture is dissolved in ethyl acetate (80 mL). The organic layer is washed with water (2 x 100 mL) and brine (1 x 100 mL). The aqueous layers are extracted with ethyl acetate (2 x 100 mL) and the combined organic layers are dried over Na₂SO₄. The solvent is removed *in vacuo* and the resulting residue purified by chromatography on silica gel eluting with petroleum ether/EtOAc (4:1 to 1:1) to give **(2)** (7.9 g, 89%).

### Reference Example 2: 2,3,4,6-Tetra-O-acetyl-a-D-mannopyranosyl trichloroacetimidate (3)

2,3,4,6-Tetra-O-acetyl-α-D-mannopyranose (**2,** 7.80 g, 22.4 mmol) is dissolved in dry dichloromethane (50 mL). Trichloroacetonitrile (11.25 mL) and cesium carbonate (730 mg, 2.24 mmol) are added and the reaction is flushed with argon. The mixture is stirred for 3.5 h at r.t. Removal of the solvent by evaporation under reduced pressure leaves a residue that is purified by chromatography on silica gel eluting with petroleum ether/EtOAc (19:1 to 1:1) to yield **3** (10.6 g, 96%).

### Reference Example 3: 4-Bromo-2-chlorophenyl 2,3,4,6-tetra-O-acetyl-α-D-manno-pyranoside (5a)

To a stirred solution of 2,3,4,6-tetra-*O*-acetyl-α-D-mannopyranosyl trichloracetimidate **3** (2.38 g, 4.84 mmol, 1.0 equiv) and 4-bromo-2-chlorophenol (**4a**, 1.20 g, 5.80 mmol, 1.2 equiv) in toluene (20 mL) under argon, TMSOTf (107 mg, 0.484 mmol, 0.1 equiv) is added dropwise via syringe. The reaction is stirred at r.t. for 5 h and then diluted with toluene (15 mL) and quenched with saturated aqueous NaHCO₃ solution (15 mL). The layers are separated and the aqueous layer is extracted with toluene (3 x 15 mL). The combined organic layers are dried over Na₂SO₄ and concentrated *in vacuo.* The residue is purified by flash chromatography (petroleum ether/EtOAc, 19:1 to 1.5:1) to yield **5a** (538 mg, 85%) as a white solid.
[α]_{D}²⁰+60.6 (c = 0.40, CHCl₃); ¹H NMR (CDCl₃): δ 2.02 (s, 3H, OAc), 2.02 (s, 3H, OAc), 2.04 (s, 3H, OAc), 2.18 (s, 3H, OAc), 4.05 (dd, *J* = 2.3 Hz, 12.2 Hz, 1 H, H-6a), 4.10 (ddd, *J =* 2.7 Hz, 5.3 Hz, 7.6 Hz, 1H, H-5), 4.24 (dd, *J* = 5.4 Hz, 12.2 Hz, 1 H, H-6b), 5.35 (t, *J =* 10.1 Hz, 1H, H-4), 5.48 (m, 2H, H-1, H-2), 5.56 (dd, *J =* 3.2 Hz, 10.1 Hz, 1H, H-3), 7.03 (d, *J* = 8.8 Hz, 1 H, C₆H₃), 7.30 (dd, *J* = 2.4 Hz, 8.8 Hz, 1 H, C₆H₃), 7.53 (d, *J* = 2.4 Hz, 1 H, C₆H₃); ¹³C-NMR (CDCl₃): δ 20.9, 21.1 (4C, 4OAc), 62.3 (C-6), 65.9 (C-4), 68.9 (C3), 69.4 (C-2), 70.1 (C-5), 96.9 (C-1), 115.9 (Ar-Cⁱ), 118.4 (Ar-C), 125.7 (Ar-Cⁱ), 130.8 (Ar-C), 133.3 (Ar-C), 150.6 (Ar-Cⁱ), 169.9, 170.0, 170.1, 170.7 (4C=O); ESI-MS calcd. for C₂₀H₂₂BrClO₁₀ [M+Na]⁺: 559.0; found 559.0; Anal.: Calcd. for C₂₀H₂₂BrClO₁₀: C 44.67, H 4.12; found C 45.08, H 4.14.

### Reference Example 4: 4-Bromo-3-chlorophenyl 2,3,4,6-tetra-O-acetyl-α-D-manno-pyranoside (5b)

Compound 3 (900 mg, 1.83 mmol) is treated with 4-bromo-3-chlorophenol (**4b,** 455 mg, 2.19 mmol) and TMSOTf (41 mg, 0.18 mmol) in toluene (9 mL) according to the procedure for **5a,** Example 3. The residue is purified by flash chromatography (petroleum ether/ EtOAc, 19:1 to 1.5:1) to yield **5b** (580 mg, 59%) as a white foam.
[α]_{D}²⁰+76.9 (c = 1.00, CHCl₃); ¹H NMR (CDCl₃): δ 2.01 (s, 3H, OAc), 2.02 (s, 3H, OAc), 2.03 (s, 3H, OAc), 2.17 (s, 3H, OAc), 4.01 (ddd, *J* = 2.3 Hz, 6.0 Hz, 9.8 Hz, 1 H, H-5), 4.05 (dd, *J =* 2.3 Hz, 12.2 Hz, 1H, H-6a), 4.25 (dd, *J =* 6.0 Hz, 12.2 Hz, 1H, H-6b), 5.32 (t, *J* = 10.1 Hz, 1H, H-4), 5.39 (dd, *J* = 1.9 Hz, 3.5 Hz, 1H, H-2), 5.44 (d, *J =* 1.7 Hz, 1H, H-1), 5.48 (dd, *J* = 3.5 Hz, 10.0 Hz, 1 H, H-3), 6.87 (dd, *J* = 2.8 Hz, 8.9 Hz, 1 H, C₆H₃), 7.26 (d, *J* = 2.8 Hz, 1 H, C₆H₃), 7.50 (d, *J* = 8.9 Hz, 1 H, C₆H₃); ¹³C-NMR (CDCl₃): δ 20.9, 21.1 (4C, 4OAc), 62.3 (C-6), 66.0 (C-4), 68.8 (C3), 69.2 (C-2), 69.7 (C-5), 96.2 (C-1), 116.0 (Ar-Cⁱ), 116.9 (Ar-C), 118.8 (Ar-C), 134.3 (Ar-C), 135.3 (Ar-Cⁱ), 155.3 (Ar-Cⁱ), 169.9, 170.1, 170.1, 170.7 (4C=O); ESI-MS calcd for C₂₀H₂₂BrClO₁₀ [M+Na]⁺: 561.0; found 561.0; Anal.: Calcd. for C₂₀H₂₂BrClO₁₀: C 44.67, H 4.12; found C 44.79, H 4.10.

### Reference Example 5: 4-Bromo-2,6-dichlorophenyl 2,3,4,6-tetra-O-acetyl-α-D-manno-pyranoside (5c)

Compound **3** (900 mg, 1.83 mmol) is treated with 4-bromo-2,6-dichlorophenol (**4c,** 530 mg, 2.19 mmol) and TMSOTf (41 mg, 0.18 mmol) in toluene (9 mL) according to the procedure for **5a,** Example 3. The residue is purified by flash chromatography (petroleum ether/EtOAc, 19:1 to 1.5:1) to yield **5c** (438 mg, 42%) as a white foam.
[α]_{D}²⁰+58.2 (c = 1.07, CHCl₃); ¹H NMR (CDCl₃): δ 2.01 (s, 3H, OAc), 2.06 (s, 3H, OAc), 2.07 (s, 3H, OAc), 2.16 (s, 3H, OAc), 4.15 (dd, *J* = 2.3 Hz, 12.4 Hz, 1H, H-6a), 4.27 (dd, *J* = 5.1 Hz, 12.4 Hz, 1 H, H-6b), 4.67 (ddd, *J* = 2.2 Hz, 5.0 Hz, 10.2 Hz, 1 H, H-5), 5.33 (d, *J* = 1.8 Hz, 1H, H-1), 5.37 (t, *J =* 10.2 Hz, 1H, H-4), 5.56 (dd, *J =* 3.3 Hz, 10.1 Hz, 1H, H-3), 5.75 (dd, *J =* 2.0 Hz, 3.3 Hz, 1 H, H-2), 7.46 (s, 2H, C₆H₂); ¹³C-NMR (CDCl₃): δ 20.9, 21.0, 21.1 (4C, 4OAc), 62.4 (C-6), 65.9 (C-4), 68.7 (C3), 69.4 (C-2), 71.0 (C-5), 101.3 (C-1), 117.9, 130.0, 132.1, 149.2 (6C, 6Ar-C), 169.9, 170.0, 170.1, 170.8 (4C=O); ESI-MS calcd. for C₂₀H₂₁BrCl₂O₁₀ [M+Na]⁺: 595.0; found 594.9; Anal.: Calcd. for C₂₀H₂₁BrCl₂O₁₀: C 41.98, H 3.70; found C 42.22, H 3.73.

### Reference Example 6: 4-Bromo-3,5-dichlorophenyl 2,3,4,6-tetra-O-acetyl-α-D-manno-pyranoside (5d)

Compound 3 (907 mg, 1.84 mmol) is treated with 4-bromo-3,5-dichlorophenol (**4d,** 535 mg, 2.21 mmol) and TMSOTf (41 mg, 0.18 mmol) in toluene (9 mL) according to the procedure for **5a,** Example 3. The residue is purified by flash chromatography (petroleum ether/EtOAc, 19:1 to 1.5:1) to yield **5d** (807 mg, 77%) as a white foam.
[α]_{D}²⁰+76.3 (c = 1.00, CHCl₃); ¹H NMR (CDCl₃): δ 2.02 (s, 3H, OAc), 2.04 (2s, 6H, 2OAc), 2.18 (s, 3H, OAc), 3.99 (m, 1 H, H-5), 4.05 (dd, *J* = 2.2 Hz, 12.2 Hz, 1 H, H-6a), 4.25 (dd, *J* = 6.4 Hz, 12.3 Hz, 1H, H-6b), 5.31 (t, *J =* 10.1 Hz, 1H, H-4), 5.38 (dd, *J =* 1.9 Hz, 3.4 Hz, 1H, H-2), 5.45 (m, 2H, H-1, H-3), 7.19 (s, 2H, C₆H₂); ¹³C-NMR (CDCl₃): δ 20.8, 20.9, 21.0 (4C, 4OAc), 62.4 (C-6), 65.9 (C-4), 68.7 (C3), 69.1 (C-2), 69.8 (C-5), 96.3 (C-1), 117.3, 117.5, 137.0, 154.8 (6C, 6Ar-C), 170.0, 170.1, 170.7 (4C, 4C=O); ESI-MS calcd. for C₂₀H₂₁BrCl₂O₁₀ [M+Na]⁺: 595.0; found 595.0; Anal.: Calcd. for C₂₀H₂₁BrCl₂O₁₀: C 41.98, H 3.70; found C 41.64, H 3.69.

### Reference Example 7: 5-Bromo-2-chlorophenyl 2,3,4,6-tetra-O-acetyl-α-D-mannopyranoside (5e)

Compound **3** (900 mg, 1.83 mmol) is treated with 5-bromo-2-chlorophenol (**4e,** 455 mg, 2.19 mmol) and TMSOTf (41 mg, 0.18 mmol) in toluene (9 mL) according to the procedure for **5a,** Example 3. The residue is purified by flash chromatography (petroleum ether/ EtOAc, 19:1 to 1.5:1) to yield **5e** (654 mg, 67%) as a white foam.
[α]_{D}²⁰ +52.2 (c = 0.96, CHCl₃); ¹H NMR (CDCl₃): δ 2.02 (s, 3H, OAc), 2.05 (s, 3H, OAc), 2.05 (s, 3H, OAc), 2.18 (s, 3H, OAc), 4.10 (m, 2H, H-5, H-6a), 4.26 (dd, *J* = 6.2 Hz, 12.1 Hz, 1H, H-6b), 5.33 (t, *J =* 10.1 Hz, 1H, H-4), 5.49 (m, 2H, H-1, H-2), 5.56 (m, 1H, H-3), 7.14 (dd, *J =* 2.1 Hz, 8.5 Hz, 1H, C₆H₃), 7.24 (m, 1H, C₆H₃), 7.35 (d, *J =* 2.1 Hz, 1H, C₆H₃); ¹³C-NMR (CDCl₃): δ 20.9, 21.1 (4C, 4OAc), 62.4 (C-6), 66.0 (C-4), 68.8 (C3), 69.3 (C-2), 70.0 (C-5), 96.9 (C-1), 120.6 (Ar-Cⁱ), 120.7 (Ar-C), 123.6 (Ar-Cⁱ), 127.2 (Ar-C), 131.6 (ArC), 152.0 (Ar-Cⁱ), 170.0, 170.1, 170.8 (4C, 4C=O); ESI-MS calcd. for C₂₀H₂₂BrClO₁₀ [M+Na]⁺: 561.0; found 561.0; Anal.: Calcd. for C₂₀H₂₂BrClO₁₀: C 44.67, H 4.12; found C 45.18, H 4.21.

### Reference Example 8: 3-Bromophenyl 2,3,4,6-tetra-O-acetyl-α-D-mannopyranoside (5f)

Compound **3** (900 mg, 1.83 mmol) is treated with 3-bromophenol (**4f,** 379 mg, 2.19 mmol) and TMSOTf (41 mg, 0.18 mmol) in toluene (9 mL) according to the procedure for **5a,** Example 3. The residue is purified by flash chromatography (petroleum ether/EtOAc, 19:1 to 1.5:1) to yield **5f** (645 mg, 70%) as a white foam.
[α]_{D}²⁰ +66.8 (c = 1.08, CHCl₃); ¹H NMR (CDCl₃): δ 2.01 (s, 3H, OAc), 2.03 (2s, 6H, 2OAc), 2.18 (s, 3H, OAc), 4.05 (m, 2H, H-5, H-6a), 4.25 (dd, *J* = 6.4 Hz, 12.6 Hz, 1 H, H-6b), 5.32 (t, *J =* 10.0 Hz, 1 H, H-4), 5.40 (dd, *J* = 1.9 Hz, 3.5 Hz, 1 H, H-2), 5.47 (d, *J =* 1.7 Hz, 1 H, H-1), 5.50 (dd, *J* = 3.5 Hz, 10.0 Hz, 1 H, H-3), 7.00 (ddd, *J =* 1.3 Hz, 2.3 Hz, 7.9 Hz, 1 H, C₆H₄), 7.15 (t, *J =* 8.0 Hz, 1H, C₆H₄), 7.18 (dt, *J =* 1.4 Hz, 7.9 Hz, 1H, C₆H₄), 7.29 (t, *J* = 2.0 Hz, 1H, C₆H₄); ¹³C-NMR (CDCl₃): δ 20.9, 21.1 (4C, 4OAc), 62.4 (C-6), 66.1 (C-4), 68.9 (C3), 69.4 (C-2), 69.5 (C-5), 95.9 (C-1), 115.7 (Ar-C), 120.1 (Ar-C), 123.0 (Ar-Cⁱ), 126.4 (Ar-C), 130.9 (Ar-C), 156.4 (Ar-Cⁱ), 169.9, 170.1, 170.2, 170.8 (4C=O); ESI-MS calcd. for C₂₀H₂₃BrO₁₀ [M+Na]⁺: 525.0; found 525.1; Anal.: Calcd. for C₂₀H₂₃BrO₁₀: C 47.73, H 4.61; found C 47.81, H 4.56.

### Reference Example 9: Methyl 4'-(2,3,4,6-tetra-O-acetyl-α-D-mannopyranosyloxy)-3'-chloro-biphenyl-4-carboxlate (6a)

### Reference Example 10: Methyl 4'-(2,3,4,6-tetra-O-acetyl-α-D-mannopyranosyloxy)-2'-chloro-biphenyl-4-carboxylate (6b)

### Reference Example 11: Methyl 4'-(2,3,4,6-tetra-O-acetyl-α-D-mannopyranosyloxy)-3',5' dichloro-biphenyl-4-carboxylate (6c)

### Reference Example 12: Methyl 4'-(2,3,4,6-tetra-O-acetyl-α-D-mannopyranosyloxy)-2',6'-dichloro-biphenyl-4-carboxylate (6d)

### Reference Example 13: Methyl 3'-(2,3,4,6-tetra-O-acetyl-α-D-mannopyranosyloxy)-4'-chloro-biphenyl-4-carboxylate (6e)

### Reference Example 14: Methyl 3'-(2,3,4,6-tetra-O-acetyl-α-D-mannopyranosyloxy)-biphenyl-4-carboxylate (6f)

### Reference Example 15: Methyl 3'-chloro-4'-(α-D-mannopyranosyloxy)-biphenyl-4-carboxylate (7a)

### Reference Example 16: Sodium 3'-chloro-4'-(α-D-mannopyranosyloxy)-biphenyl-4-carboxylate (8a)

### Reference Example 17: Methyl 2'-chloro-4'-(α-D-mannopyranosyloxy)-biphenyl-4-carboxylate (7b) and sodium 2'-chloro-4'-(α-D-mannopyranosyloxy)-biphenyl-4-carboxylate (8b)

### Reference Example 18: Methyl 3'5'-dichloro-4'-(α-D-mannopyranosyloxy)-biphenyl-4-carboxylate (7c)

### Reference Example 19: Methyl 2',6'-dichloro-4'-(α-D-mannopyranosyloxy)-biphenyl-4-carboxylate (7d)

### Reference Example 20: Methyl 4'-chloro-3'-(a-D-mannopyranosyloxy)-biphenyl-4-carboxylate (7e) and sodium 4'-chloro-3'-(α-D-mannopyranosyloxy)-biphenyl-4-carboxylate (8e)

### Reference Example 21: Methyl 3'-(α-D-mannopyranosyloxy)-biphenyl-4-carboxylate (7f) and sodium 3'-(α-D-mannopyranosyloxy)-biphenyl-4-carboxylate (8f)

For the preparation of compounds of formula **(I)** wherein R¹ is 4-cyanophenyl and 4-tetrazolylphenyl (Examples 22-24) the following procedure is used:

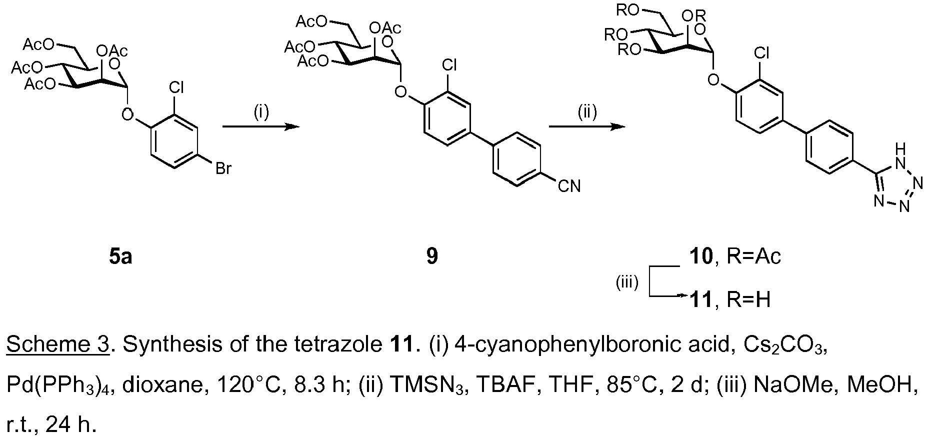

### Reference Example 22: 4'-(2,3,4,6-Tetra-O-acetyl-α-D-mannopyranosyloxy)-3'-chloro-biphenyl-4-carbonitrile (9)

### Reference Example 23: 5-[4'-(2,3,4,6-Tetra-O-acetyl-α-D-mannopyranosyloxy)-3'-chlorobiphenyl-4-yl]-1H-tetrazole (10)

### Reference Example 24: 5-[3'-Chloro-4'-(α-D-mannopyranosyloxy)-biphenyl-4-yl]-1H-tetrazole (11)

In an alternative mode of synthesis, α-D-mannose pentaacetate is directly converted to the 4-bromophenyl derivative, which in turn is reacted to give a further compound of formula **(I)** wherein R¹ is 4-methoxycarbonylphenyl and 4-carboxyphenyl (Examples 25 to 27):

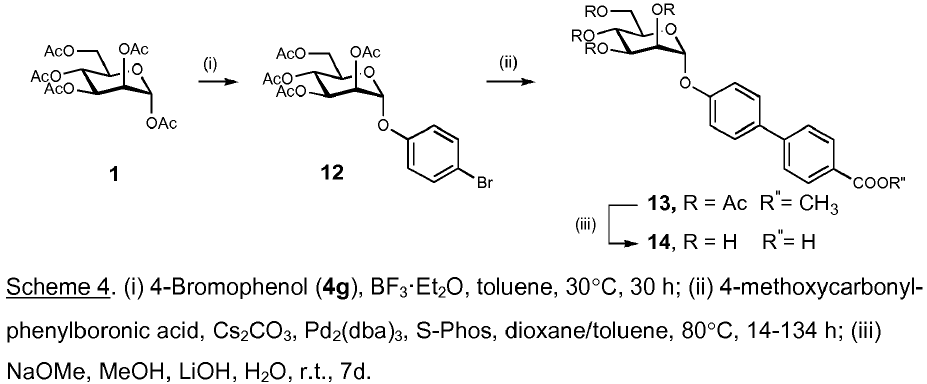

### Reference Example 25: 4-Bromophenyl 2,3,4,6-tetra-O-acetyl-α-D-mannopyranoside (12)

### Reference Example 26: Methyl 4'-(2,3,4,6-tetra-O-acetyl-α-D-mannopyranosyloxy)-biphenyl-4-carboxylate (13)

### Reference Example 27: 4'-(α-D-Mannopyranosyloxy)-biphenyl-4-carboxylic acid (14)

For the preparation of compounds of formula **(I)** wherein R¹ is 3-(5-nitroindolin-1-yl) (Examples 28 to 30) the following procedure is used:

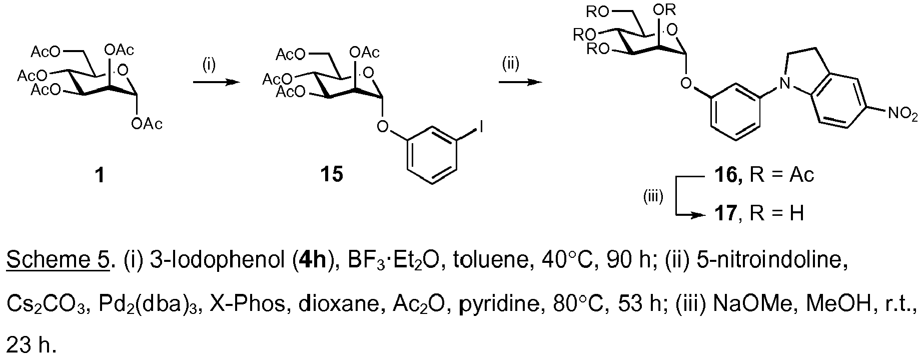

### Reference Example 28: 3-Iodophenyl 2,3,4,6-tetra-O-acetyl-α-D-mannopyranoside (15)

A heated and evacuated flask is charged with α-D-mannose pentaacetate (1, 2.00 g, 5.12 mmol, 1.0 equiv) and 3-iodophenol (**4h,** 1.36 g, 6.15 mmol, 1.2 equiv). The reagents are dissolved in dry toluene under stirring and argon atmosphere. Then BF₃-Et₂O (125 µL, 1.02 mmol, 0.2 equiv) is added dropwise. The reaction mixture is stirred for 90 h at 40°C. Ice-cold NaOH solution (1 M, 40 mL) and toluene (50 mL) are added to the reaction mixture and the phases separated. The organic layer is washed with brine (2 x 50 mL) and the aqueous layers are extracted with toluene (2 x 50mL). The organic layers are combined and dried over Na₂SO₄. Removal of the solvent by evaporation under reduced pressure leaves a residue that is purified by chromatography on silica gel (petroleum ether/EtOAc, 10:1.5 to 2:1) to obtain **15** (1.57 g, 56%).
[α]_{D}²⁰+110.1 (c = 1, CHCl₃); ¹H NMR (CDCl₃): δ 2.02, 2.16 (s, 12H, OAc), 4.04 (m, 2H, H-5, H-6a), 4.25 (m, 1 H, H-6b), 5.32 (t, *J =* 10.0 Hz, 1 H, H-4), 5.39 (dd, *J* = 1.9 Hz, 3.5 Hz, 1H, H-2), 5.47 (d, *J =* 1.8 Hz, 1H, H-1), 5.50 (dd, *J =* 3.5 Hz, 10.0 Hz, 1H, H-3), 7.02 (m, 2H, C₆H₄), 7.38 (d, *J* = 1.4 Hz, 7.3 Hz, 1H, C₆H₄), 7.47 (s, 1 H, C₆H₄); ¹³C-NMR (CDCl₃): δ 20.90, 20.99, 21.06 (4 OAc), 62.36 (C-6), 66.07 (C-4), 68.90 (C-3), 69.40, 69.51 (2C, C-2, C-5), 95.98 (C-1), 116.33, 125.80, 131.16, 132.44, 156.16 (6C, C₆H₄), 169.93, 170.11, 170.15, 170.77 (4 C=O); MS (ESI) calcd. for C₂₀H₂₃INaO₁₀ [M+Na]⁺: 573.0; found 572.9.

### Example 29: 3-(5-Nitroindolin-1-yl)phenyl 2,3,4,6-tetra-O-acetyl-α-D-mannopyranoside (16)

A dry Schlenk tube is charged with Cs₂CO₃ (266 mg, 0.816 mmol, 3 equiv). The tube is evacuated for 30 min and then flushed with argon gas and 3-iodophenyl 2,3,4,6-tetra-O-acetyl-α-D-mannopyranoside **15** (150 mg, 0.272 mmol, 1 equiv) is added to the tube, followed by Pd₂(dba)₃ (2.8 mg, 0.0027 mmol, 0.01 equiv) and X-Phos (6.5 mg, 0.0136 mmol, 0.05 equiv). The mixture is dissolved in dry dioxane (5 mL). The solvent is degassed in a ultrasonic bath for 20 min. Then 5-nitroindoline (67.3 mg, 0.41 mmol, 1.5 equiv) is added. The mixture is heated to 80°C and stirred for 53 h. TLC (petroleum ether/ EtOAc 3:1) and mass spectroscopy helps monitoring the reaction and indicates formation of partially deacetylated mannoside during the progress of the reaction. For that reason dry pyridine (2 mL) and dry acetic anhydride (1 mL) are added 50 h after reaction start to regain fully protected mannoside. Then EtOAc (30 mL) and saturated aqueous NaHCO₃ solution (50 mL) are added to the reaction mixture. The layers are separated and the organic phase is washed with brine (2 x 50 mL). The aqueous layers are extracted with EtOAc (3 x 30 mL). The combined organic layers are dried with Na₂SO₄, filtered and concentrated under reduced pressure. The residue is purified with silica gel chromatography (petroleum ether/EtOAc, gradient from 10:1 to 1:1). Compound **16** (128 mg, 80%) is obtained as an orange solid.
[α]_{D}²⁰+ 59.3 (c = 1, CHCl₃); ¹H NMR (CDCl₃): δ 2.02 (m, 9H, OAc), 2.20 (s, 3H, OAc), 3.21 (t, J = 8.5 Hz, 2H, CH₂), 4.11 (m, 4H, CH₂, H-6a, H-5), 4.26 (dd, J = 7.3 Hz, 1 H, H-6b), 5.37 (t, J = 9.9 Hz, 1H, H-4), 5.43 (s, 1H, H-2), 5.53 (m, 2H, H-1, H-3), 6.84 (d, J = 8.3 Hz, 1 H, C₆H₄), 6.98 (m, 3H, C₆H₄, C₆H₃), 7.30 (t, J = 8.1 Hz, 1 H, C₆H₄), 8.00 (s, 1 H, C₆H₃), 8.05 (d, J = 8.8 Hz, 1H, C₆H₃); ¹³C-NMR (CDCl₃): δ 20.89, 20.92, 20.94, (3 OAc), 21.12 (OAc), 27.29 (CH₂), 53.36 (CH₂), 62.30 (C-6), 66.07 (C-4), 69.01 (C-3), 69.54, 69.56 (2C, C-2, C-5), 96.00 (C-1), 106.66, 107.96, 111.47, 114.09, 121.37, 126.13,130.70 (8C, arom. C), 143.38 (1C, arom. C-O) 152.64, (1C, arom. C-N), 169.92, 170.23, 170.25, 170.71 (4 C=O); MS (ESI) calcd. for C₂₈H₃₁N₂O₁₂[M+H]⁺: 587.2; found 587.2.

### Example 30: 3-(5-Nitroindolin-1-yl)phenyl α-D-mannopyranoside (17)

Acetylated compound **16** (127 mg, 0.21 mmol) is dissolved in dry methanol (5 mL), a freshly prepared solution of sodium methoxide (0.2 mL) is added and the reaction mixture is stirred at r.t. until TLC (CH₂Cl₂/MeOH 4:1) shows disappearance of the starting material (23 h). Glacial acetic acid is used to neutralize the reaction mixture. The mixture is concentrated and purified by reversed phase chromatography (H₂O/MeOH, gradient from 100:0 to 60:40) to yield the unprotected mannoside **17** (68 mg, 60%).
[α]_{D}²⁰+105.5 (c = 1, MeOH); ¹H NMR (MeOD): δ 3.07 (t, *J* = 8.6 Hz, 2H, CH2), 3.53 (m, 1 H, H-5), 3.66 (m, 3H, H-6a, H-6b, H-4), 3.81 (dd, *J* = 3.3 Hz, 9.4 Hz, 1 H, H-3), 3.92 (dd, *J* = 1.72 Hz, 3.1 Hz, 1 H, H-2), 4.00 (t, *J =* 8.9 Hz, 2H, CH₂), 5.40 (s, 1 H, H-1), 6.79 (d, *J =* 8.2 Hz, 1 H, C₆H₄), 6.88 (m, 2H, C₆H₄, C₆H₃), 6.98 (s, 1 H, C₆H₄), 7.22 (t, *J* = 8.2 Hz, 1 H, C₆H₄), 7.85 (s, 1 H, C₆H₃), 7.89 (d, 1 H, C₆H₃); ¹³C-NMR (MeOD): δ 27.88 (1 C, CH₂), 54.34 (1C, CH₂), 62.73 (C-6), 68.38 (C-4), 71.98 (C-2), 72.42 (C-3), 75.55 (C-5), 100.27 (C-1), 107.33 , 109.34, 113.37 (3C, C₆H₄), 114.68, 121.91, 126.81 (3C, C₆H₃), 131.45 (1 C, C₆H₄), 133.70, 144.43 (2C, arom. C-N), 154.32 (1C, arom. C-O), 158.89 (1C, arom. C-N); MS (ESI) calcd. for C₂₀H₂₂N₃O₈ [M+Na]⁺: 441.1; found 441.2.

In an alternative mode of synthesis, compounds of formula **(I)** wherein R¹ is 4-(5-nitro-indolin-1-yl) (Examples 31 to 40) are prepared:

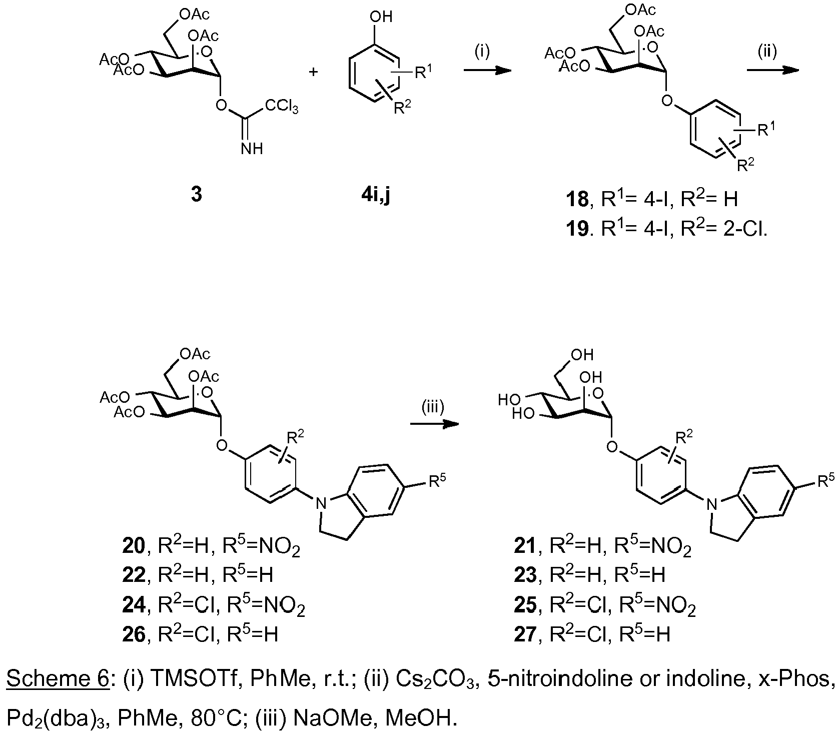

Reference Example 31: 4-Iodophenyl 2,3,4,6-tetra-*O*-acetyl-α-D-mannopyranoside (**18**) In a dry flask **3** (1.0 g, 2.03 mmol) and 4-iodophenol (**4i,** 536 mg, 2.44 mmol) are dissolved in dry toluene (6 mL) under an atmosphere of argon. TMSOTf (45.1 mg, 0.203 mmol) is added and the mixture is stirred for 2.5 h at r.t.. The reaction mixture is diluted with EtOAc (50 mL) and saturated aqueous NaHCO₃ solution (100 mL). The aqueous layers are extracted with EtOAc (2 x 100 mL). The combined organic layer is dried over Na₂SO₄ and the solvent is removed by evaporation under reduced pressure. The residue is purified by silica gel chromatography (gradient of 5%-20% EtOAc in petrol ether) to yield product **18** (2.7 g, 100%) as a white solid.
[α]_{D}²⁰+72 (c = 1, CHCl₃); ¹H NMR (CDCl₃): δ 2.02 (s, 3H, OAc), 2.04 (s, 6H, 20Ac), 2.18 (s, 3H, OAc), 4.02 (m, 2H, H-5, H-6a), 4.23 (dd, *J* = 5.6, 12.5 Hz, 1 H, H-6b), 5.32 (t, *J =* 10.2 Hz, 1H, H-4), 5.39 (dd, *J =* 1.8, 3.3 Hz, 1H, H-2), 5.45 (s, 1H, H-1), 5.49 (dd, *J =* 3.5, 10.1 Hz, 1 H, H-3), 6.84 (d, *J* = 8.9 Hz, 2H, C₆H₄), 7.56 (d, *J* = 8.9 Hz, 1 H, C₆H₄); ¹³C-NMR (CDCl₃): δ 20.86, 20.88, 21.05, (4C, 4OAc), 62.24 (C-6), 66.00 (C-4), 68.92 (C-3), 69.41, 69.48 (C-2, C-5), 86.01 (C₆H₄-I), 95.91 (C-1), 118.95, 138.69, (4C, C₆H₄), 155.57 (C₆H₄-O), 169.89, 170.11, 170.13, 170.67 (4 C=O); ESI-MS calcd. for C₂₀H₂₃INaO₁₀ [M+Na] : 573.02; found 573.02.

### Reference Example 32: 2-Chloro-4-iodophenyl 2,3,4,6-tetra-O-acetyl-α-D-mannopyranoside (19)

Following the procedure of Example 31 for the synthesis of **18,** trichloroacetimidate **3** (500 mg, 1.01 mmol) and 2-chloro-4-iodophenol (**4j,** 309 mg, 1.22 mmol) are dissolved in dry (anhydrous) toluene (7 mL) under an atmosphere of argon. TMSOTf (22.56 mg, 0.1 mmol) is added and the mixture is stirred for 2.5 h at r.t. After work-up the residue is purified by silica gel chromatography (5-30% gradient of EtOAc in petrol ether to yield **19** (430 mg, 73%) as a white solid.
[α]_{D}²⁰+60.2 (c = 0.5, CH₂Cl₂); ¹H NMR (CDCl₃): δ 2.02 (s, 3H, OAc), 2.02 (s, 3H, OAc), 2.05 (s, 3H, OAc), 2.18 (s, 3H, OAc), 4.05 (dd, *J* = 2.3, 12.2 Hz 1 H, H-6a), 4.09 (ddd, *J* = 2.3, 5.3, 10.2 Hz, 1H, H-5), 4.25 (dd, *J =* 5.3, 12.2 Hz, 1H, H-6b), 5.35 (t, *J =* 10.1 Hz, 1H, H-4), 5.48 (dd, *J =* 1.9, 3.4 Hz, 1H, H-2), 5.50 (d, *J =* 1.7 Hz, 1H, H-1), 5.55 (dd, *J =* 3.4, 10.1 Hz, 1H, H-3), 6.90 (d, *J* = 8.7 Hz, 1 H, C₆H₃), 7.48 (dd, *J* = 2.1, 8.7 Hz, 1 H, C₆H₃), 7.70 (d, *J* = 2.1 Hz, 1 H, C₆H₃); ¹³C NMR (CDCl₃): δ 20.90, 20.92, 21.08 (4C, 4OAc), 62.24 (C-6), 65.90 (C-4), 68.85 (C-3), 69.38 (C-2), 70.04 (C-5), 85.77 (1C, C₆H₃), 96.72 (C-1), 118.78, 125.80, 136.85, 138.96, 151.42 (5C, C₆H₃), 169.95, 169.97, 170.14, 170.66 (4 C=O). ESI-MS calcd. for C₂₀H₂₂ClIO₁₀[M+Na]⁺: 607.47; found 606.94.

### Example 33: 4-(5-Nitroindolin-1-yl)phenyl 2,3,4,6-tetra-O-acetyl-α-D-mannopyranoside (20)

To a mixture of 4-iodophenyl 2,3,4,6-tetra-*O*-acetyl-α-D-mannopyranoside (**18,** 200 mg, 0.37 mmol), Cs₂CO₃ (364 mg 1.12 mmol) and 5-nitroindoline (91.6 mg, 0.56 mmol) in a Schlenk tube under argon, x-Phos (9.1 mg, 0.019 mmol) and Pd₂(dba)₃ (3.85 mg, 0.0037 mmol), which had been pre-stirred for 15 min at 40°C in dry toluene (3.5 mL), are added. The reaction mixture is degassed in an ultrasonic bath and stirred 140 h at 80°C. The reaction mixture is diluted in EtOAc (50 mL) and washed with aqueous satd. NaHCO₃ solution (2 x 50-100 mL) and brine (50-100 mL). The aqueous layers are each extracted with EtOAc (2 x 50-100 mL) and the combined organic layers are dried over Na₂SO₄, filtered and concentrated under reduced pressure. **20** (163 mg, 75%) is obtained as an orange solid after silica gel chromatography (5-40% gradient of EtOAc in petrol ether).
[α]_{D}²⁰+55 (c = 1, CHCl₃); ¹H NMR (CDCl₃): δ 2.02 (s, 3H, OAc), 2.04 (s, 6H, 2OAc), 2.18 (s, 3H, OAc), 2.20 (s, 3H, OAc), 3.19 (t, *J* = 8.6 Hz, 2H, CH₂), 4.08 (m, 4H, CH₂, H-6a, H-5), 4.28 (dd, *J* = 5.2, 12.5 Hz, 1H, H-6b), 5.38 (t, *J =* 10.1 Hz, 1H, H-4), 5.44 (dd, *J* = 1.8, 3.5 Hz, 1H, H-2), 5.50 (d, *J =* 1.6 Hz, 1H, H-1), 5.55 (dd, *J* = 3.5, 10.1 Hz, 1 H, H-3), 6.73 (d, *J* = 8.9 Hz, 1 H, C₆H₄, C₆H₃) 7.13 (m, 3H, C₆H₄, C₆H₃), 7.21 (m, 1 H, C₆H₄, C₆H₃), 7.95 (s, 1 H, C₆H₃, C₆H₄), 7.98 (dd, *J* = 2.3 Hz, 8.9 Hz, 1 H, C₆H₃, C₆H₄); ¹³C-NMR (CDCl₃): δ 20.90, 20.92, 21.09, 21.65 (4OAc), 27.27 (CH₂), 53.85 (CH₂), 62.28 (C-6), 66.03 (C-4), 68.98 (C-3), 69.40 (C-2), 69.53 (C-5), 96.36 (C1), 105.52 (C₆H₄), 117.81,117.92 (C₆H₄, C₆H₃), 121.32 (C₆H₃), 122.03 (C₆H₄), 126.27 (C₆H₃), 128.40 (C₆H₃), 137.21 (Car-N), 169.90, 170.19, 170.23, 170.70 (C=O); ESI-MS calcd. for C₂₈H₃₁N₂O₁₂ [M+H]⁺: 587.19; found 587.29.

### Example 34: 4-(5-Nitroindolin-1-yl)phenyl α-D-mannopyranoside (21)

Acetylated compound **20** (218 mg, 0.37 mmol) is dissolved in dry MeOH (2 mL). A freshly prepared solution of sodium methoxide (1 M, 1 mL) is added and the reaction is stirred at r.t., until complete consumption of starting material as observed by TLC (CH₂Cl₂/MeOH 4:1). The reaction mixture is neutralized with acetic acid, concentrated and the residue purified by reversed phase chromatography (H₂O/MeOH, gradient from 100% to 80% H₂O) to yield the unprotected mannoside **21** (77.7 mg, 50%).
[α]_{D}²⁰ +57 (c = 0.1, MeOH); ¹H NMR (CD₃OD): δ 3.21 (t, *J* = 8.6 Hz, 2H, OCH₂), 3.62 (m, 1 H, H-5), 3.75 (m, 3H, H-6a, H-6b, H-4), 3.90 (dd, *J* = 3.4, 9.5 Hz, 1 H, H-3), 4.01 (t, *J* = 4.8, 13.1 Hz, 2H, CH₂), 5.48 (m, 1 H, H-1), 6.77 (m, 1 H, C₆H₃), 7.20 (d, *J* = 9.0 Hz, 2H, C₆H₄), 7.3 (d, *J =* 9.0 Hz, 2H, C₆H₄), 7.99 (m, 2H, C₆H₃); ¹³C-NMR (CD₃OD): δ 28.03, 55.07, (2CH₂), 62.86 (C-6), 68.50 (C-4), 72.13 (C-2), 72.54 (C-3), 75.62 (C-5), 100.69 (C-1), 106.41 (C₆H₃), 119.08 (2C, C₆H₄ ), 122.02 (C₆H₃), 123.52 (2C, C₆H₄), 127.14 (C₆H₃), 137.95 (Car-N), 154.98 (C=O); ESI-MS calcd. for C₂₀H₂₃N₂O₈ [M+H]⁺: 419.14; found 419.17.

### Example 35: 4-(Indolin-1-yl)phenyl 2,3,4,6-tetra-O-acetyl-α-D-mannopyranoside (22)

Following the procedure of Example 33, compound **18** (200 mg, 0.37 mmol) is reacted in a Schlenk tube with Cs₂CO₃ (364 mg 1.12 mmol), x-Phos (9.1 mg, 0.019 mmol), Pd₂(dba)₃ (3.85 mg, 0.0037 mmol) and indoline (91.6 mg, 0.56 mmol) in toluene (3.5 mL) for 140 h. Compound 22 (94.3 mg, 47%) is obtained as a white solid after chromatography on silica gel (5-20% gradient of EtOAc in petrol ether).
[α]_{D}²⁰ +39.3 (c = 1, CH₂Cl₂); ¹H NMR (CDCl₃): δ 2.02 (s, 3H, OAc), 2.04 (s, 3H, OAc), 2.04 (s, 3H, OAc), 2.18 (s, 3H, OAc), 3.10 (t, *J =* 8.4 Hz, 2H, CH₂), 3.87 (t, *J =* 9.4 Hz, 2H, CH₂), 4.09 (dd, J = 2.2, 12.2 Hz, 1 H, H-6a), 4.14 (dd, *J* = 5.3, 10.1 Hz, 1H, H-5), 4.28 (dd, *J* = 5.3, 12.2 Hz, 1H, H-6b), 5.36 (t, *J* = 10.1 Hz, 1H, H-4), 5.43 (dd, *J* = 1.8, 3.4 Hz, 1 H, H-2), 5.45 (d, *J* = 1.5 Hz, 1H, H-1), 5.55 (dd, *J* = 3.4, 10.0 Hz, 1 H, H-3), 6.71 (t, *J* = 7.3 Hz, 1 H, C₆H₃, C₆H₄), 6.95 (d, *J* = 7.9 Hz, 1 H, C₆H₃, C₆H₄), 7.00 - 7.08 (m, 3H, C₆H₃, C₆H₄), 7.11 - 7.17 (m, 3H, C₆H₃, C₆H₄); ¹³C NMR (CDCl₃): δ 20.96, 21.14 (4C, OAc), 28.42 (CH₂), 52.90 (CH₂), 62.41 (C-6), 66.23 (C-4), 69.14 (C-5), 69.27 (C-3), 69.70 (C-2), 96.62 (C-1), 107.80, 108.70, 110.20, 117.69, 118.88, 119.82, 125.22, 127.32 (12C, C₆H₃, C₆H₄), 169.99, 170.19, 170.25, 170.3 (4 C=O); ESI-MS calcd. for C₂₈H₃₁NO₁₀ [M+Na]⁺: 564.55; found 564.29.

### Example 36: 4-(Indolin-1-yl)phenyl α-D-mannopyranoside (23)

Compound **22** is dissolved in dry MeOH (3 mL), treated with NaOMe as described in Example 34, and stirred over night at r.t. to afford the deprotected compound **23** (58 mg, 37%).
[α]_{D}²⁰ +125.6 (c = 0.5, MeOH); ¹H NMR (CD₃OD): δ 2.99 (t, *J* = 8.4 Hz, 2H, CH₂), 3.64 (ddd, J = 2.4, 5.1, 9.8 Hz, 1 H, H-5), 3.68 - 3.82 (m, 5H, H-6a, H-6b, H-4, CH₂), 3.89 (dd, *J* = 3.4, 9.4 Hz, 1H, H-3), 3.99 (dd, *J* = 1.8, 3.3 Hz, 1 H, H-2), 5.39 (d, *J* = 1.4 Hz, 1 H, H-1), 6.58 - 6.67, 6.78 - 6.87, 6.88 - 6.99, 7.01 - 7.17 (m, 7H, C₆H₃, C₆H₄); ¹³C NMR (CD₃OD): δ 29.18 (CH₂), 54.02 (CH₂), 62.81 (C-6), 68.50 (C-4), 72.21 (C-2), 72.56 (C-3), 75.38 (C-5), 100.91 (C-1), 108.61, 118.96, 119.67, 121.13, 125.97, 126.80, 128.08, 132.24, 141.00, 149.39, 152.84 (12C, C₆H₃, C₆H₄); ESI-MS calcd. for C₂₀H₂₃NO₆ [M+Na]⁺: 396.4; found 396.08.

### Example 37: 2-Chloro-4-(5-nitroindolin-1-yl)phenyl 2,3,4,6-tetra-O-acetyl-α-D-mannopyranoside (24)

Following the procedure of Example 33, but using micro wave irradiation in place of heat, 2-chloro-4-iodophenyl 2,3,4,6-tetra-*O*-acetyl-α-D-mannopyranoside **19** (60 mg, 0.1 mmol) is microwave irradiated with Cs₂CO₃ (100.2 mg 0.3 mmol), x-Phos (4.9 mg, 0.01 mmol), Pd₂(dba)₃ (2.21 mg, 0.002 mmol) and 5-nitroindoline (50.5 mg, 0.3 mmol) in toluene (1 mL). Compound **24** (36 mg, 56%) is obtained as an orange solid after silica gel chromatography (0-35% gradient of EtOAc in petrol ether).
¹H NMR (CDCl₃): δ 2.03 (s, 3H, OAc), 2.05 (s, 3H, OAc), 2.06 (s, 3H, OAc), 2.19 (s, 3H, OAc), 3.20 (t, *J =* 8.6 Hz, 2H, CH₂), 4.06 (t, *J =* 9.0 Hz, 2H, CH₂), 4.10 (dd, *J* = 2.2, 12.3 Hz, 1H, H-6a), 4.21 (m, 1H, H-5), 4.28 (dd, *J* = 5.1, 12.2 Hz, 1H, H-6b), 5.39 (t, *J =* 10.1 Hz, 1 H, H-4), 5.46 - 5.54 (m, 2H, H-2, H-1), 5.59 (dd, *J* = 3.4, 10.1 Hz, 1 H, H-3), 6.82 (d, *J* = 8.9 Hz, 1 H, C₆H₃), 7.08 - 7.26 (m, 2H, C₆H₃), 7.30 (d, *J* = 2.7 Hz, 1 H, C₆H₃), 7.99 (m, 1 H, C₆H₃), 8.03 (dd, *J* = 2.3, 8.8 Hz, 1 H, C₆H₃); ¹³C NMR (CDCl₃): δ 20.92, 20.94, 21.11 (4C, 4OAc), 27.31 (CH₂), 53.70 (CH₂), 62.31 (C-6), 65.99 (C-4), 68.90 (C-5), 69.53 (C-2), 70.00 (C-3), 97.42 (C-1), 106.00, 118.45, 119.52, 121.42, 122.33, 125.72, 128.43, 129.24, 131.61, 138.24, 147.91, 152.91 (12C, 2C₆H₃), 169.94, 170.04, 170.21, 170.69 (4 C=O).

### Example 38: 2-Chloro-4-(5-nitroindolin-1-yl)phenyl α-D-mannopyranoside (25)

According to Example 34, compound **24** (36 mg, 0.058 mmol) is treated with NaOMe (475 µL) / MeOH (1 mL) and stirred over night to afford the deprotected compound 25 (16.5 mg, 63%).
¹H NMR (CD₃OD): δ 3.19 (t, *J* = 8.6 Hz, 2H, CH₂), 3.60 - 3.82 (m, 6H, CH₂, H-4, H-5, H-6), 3.94 (dd, *J* = 3.3, 9.4 Hz, 1 H, H-3), 4.08 (dd, *J* = 5.2, 13.5 Hz, 1 H, H-2), 5.47 (d, *J =* 7.7 Hz, 1 H, H-1), 6.80 (m, 1 H, C₆H₃), 7.21 (m, 1 H, C₆H₃), 7.30 - 7.43 (m, 2H, C₆H₃), 7.79-8.05 (m, 2H, C₆H₃); ¹³C NMR (CD₃OD): δ 27.93 (CH₂), 54.74 (CH₂), 62.73 (C-6), 68.27 (C-4), 71.88 (C-2), 72.40 (C-3), 76.04 (C-5), 101.25 (C-1), 106.72, 119.65, 121.31, 121.97, 123.31, 126.83, 129.03, 130.67, 132.87, 133.05, 134.69 (12C, 2C₆H₃); ESI-MS calcd. for C₂₀H₂₁ClN₂O₈ [M+Na]⁺: 475.8; found 474.96.

### Example 39: 2-Chloro-4-(indolin-1-yl)phenyl 2,3,4,6-tetra-O-acetyl-α-D-mannopyranoside (26)

Following the procedure of Example 34, compound **19** (60 mg, 0.1 mmol) is microwave irradiated with Cs₂CO₃ (100.2 mg 0.3 mmol), x-Phos (4.9 mg, 0.01 mmol), Pd₂(dba)₃ (2.21 mg, 0.002 mmol) and indoline (36.7 mg, 0.3 mmol) in toluene (1 mL). Compound **26** (25.6 mg, 43.3%) is obtained as an orange solid after silica gel chromatography (0-25% gradient of EtOAc in petrol ether).
[α]_{D}²⁰ +42.6 (c = 1, MeOH); ¹H NMR (CDCl₃): δ 1.97 (s, 3H, OAc), 2.00 (s, 3H, OAc), 2.01 (s, 3H, OAc), 2.14 (s, 3H, OAc), 3.06 (t, *J* = 8.4 Hz, 2H, CH₂), 3.82 (t, *J* = 8.5 Hz, 2H, CH₂), 4.05 (dd, *J* = 6.6, 12.2 Hz, 1 H, H-6a), 4.14 - 4.28 (m, 2H, H-5, H-6b), 5.32 (t, *J* = 10.0 Hz, 1H, H-4), 5.40 (d, *J =* 1.6 Hz, 1H, H-1), 5.48 (dd, *J* = 1.8, 3.4 Hz, 1H, H-2), 5.55 (dd, J = 3.4, 10.0 Hz, 1 H, H-3), 6.67 - 6.74, 6.94 - 7.19 (m, 7H, C₆H₃, C₆H₄); ¹³C NMR (CDCl₃): δ 20.93, 20.96 20.96, 21.13 (4OAc), 28.31 (CH₂), 52.60 (CH₂), 62.44 (C-6), 66.16 (C-4), 69.03 (C-3), 69.64 (C-2), 69.84 (C-5), 97.73 (C-1), 108.14, 117.18, 118.87, 119.53, 119.78, 125.38, 125.49, 127.37, 131.30, 140.98, 145.58, 146.82 (12C, C₆H₃, C₆H₄), 170.01, 170.02, 170.19, 170.76 (4 C=O).

### Example 40: 2-Chloro-4-(indolin-1-yl)phenyl α-D-mannopyranoside (27)

According to the procedure of Example 34, compound **26** (26 mg, 0.045 mmol) is treated with NaOMe (370 µL) / MeOH (1 mL) and stirred over night to afford the deprotected compound **27** (14.1 mg, 77%).
[a]D²⁰ +87.8 (c = 0.55, MeOH); ¹H NMR (CD₃OD): δ 3.05 (t, *J =* 8.4 Hz, 2H, CH₂), 3.59-3.88 (m, 6H, CH₂, H-4, H-5, H-6), 3.94 (dd, *J* = 3.9, 8.3 Hz, 1 H, H-3), 4.08 (dd, *J* = 1.8, 3.2 Hz, 1H, H-2), 5.39 (d, *J =* 1.5 Hz, 1 H, H-1), 6.68, 6.85-7.02, 7.05 - 7.16, 7.19, 7.31 (m, 7H, C₆H₃, C₆H₄); ¹³C NMR (CD₃OD): δ 29.29 (CH₂), 53.92 (CH₂), 63.02 (C-6), 68.60 (C-4), 72.26 (C-2), 72.70 (C-3), 76.16 (C-5), 101.97 (C-1), 109.11, 119.08, 120.50, 120.58, 120.87, 126.38, 128.35, 128.88, 130.59, 132.75, 142.02, 147.98. (12C, C₆H₃)
ESI-MS calcd. for C₂₀H₂₂ClNO₆ [M+Na]⁺: 430.84; found 430.74.

For the preparation of compounds of formula (I) wherein R¹ is 4-(1-indolyl) (Examples 41 to 58) the following procedure is used:

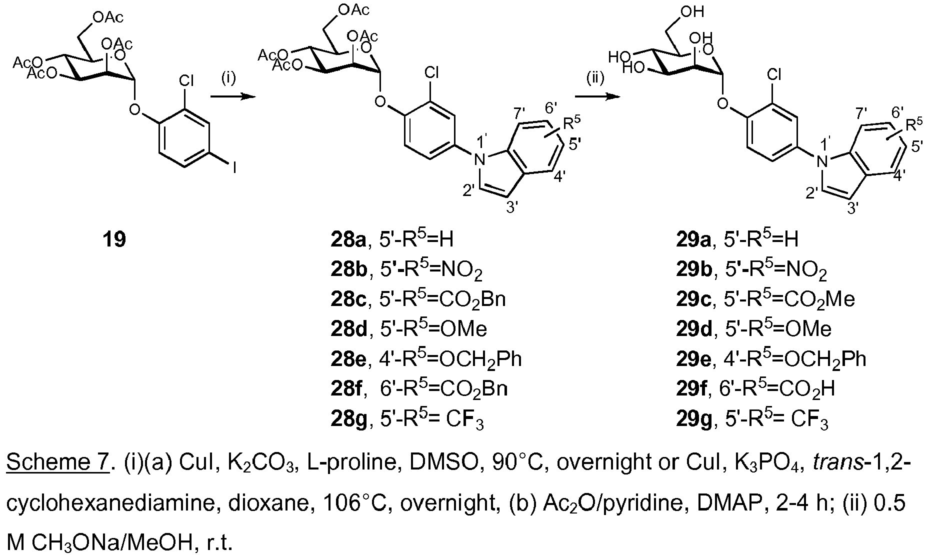

### Example 41: 2-Chloro-4-(indol-1-yl)phenyl α-D-mannopyranoside (29a)

Acording to Example 32, to a resealable Schlenk tube compound 19 (146 mg, 0.25 mmol), Cul (10 mg, 0.05 mmol), indole (35 mg, 0.3 mmol), K₂CO₃ (86 mg, 0.625 mmol), L-proline (11.5 mmol, 0.1 mmol), and a stir bar are added, and the reaction vessel fitted with a rubber septum. The vessel is twice evacuated and flashed with argon. Then DMSO is added under a stream of argon. The reaction tube is quickly sealed and the contents are stirred at 90°C overnight. The reaction mixture is cooled to r.t., diluted with ethyl acetate, and filtered through a plug of celite. The filtrate is concentrated and the resulting crude mixture acetylated with Ac₂O/pyridine (DMAP). The reaction is quenched by addition of methanol, the mixture concentrated and the residue purified by silica-gel column chromatography (petroleum ether/ethyl acetate 4:1-1:1) to provide 2-chloro-4-(indol-1-yl)phenyl 2,3,4,6-tetra-*O*-acetyl-α-D-mannopyranoside **28a** (40 mg, 28%).
**28a** (40 mg, 0.07 mmol) is dissolved in dry methanol and treated at r.t. with 0.5 M CH₃ONa/MeOH (14 µL) until completion of the reaction. The reaction mixture is neutralized with amberlyst-15, filtered, the filtrate is concentrated and the residue is purified by silica-gel column chromatography (CH₂Cl₂/MeOH 10:1) to afford the desired compound **29a** (20 mg, 70%) as a white solid.
[α]²⁰_{D} +171.6 (c = 0.18, MeOH); ¹H NMR (CD₃OD): δ 7.62-7.54 (m, 3H), 7.45-7.38 (m, 3H), 7.18 (t, *J =* 7.0 Hz, 1 H), 7.11 (t, *J =* 7.0 Hz, 1 H), 6.65 (s, 1 H), 5.61 (s, 1 H, H-1), 4.14 (m, 1 H, H-2), 4.01 (dd, *J* = 9.0, 2.5 Hz, 1 H, H-3), 3.81-3.69 (m, 4H, H-6a, H-4, H-6b, H-5); ¹³C NMR (CD₃OD): δ 151.81, 137.30, 136.22, 130.81, 128.98, 127.04, 125.58, 124.98, 123.49, 122.06, 121.42, 119.23, 111.00, 104.71 (Ar-C), 101.03 (C-1), 76.11 (C-5), 72.38 (C-3), 71.84 (C-2), 68.22 (C-4), 62.70 (C-6); ESI-MS calcd. for C₂₀H₂₀ClNO₆ [M+Na]⁺: 428.09, found 428.04.

### Example 42: 2-Chloro-4-(5-nitroindol-1-yl)phenyl α-D-mannopyranoside (29b)

Following the procedure of Example 41, starting from **19** (117 mg, 0.2 mmol), but replacing indole by 5-nitroindole, compound **29b** (54 mg, 60%) is obtained as a yellow solid.
[α]_{D}²⁰ +85.7 (c = 0.25, MeOH); ¹H NMR (CD₃OD): δ 8.63 (d, *J* = 2.0 Hz, 1 H), 8.11 (dd, *J* = 9.0, 2.0 Hz, 1 H), 7.65-7.55 (m, 4H), 7.48 (dd, *J* = 8.5, 2.5 Hz, 1 H), 6.91 (d, *J* = 3.0 Hz, 1H), 5.65(s, 1 H, H-1), 4.14 (m, 1 H, H-2), 4.01 (dd, *J* = 9.5, 3.5 Hz, 1 H, H-3), 3.83-3.72 (m, 3H, H-6a, H-4, H-6b), 3.66 (m, 1H, H-5); ¹³C NMR (CD₃OD): δ 152.73, 143.52, 140.14, 134.75, 132.99, 130.01, 127.65, 125.75, 125.57, 119.11, 118.95, 118.79, 111.51, 106.68 (Ar-C), 100.90 (C-1), 76.19 (C-5), 72.37 (C-3), 71.78 (C-2), 68.18 (C-4), 62.69 (C-6); ESI-MS calcd. for C₂₀H₁₉ClN₂O₈ [M+Na]⁺: 473.07, found 473.03.

### Example 43: Methyl 1-[3-chloro-4-(α-D-mannopyranosyloxy)phenyl)-1H-indole-5-carboxylate (29c)

To a resealable Schlenk tube compound **19** (117 mg, 0.2 mmol), Cul (3.05 mg, 0.016 mmol), benzyl indole-5-carboxylate (55 mg, 0.22 mmol), K₃PO₄ (159 mg, 0.72 mmol), *trans-1,2-cyclohexanediamine* (6.85 mg, 0.06 mmol), and a stir bar are added, and the reaction vessel fitted with a rubber septum. The vessel is twice evacuated and flashed with argon. Then 1,4-dioxane is added under a stream of argon. The reaction tube is quickly sealed and the contents stirred at 105°C overnight. The reaction mixture is cooled to r.t., diluted with ethyl acetate and filtered through a plug of celite. The filtrate is concentrated and the resulting crude mixture is acetylated with Ac₂O/pyridine (DMAP). The reaction is quenched by addition of methanol, the mixture is concentrated and the residue is purified by silica-gel column chromatography (petroleum ether/EtOAc 4:1 to 1:1) to provide **28c** (57 mg, 40%).
**28c** (19 mg, 0.027 mmol) is dissolved in dry MeOH and treated at r.t. with 0.5 M CH₃ONa/MeOH (0.15 mL) until completion of the reaction. The reaction mixture is neutralized with amberlyst-15, filtered, the filtrate is concentrated and the residue is purified by silica-gel column chromatography (CH₂Cl₂/MeOH 10:1) to afford the desired methyl ester **29c** (10 mg, 80%) as an off-white solid.
¹H NMR (DMSO): δ 8.36 (s, 1H), 7.82 (d, *J =* 8.5 Hz, 1H), 7.77 (m, 2H), 7.56 (m, 3H), 6.86 (d, *J* = 3.0 Hz, 1 H), 5.57 (s, 1 H, H-1), 3.93 (m, 1 H, H-2), 3.87 (s, 3H, OCH₃), 3.77 (dd, *J =* 9.0, 3.0 Hz, 1 H, H-3), 3.63 (m, 1 H, H-6a), 3.54 (t, *J =* 9.0 Hz, 1 H, H-4), 3.50-3.44 (m, 2H, H-6b, H-5); ESI-MS calcd. for C₂₂H₂₂ClNO₈ [M+Na]⁺: 486.09, found 486.10.

### Example 44: 2-Chloro-4-(5-methoxyindol-1-yl)phenyl 2,3,4,6-tetra-O-acetyl-α-D-manno-pyranoside (28d)

According to Example 43, starting from **19** (100 mg, 0.17 mmol), but using 5-methoxyindole instead of benzyl indole-5-carboxylate, compound **28d** (56 mg, 53%) is obtained as a white solid.
¹H NMR (CDCl₃): δ 7.55 (d, *J* = 2.5 Hz, 1 H), 7.38 (d, *J* = 9.0 Hz, 1 H), 7.33 (dd, J = 9.0, 2.5 Hz, 1 H), 7.29 (d, *J* = 9.0 Hz, 1 H), 7.22 (d, *J* = 2.5 Hz, 1 H), 7.12 (d, *J* = 2.5 Hz, 1 H), 6.89 (dd, *J* = 9.0, 2.5 Hz, 1 H), 6.60 (d, *J =* 3.0 Hz, 1 H), 5.64 (dd, *J =* 10.0, 3.5 Hz, 1 H, H-3), 5.60 (d, *J =* 1.5 Hz, 1H, H-1), 5.57 (dd, *J* = 3.5, 2.0 Hz, H-2), 5.42 (t, *J =* 10.0 Hz, 1 H, H-4), 4.31 (dd, *J =* 10.0, 5.0 Hz, 1H, H-6a), 4.23 (ddd, *J =* 10.0, 5.0, 2.0 Hz, 1H, H-5), 4.13 (dd, *J =* 12.0, 2.0 Hz, 1 H, H-6b), 3.87 (s, 3H, OMe), 2.22 (s, 3H, OAc), 2.09 (s, 3H, OAc), 2.06 (s, 6H, 20Ac); ¹³C NMR (CDCl₃): δ 170.47, 169.97, 169.80, 169.74 (4C, CH₃CO), 154.68, 149.53, 135.79, 130.96, 129.78, 128.08, 126.07, 125.24, 123.20, 117.77, 112.74, 110.90, 103.73, 102.78 (Ar-C), 96.94 (C-1), 69.82 (C-3), 69.26 (C-2), 68.68 (C-4), 65.74 (C-6), 55.80 (O*C*H₃), 20.87, 20.70, 20.69, 20.68 (4C, *C*H₃CO).

### Example 45: 2-Chloro-4-(5-methoxyindol-1-yl)phenyl α-D-mannopyranoside (29d)

Acetylated glycoside **28d** (50 mg, 0.08 mmol) is dissolved in dry methanol and treated at r.t. with 0.5 M CH₃ONa/MeOH (17 µL) until TLC control indicated the completion of the reaction. The reaction mixture is neutralized with amberlyst-15, filtered, the filtrate is concentrated and the residue is purified by silica-gel column chromatography (CH₂Cl₂/MeOH 10:1) to afford **29d** (25 mg, 69%) as a white solid.
¹H NMR (CD₃OD): δ 7.55-7.52 (m, 2H), 7.41 (dd, *J* = 9.0, 2.5 Hz, 1 H), 7.36-7.34 (m, 2H), 7.12 (d, *J* = 1.5 Hz, 1 H), 6.83 (dd, *J* = 9.0, 2.5 Hz, 1 H), 6.57 (d, *J* = 3.0 Hz, 1 H), 5.60 (s, 1H, H-1), 4.14 (m, 1H, H-2), 4.01 (dd, *J* = 9.5, 3.5 Hz, 1H, H-3), 3.82 (s, 3H, OCH₃), 3.81-3.67 (m, 4H, H-6a, H-4, H-6b, H-5); ¹³C NMR (CD₃OD): δ 156.06, 151.61, 136.41, 132.45, 131.45, 129.37, 126.65, 125.59, 124.59, 119.28, 113.47, 111.78, 104.55, 103.81 (ArC), 101.04 (C-1), 76.08 (C-5), 72.38 (C-3), 71.84 (C-2), 68.22 (C-4), 62.69 (C-6), 56.13 (OCH₃); ESI-MS calcd. for C₂₁H₂₂ClNO₇ [M+Na]⁺: 458.10, found 458.11.

### Example 46: 2-Chloro-4-(4-benzyloxyindol-1-yl)phenyl α-D-mannopyranoside (29e)

According Example 43, starting from **19** (72 mg, 0.122 mmol), but using 4-benzyloxyindole instead of indole-5-carboxylic acid benzyl ester, **29e** (20 mg, 34%) is obtained as an off-white solid.
¹H NMR (CD₃OD): δ 7.76-7.26 (m, 9H), 7.07 (m, 2H), 6.75 (d, *J* = 3.0 Hz, 1 H), 6.66 (d, *J* = 6.5 Hz, 1H), 5.61 (s, 1H, H-1), 5.22 (s, 2H, OC*H*₂Ph), 4.14 (s, 1H, H-2), 4.01 (dd, J= 9.0, 3.0 Hz, 1H, H-3), 3.83-3.67 (m, 4H, H-6a, H-4, H-6b, H-5); ¹³C NMR (CD₃OD): δ 153.85, 151.82, 139.06, 138.75, 136.30, 129.49, 128.79, 128.48, 127.49, 127.05, 125.53, 124.99, 124.43, 121.52, 119.17, 104.69, 103.09, 102.06 (ArC), 101.01 (C-1), 76.08 (C-5), 72.38 (C-3), 71.83 (C-2), 71.01 (OCH₂OPh), 68.22 (C-4), 62.69 (C-6); ESI-MS calcd. for C₂₇H₂₆ClNO₇ [M+Na]⁺: 534.13, found 534.10.

### Example 47: 1-[3-Chloro-4-(α-D-mannopyranosyloxy)phenyl)-1H-indole-6-carboxylic acid (29f)

Following the procedure of Example 43, starting from **28f** (23 mg, 0.033 mmol) but using 2 N aqueous NaOH instead of sodium methoxide for deacylation, **29f** (10 mg, 68%) is obtained as an off-white solid.
¹H NMR (CD₃OD): δ 8.16 (s, 1 H, H-1), 7.81 (d, *J* = 8.0 Hz, 1 H), 7.66 (d, *J* = 8.0 Hz, 1 H), 7.62-7.57 (m, 3H), 7.47 (m, 1H), 6.73 (s, 1H), 5.64 (s, 1H, H-1), 4.15 (s, 1H, H-2), 4.02 (dd, *J* = 9.0, 3.0 Hz, 1 H, H-3), 3.83-3.73 (m, 3H, H-6a, H-4, H-6b), 3.68 (m, 1 H, H-5); ¹³C NMR (CD₃OD): δ 173.1 (C=O), 152.27 136.87, 135.55, 134.00, 132.23, 127.42, 125.71, 125.36, 122.69, 121.52, 119.21, 113.29, 104.84 (ArC), 101.02 (C-1), 76.14 (C-5), 72.39 (C-3), 71.82 (C-2), 68.22 (C-4), 62.70 (C-6); ESI-MS calcd. for C₂₁H₂₀ClNO₈ [M+Na]⁺: 472.08, found 472.04.

### Example 48: 2-Chloro-4-(5-trifluoromethylindol-1-yl)phenyl 2,3,4,6-tetra-O-acetl-α-D-mannopyranoside (28g)

Following the procedure of Example 43, starting from **19** (146 mg, 0.25 mmol) but using 5-trifluoromethylindole instead of 5-methoxyindole, **28g** (140 mg, 87%) is obtained as off-white foam.
¹H NMR (CDCl₃): δ 7.98 (s, 1 H), 7.56 (s, 1 H), (d, *J* = 2.0 Hz, 1 H), 7.53 (d, *J* = 8.5 Hz, 1 H), 7.46 (d, *J* = 8.5 Hz, 1 H), 7.35 (m, 3H), 6.77 (d, *J =* 3.0 Hz, 1 H), 5.64 (dd, *J =* 10.0, 3.5 Hz, 1 H, H-3), 5.62 (d, *J* = 2.0 Hz, 1 H, H-1), 5.57 (dd, *J* = 3.5, 2.0 Hz, 1 H, H-2), 5.43 (t, *J =* 10.0 Hz, 1H, H-4), 4.31 (dd, *J* = 12.0, 5.0 Hz, 1H, H-6a), 4.23 (ddd, *J* = 10.0, 5.0, 2.5 Hz, 1 H, H-5), 4.15 (dd, *J =* 12.0, 2.5 Hz, 1 H, H-6b), 2.23 (s, 3H, OAc), 2.09 (s, 3H, OAc), 2.06 (s, 6H, OAc); ¹³C NMR (CDCl₃): δ 170.42, 169.97, 169.79, 169.71 (4 CO), 150.37, 137.23, 134.86, 129.51, 128.50, 126.85, 125.49, 125.10 (q, *J =* 269.87 Hz), 123.20, 122.94, 119.42 (q, *J =* 3.5 Hz), 119.00 (q, *J* = 4.4 Hz,), 117.78, 110.41, 104.75, 96.97 (C-1), 69.95 (C-5), 69.28 (C-2), 68.66 (C-3), 65.76 (C-4), 62.10 (C-6), 20.85, 20.68, 20.67 (4C, CH₃CO).

### Example 49: 2-Chloro-4-(5-trifluoromethylindol-1-yl)phenyl α-D-mannopyranoside (29g)

Acetylated glycoside **28g** (110 mg, 0.17 mmol) is dissolved in dry methanol and treated at r.t. with 0.5 M CH₃ONa/MeOH (34 µL, 0.017 mmol). The reaction mixture is neutralized with amberlyst-15, filtered, the filtrate is concentrated and the residue is purified by silica-gel column chromatography (CH₂Cl₂/MeOH 10:1) to afford **29g** (70 mg, 87%) as a white solid.
¹H NMR (CD₃OD): δ 7.97 (s, 1H), 7.61-7.55 (m, 4H), 7.45 (m, 2H), 6.80 (d, *J* = 2.5 Hz, 1 H), 5.64 (s, 1 H, H-1), 4.15 (m, 1 H, H-2), 4.01 (dd, *J* = 9.5, 3.5 Hz, 1 H, H-3), 3.83-3.73 (m, 3H, H-6a, H-4, H-6b), 3.66 (m, 1H, H-5); ¹³C NMR (CD₃OD) δ 152.41, 138.75, 135.32, 131.37, 130.14, 127.49, 126.75 (q, *J* = 268.75 Hz), 125.73, 125.41, 123.64, 120.03 (q, *J* = 3.5 Hz), 119.65 (q, *J* = 4.13 Hz), 119.20, 111.73, 105.43, 100.99 (C-1), 76.16 (C-5), 72.20 (C-3), 71.81 (C-2), 68.23 (C-4), 62.71 (C-6); ESI-MS calcd. for C₂₁H₁₉ClF₃NO₆ [M+Na]⁺: 496.08, found 496.05.

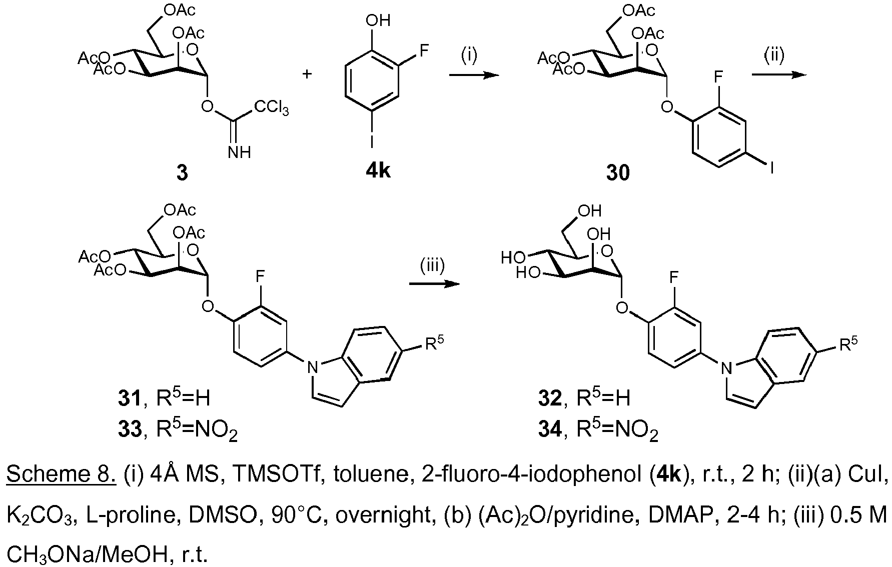

### Reference Example 50: 2-Fluoro-4-iodophenyl 2,3,4,6-tetra-O-acetyl-α-D-manno-pyranoside (30)

A mixture of imidate **3** (4.05 g, 8.22 mmol), 2-fluoro-4-iodophenol **(4k,** 2.40 g, 10.08 mmol) and 4Å molecular sieves in dry toluene is stirred at r.t. for 15 min, then TMSOTf (160 µL, 0.84 mmol) is added at r.t. The mixture is stirred for 2 h, then filtered. The filtrate is diluted with EtOAc, washed with 5% aqueous NaHCO₃, brine and dried over Na₂SO₄. The solvent is removed *in vacuo* and the residue is purified by silica-gel column chromatography (petroleum ether/EtOAc 4:1 to 2:1) to give compound **30** (4.44 g, 94%) as a white solid. ¹H NMR (CDCl₃): δ 7.46 (dd, *J =* 10.0, 2.0 Hz, 1 H), 7.38 (dt, *J* = 8.5, 2.0 Hz, 1 H), 6.94 (t, *J* = 8.5 Hz, 1 H), 5.53 (dd, *J =* 10.0, 3.5 Hz, 1 H, H-3), 5.49 (dd, *J* = 3.5, 2.0 Hz, 1 H, H-2), 5.46 (d, *J =* 2.0 Hz, 1H, H-1), 5.35 (t, *J =* 10.0 Hz, 1 H, H-4), 4.25 (dd, *J =* 12.0, 5.5 Hz, 1H, H-6a), 4.15 (ddd, *J =* 10.0, 5.5, 2.0 Hz, 1 H, H-5), 4.07 (dd, *J =* 12.0, 2.0 Hz, 1 H, H-6b), 2.19 (s, 3H, OAc), 2.06 (s, 3H, OAc), 2.031 (s, 3H, OAc), 2.026 (s, 3H, OAc); ¹³C NMR (CDCl₃): δ 170.46, 169.90, 169.77, 169.71 (4 C=O), 153.12 (d, ArC-2, *¹J*_{CF} =252.12 Hz), 143.37 (d, *²J*_{CF} =10.75 Hz, ArC-1), 133.59 (d, J=4.0 Hz, ArC), 125.98 (d, ²*J*_{CF} =20.6 Hz, ArC-3), 120.61 (ArC), 97.29 (C-1), 85.26 (d, ³*J*_{CF} =6.8 Hz, ArC-4), 69.70 (C-5), 69.09 (C-2), 68.55 (C-3), 65.70 (C-4), 62.02 (C-6), 20.83, 20.67, 20.65 (4C, *C*H₃CO).

### Example 51: 2-Fluoro-4-(indol-1-yl)phenyl α-D-mannopyranoside (32)

Following the procedure of Example 43, starting from **30** (142 mg, 0.25 mmol) the iodo group is replaced by indole and the tetraacetate **31** hydrolysed to give compound **32** (22.3 mg, 23%) as a white solid.
[α]_{D}²⁰+125.7 (c = 0.19, MeOH); ¹H NMR (CD₃OD): δ 7.61 (d, *J =* 8.0 Hz, 1 H), 7.55 (t, *J =* 9.0 Hz, 1 H), 7.50 (d, *J* = 8.0 Hz, 1 H), 7.39-7.36 (m, 2H), 7.30 (d, *J* = 9.0 Hz, 1 H), 7.17 (t, *J* = 8.0 Hz, 1 H), 7.11 (t, *J* = 7.5 Hz, 1 H), 6.64 (d, *J* = 3.0 Hz, 1 H), 5.56 (d, *J* = 1.5 Hz, 1 H, H-1), 4.12 (dd, *J =* 3.0, 1.5 Hz, 1 H, H-2), 3.95 (dd, *J* = 8.5, 3.0 Hz, 1 H, H-3), 3.77-3.72 (m, 4H, H-6a, H-4, H-6b, H-5); ¹³C NMR (CD₃OD) δ 154.70 (d, ¹J_{CF} =245.88 Hz), 143.96 (d, J=10.88 Hz), 137.22, 136.46 (d, J=8.88 Hz), 130.87, 128.97, 123.51, 122.06, 121.44, 121.35 (d, J=3.38 Hz), 121.00 (d, J=2.0 Hz), 113.74 (d, J=21.3 Hz), 111.08, 104.74 (ArC), 101.78 (C-1), 76.05 (C-5), 72.31 (C-3), 71.83 (C-2), 68.21 (C-4), 62.70 (C-6); ESI-MS calcd. for C₂₀H₂₀FNO₆ [M+Na]⁺: 412.12, found 411.97.

### Example 52: 2-Fluoro-4-(5-nitroindol-1-yl)phenyl α-D-mannopyranoside (34)

Following the procedure of Example 43, starting from **30** (114 mg, 0.2 mmol) the iodo group is replaced by 5-nitroindole and the tetraacetate **33** hydrolysed to give compound **34** (45 mg, 52%) as a yellow solid.
[α]_{D}²⁰+54.0 (c 0.44, MeOH); ¹H NMR (CD₃OD) δ 8.63 (d, *J =* 2.0Hz, 1 H), 8.11 (dd, *J* = 9.0, 2.0 Hz, 1 H), 7.65-7.58 (m, 3H), 7.44 (dd, *J* = 8.5, 2.5 Hz, 1 H), 7.34 (d, *J* = 9.0 Hz, 1 H), 6.91 (d, *J =* 3.5 Hz, 1 H), 5.59 (s, 1 H, H-1), 4.12 (m, 1 H, H-2), 3.95 (dd, *J* = 9.0, 3.5Hz, 1 H, H-3), 3.84-3.69 (m, 4H, H-6a, H-4, H-6b, H-5); ¹³C NMR (CD₃OD) δ 154.62 (d, ¹J_{CF} =252.12 Hz), 145.01 (d, J=10.63 Hz), 143.54, 140.08, 134.79 (d, J=8.88 Hz), 132.98, 130.06, 122.03 (d, J=3.50 Hz), 120.89 (d, J=1.88 Hz), 118.95, 118.81, 114.42 (d, J=21.25 Hz), 111.60, 106.71 (ArC), 101.61 (C-1), 76.13 (C-5), 72.30 (C-3), 71.77 (C-2), 68.18 (C-4), 62.70 (C-6); ESI-MS calcd. for C₂₀H₁₉FN₂O₈ [M+Na]⁺: 457.10, found 457.15.

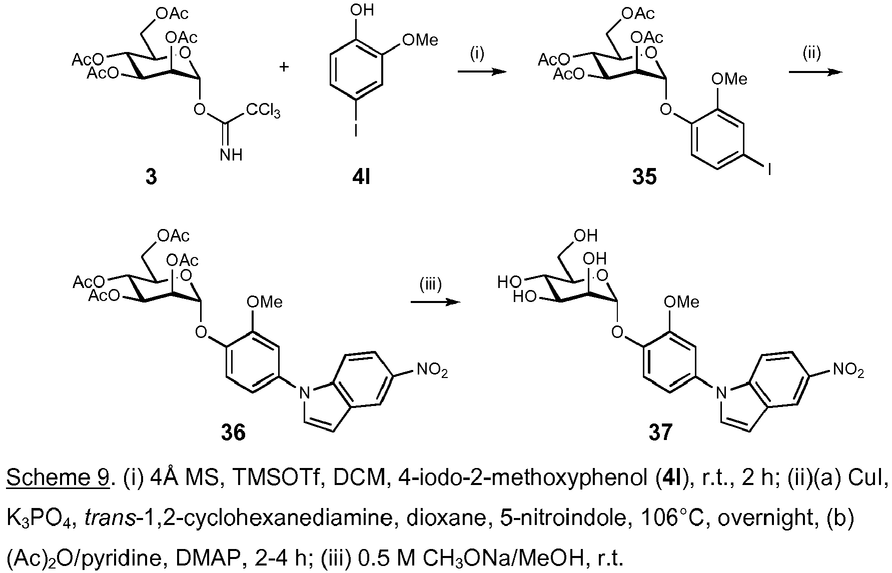

### Reference Example 53: 4-Iodo-2-methoxyphenyl 2,3,4,6-tetra-O-acetyl-α-D-manno-pyranoside (35)

Following the procedure of Example 50, imidate **3** (805 mg, 1.64 mmol) is reacted with 4-iodo-2-methoxyphenol (**4I**), 4Å molecular sieves, and TMSOTf in dry dichloromethane (in place of toluene), to give compound **35** (843 mg mg, 89%) as a white solid.
¹H NMR (CDCl₃) δ 7.21-7.17 (m, 2H), 6.82 (d, *J* = 8.0 Hz, 1 H), 5.57 (dd, *J* = 10.0, 3.5 Hz, 1 H, H-3), 5.51 (dd, *J* = 3.5, 1.5 Hz, 1 H, H-2), 5.42 (d, *J* = 1.5 Hz, 1 H, H-1), 5.34 (t, *J =* 10.0 Hz, 1H, H-4), 4.28-4.23 (m, 2H, H-6a, H-5), 4.07 (m, 1H, H-6b), 3.82 (s, 3H, OCH₃), 2.18 (s, 3H, OAc), 2.06 (s, 3H, OAc), 2.03 (s, 3H, OAc), 2.02 (s, 3H, OAc); ¹³C NMR (CDCl₃) δ 170.54, 169.93, 169.84, 169.76 (4 CO), 151.54, 144.80, 129.81, 121.74, 120.81 (ArC), 97.42 (C-1), 69.46 (C-5), 69.37 (C-2), 68.81 (C-3), 66.08 (C-4), 62.27 (C-6), 56.06 (OCH₃), 20.88, 20.70, 20.68, 20.67 (CO*C*H₃); ESI-MS calcd. for C₂₁H₂₅IO₁₁ [M+Na]⁺: 603.03, found 603.16.

### Example 54: 2-Methoxy-4-(5-nitroindol-1-yl)phenyl 2,3,4,6-tetra-O-acetyl-α-D-manno-pyranoside (36)

According to Example 43, starting from compound **35** (84 mg, 0.145 mmol) and replacing benzyl indole-5-carboxylate by 5-nitroindole, compound **36** (78 mg, 88%) is obtained as yellow solid.
¹H NMR (CDCl₃) δ 8.63 (d, *J* = 2.0 Hz, 1 H), 8.11 (dd, *J* = 9.0, 2.0 Hz, 1 H), 7.46 (d, *J* = 9.5 Hz, 1 H), 7.42 (d, *J* = 3.0 Hz, 1 H), 7.25 (m, 1 H), 6.99 (m, 2H), 6.84 (dd, *J* = 3.0, 0.5 Hz, 1H), 5.63 (dd, *J =* 10.0, 3.5 Hz, 1H, H-3), 5.57 (dd, *J* = 8.5, 2.0 Hz, 1H, H-2), 5.21 (d, *J* = 2.0 Hz, 1H, H-1), 5.40 (t, *J =* 10.0 Hz, 1H, H-4), 4.34 (m, 1H, H-5), 4.31 (m, 1H, H-6a), 4.15 (dd, *J =* 12.0, 2.0 Hz, 1H, H-6b), 3.90 (s, 3H, OCH₃), 2.21 (s, 3H, OAc), 2.08 (s, 3H, OAc), 2.06 (s, 3H, OAc), 2.05 (s, 3H, OAc); ¹³C NMR (CDCl₃) δ 170.48, 169.98, 169.89, 169.72 (4 CO), 151.72, 144.39, 142.18, 138.88, 134.80, 131.35, 128.27, 119.76, 118.29, 117.98, 116.95, 110.34, 109.58, 105.50 (ArC), 97.76 (C-1), 69.59 (C-5), 69.41 (C-2), 68.78 (C-3), 66.06 (C-4), 62.28 (C-6), 56.16 (O*C*H₃), 21.01, 20.88, 20.70, 20.68 (COCH₃); ESI-MS calcd. for C₂₉H₃₀N₂O₁₃ [M+Na]⁺: 637.16, found 637.15.

### Example 55: 2-Methoxy-4-(5-nitroindol-1-yl)phenyl α-D-mannopyranoside (37)

Compound **36** (67 mg, 0.109 mmol) is dissolved in dry methanol and treated at r.t. with 0.5 M CH₃ONa/MeOH (33 µL) until completion of the reaction. The reaction mixture is neutralized with amberlyst-15, filtered, the filtrate is concentrated and the residue is purified by silica-gel column chromatography (CH₂Cl₂/MeOH 10:1) to afford compound 37 (42 mg, 86%) as a yellow solid.
¹H NMR (CD₃OD) δ 8.62 (d, *J* = 2.0 Hz, 1 H), 8.08 (dd, *J* = 9.5, 2.0 Hz, 1 H), 7.64 (d, *J* = 3.5 Hz, 1 H), 7.57 (d, *J* = 9.0 Hz, 1 H), 7.41 (d, *J* = 8.5 Hz, 1 H), 7.16 (d, *J* = 2.0 Hz, 1 H), 7.07 (dd, *J* = 8.5, 2.5 Hz, 1 H), 6.89 (d, *J =* 3.0 Hz, 1 H), 5.50 (s, 1 H, H-1), 4.13 (d, *J =* 1.5 Hz, 1 H, H-2), 3.97 (dd, *J* = 9.0, 3.5 Hz, 1 H, H-3), 3.90 (s, 3H, OMe), 3.83-3.73 (m, 4H, H-5, H-4, H-6a, H-6b); ¹³C NMR (CD₃OD) δ 152.99, 146.56, 143.35, 140.30, 135.37, 133.22, 129.90, 120.56, 118.89, 118.58, 118.09, 111.70, 110.72, 106.26 (ArC), 101.74 (C-1), 75.82 (C-5), 72.41 (C-2), 71.97 (C-3), 68.37 (C-4), 62.77 (C-6), 56.76 (OCH₃); ESI-MS calcd. for C₂₁H₂₂N₂O₉ [M+Na]⁺: 469.12, found 469.19.

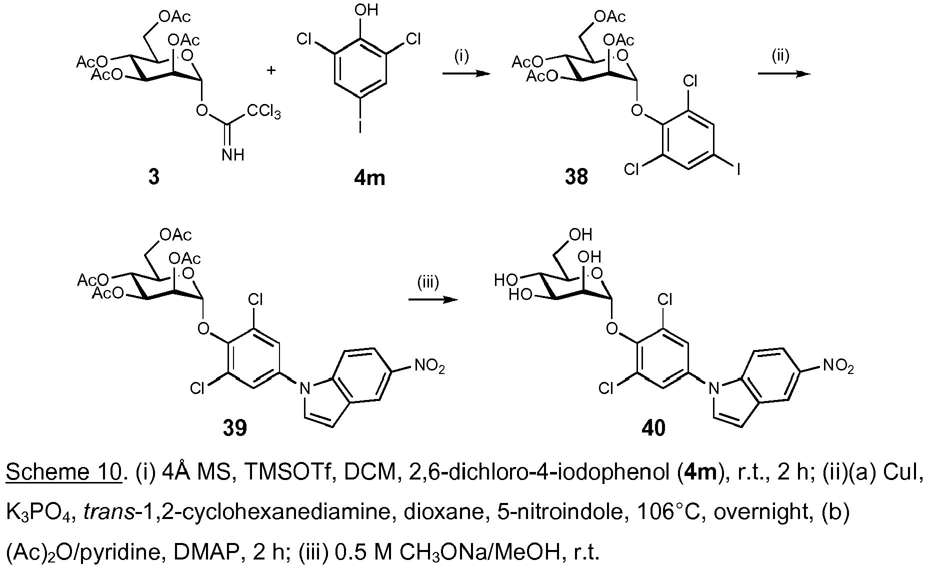

### Reference Example 56: 2,6-Dichloro-4-iodophenyl 2,3,4,6-tetra-O-acetyl-α-D-mannopyranoside (38)

Following the procedure of Example 50, imidate **3** (1.37 g, 2.85 mmol) is reacted with 2,6-dichloro-4-iodophenol **(4m,** 988 mg, 3.42 mmol), 4Å molecular sieves, and TMSOTf (77 µL, 0.428 mmol) in dry CH₂Cl₂ to yield compound **38** (647 mg, 37%) as a white solid.
¹H NMR (CDCl₃) δ 7.46 (s, 2H), 5.76 (dd, *J =* 3.0, 2.0 Hz, 1 H, H-2), 5.58 (dd, *J =* 10.0, 3.0 Hz, 1H, H-3), 5.41-5.36 (m, 2H, H-4, H-1), 4.68 (ddd, *J =* 10.0, 5.0, 2.5 Hz, 1 H, H-5), 4.28 (dd, *J* = 12.5, 5.0 Hz, 1H, H-6a), 4.17 (dd, *J* = 12.5, 2.5 Hz, 1H, H-6b), 2.18 (s, 3H, OAc), 2.08 (s, 6H, OAc), 2.03 (s, 3H, OAc); ¹³C NMR (CDCl₃) δ 170.60, 169.82, 169.71, 169.65 (4 CO), 149.64, 137.61, 129.84 (ArC), 101.04 (C-1), 70.84 (C-5), 69.20 (C-2), 68.60 (C-3), 65.68 (C-4), 62.25 (C-6), 20.82, 20.72, 20.71, 20.65 (CO*C*H₃); ESI-MS calcd. for C₂₀H₂₁Cl₂IO₁₀ [M+Na]⁺: 640.95, found 641.06.

### Example 57: 2,6-Dichloro-4-(5-nitroindol-1-yl)phenyl 2,3,4,6-tetra-O-acetyl-α-D-manno-pyranoside (39)

According to Example 43, starting from **38** (124 mg, 0.2 mmol) and replacing benzyl indole-5-carboxylate by 5-nitroindole, **39** (74 mg, 57%) is obtained as yellow solid.
¹H NMR (CDCl₃) δ 8.63 (d, *J* = 2.5 Hz, 1 H), 8.17 (dd, *J* = 9.5, 2.5 Hz, 1 H), 7.50 (m, 3H), 7.40 (d, *J* = 2.0 Hz, 1 H), 6.88 (dd, *J* = 3.0, 0.5 Hz, 1 H), 5.83 (dd, *J* = 3.5, 2.0 Hz, 1 H, H-2), 5.64 (dd, *J =* 10.0, 3.0 Hz, 1H, H-3), 5.47-5.42 (m, 2H, H-1, H-4), 4.76 (ddd, *J =* 10.0, 5.0, 2.5 Hz, 1H, H-5), 4.32 (dd, *J* = 12.5, 5.0 Hz, 1 H, H-6a), 4.24 (dd, *J* = 12.5, 2.5 Hz, 1 H, H-6b), 2.21 (s, 3H, OAc), 2.11 (s, 3H, OAc), 2.10 (s, 3H, OAc), 2.06 (s, 3H, OAc); ¹³C NMR (CDCl₃) δ 170.59, 169.89, 169.72, 169.71 (4 CO), 148.95, 142.68, 138.38, 135.99, 130.60, 130.27, 128.68, 125.23, 118.60, 118.43, 110.16, 106.73 (ArC), 101.40 (C-1), 71.00 (C-5), 68.58 (C-2), 65.68 (C-3), 62.26 (C-4), 60.37 (C-6), 20.84, 20.74, 20.72, 20.66 (CO*C*H₃); ESI-MS calcd. for C₂₈H₂₆Cl₂N₂O₁₂ [M+Na]⁺: 675.08, found 675.13.

### Example 58: 2,6-Dichloro-4-(5-nitroindol-1-yl)phenyl α-D-mannopyranoside (40)

Compound **39** (57.2 mg, 0.0875 mmol) is dissolved in dry methanol (4 mL) and treated at r.t. with K₂CO₃ (12 mg, 0.0875 mmol) until completion of the reaction. The reaction mixture is concentrated and the residue is purified by silica-gel column chromatography (CH₂Cl₂/MeOH 10:1) to afford compound **40** (21 mg, 50%) as a yellow solid.
¹H NMR (CD₃OD) δ 8.63 (d, *J* = 2.0 Hz, 1 H), 8.14 (dd, *J* = 9.0, 2.0 Hz, 1 H), 7.70 (s, 2H), 7.68 (d, *J* = 3.5 Hz, 1 H), 7.62 (d, *J* = 9.0 Hz, 1 H), 6.94 (d, *J* = 3.0 Hz, 1 H), 5.51 (d, *J* = 1.5 Hz, 1 H, H-1), 4.39 (dd, *J* = 8.5, 2.0 Hz, 1 H, H-2), 4.27 (dt, *J* = 10.0, 3.5 Hz, 1 H, H-5), 3.99 (dd, *J* = 9.5, 3.5 Hz, 1H, H-3), 3.87 (d, *J =* 10.0 Hz, 1H, H-4), 3.83 (m, 2H, H-6a, H-6b); ¹³C NMR (CD₃OD) δ 150.84, 143.85, 139.87, 136.99, 132.73, 131.30, 130.34, 126.57, 119.15, 118.98, 111.58, 107.35 (ArC), 106.48 (C-1), 77.06 (C-5), 72.22 (C-3), 71.93 (C-2), 67.79 (C-4), 62.58 (C-6); ESI-MS calcd. for [M+Na]⁺: 507.03, found 507.16.

For the preparation of compounds of formula (**I**) wherein R¹ is 4-(7-aza-5-chloroindol-1-yl) and is 4-(7-aza-5-cyanoindol-1-yl) (Examples 59-62) the following procedure is used:

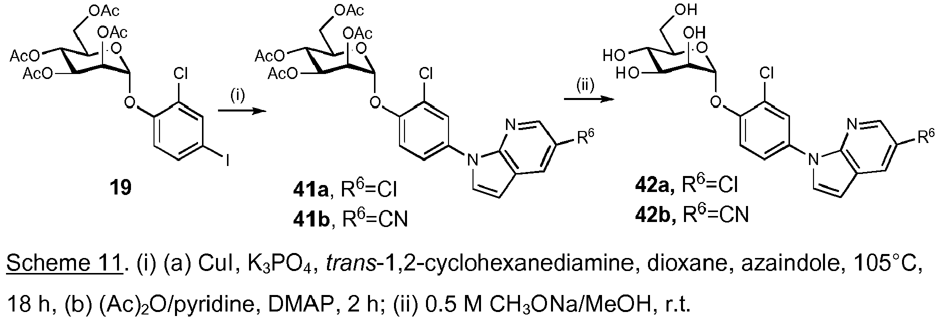

### Example 59: 2-Chloro-4-(5-chloro-1H-pyrrolo[2,3-b]pyridin-1-yl)phenyl 2,3,4,6-tetra-O-acetyl-α-D-mannopyranoside (41 a)

To a resealable Schlenk tube compound **19** (88 mg, 0.15 mmol), Cul (2.3 mg, 0.012 mmol), 5-chloro-1*H*-pyrrolo[2,3-*b*]pyridine (27 mg, 0.18 mmol), K₃PO₄ (133 mg, 0.6 mmol), *trans-1,2-cyclohexanediamine* (7.0 mg, 0.06 mmol), and a stir bar are added, and the reaction vessel is fitted with a rubber septum. The vessel is twice evacuated and flashed with argon. Then 1,4-dioxane (1.5 mL) is added under a stream of argon. The reaction tube is quickly sealed and the contents are stirred at 105°C overnight. The reaction mixture is cooled to r.t., diluted with ethyl acetate, filtered through a plug of celite, eluting with additional ethyl acetate. The filtrate is concentrated and the resulting crude mixture is acetylated with Ac₂O/pyridine (DMAP). The reaction is quenched by addition of methanol, concentrated and the residue is purified by silica-gel column chromatography (petroleum ether/EtOAc 4:1 to 1:1) to provide **41a** (74.5 mg, 82%) as white solid.
¹H NMR (CDCl₃): δ 8.28 (d, *J* = 2.0 Hz, 1 H), 7.93 (d, *J* = 2.0 Hz, 1 H), 7.81 (d, *J* = 2.5 Hz, 1 H), 7.60 (dd, *J* = 9.0, 2.5 Hz, 1 H), 7.47 (d, *J* = 3.5 Hz, 1 H), 7.31 (d, *J* = 9.0 Hz, 1 H), 6.59 (d, *J* = 3.5 Hz, 1H), 5.63 (dd, *J* = 10.0, 3.5 Hz, 1H, H-3), 5.59 (d, *J* = 1.5 Hz, 1H, H-1), 5.56 (dd, *J* = 3.5, 2.0 Hz, 1H, H-2), 5.41 (t, *J =* 10.0 Hz, 1H, H-4), 4.31 (dd, *J* = 12.5, 5.0 Hz, 1H, H-6b), 4.21 (ddd, *J =* 10.0, 5.0, 2.0 Hz, 1 H, H-5), 4.11 (dd, *J =* 12.0, 2.5 Hz, 1 H, H-6b), 2.22 (s, 3H, OAc), 2.08 (s, 3H, OAc), 2.06 (s, 3H, OAc), 2.05 (s, 3H, OAc); ¹³C NMR (CDCl₃): δ 170.49, 169.94, 169.77, 169.73 (4 CO), 149.75, 145.60, 142.32, 133.84, 128.98, 128.37, 125.49, 125.02, 124.88, 123.09, 122.20, 117.46, 101.63, 96.88 (C-1), 69.85 (C-5), 69.29 (C-2), 68.73 (C-3), 65.77 (C-4), 62.06 (C-6), 20.85, 20.69, 20.68, 20.66 (CO*C*H₃); ESI-MS calcd. for C₂₇H₂₆Cl₂N₂O₁₀ [M+Na]⁺: 631.09, found 631.32.

### Example 60: 1-[4-(2,3,4,6-Tetra-O-acetyl-α-D-mannopyranosyloxy)-3-chlorophenyl]-1H-pyrrolo[2,3-b]pyridine-5-carbonitrile (41b)

To a resealable Schlenk tube **19** (146.2 mg, 0.25 mmol), Cul (3.81 mg, 0.02 mmol), 1H-pyrrolo[2,3-*b*]pyridine-5-carbonitrile (39 mg, 0.0.275 mmol), K₃PO₄ (221 mg, 1.0 mmol), *trans*-1,2-cyclohexanediamine (8.6 mg, 0.075 mmol), and a stir bar are added, and the reaction vessel is fitted with a rubber septum. The vessel is twice evacuated and flashed with argon. Then 1,4-dioxane (2.0 mL) is added under a stream of argon. The reaction tube is quickly sealed and the contents are stirred at 105°C overnight. The reaction mixture is cooled to r.t., diluted with ethyl acetate and filtered through a plug of celite. The filtrate is concentrated and the resulting crude mixture is acetylated with Ac₂O/pyridine (DMAP). The reaction is quenched by addition of methanol, the mixture is concentrated and the residue is purified by silica-gel column chromatography (petroleum ether/EtOAc 2:1 to 3:2) to provide **41 b** (120 mg, 80%) as white solid.
¹H NMR (CDCl₃): δ 8.57 (d, *J* = 2.0 Hz, 1 H), 8.25 (d, *J* = 2.0 Hz, 1 H), 7.78 (d, *J* = 2.5 Hz, 1 H), 7.58 (m, 2H), 7.33 (d, *J* = 8.5 Hz, 1 H), 6.73 (d, *J* = 3.5 Hz, 1 H), 5.62 (dd, *J* = 10.0, 3.5 Hz, 1H, H-3), 5.60 (s, 1H, H-1), 5.54 (dd, J= 3.5, 2.0 Hz, 1H, H-2), 5.41 (t, *J =* 10.0 Hz, 1 H, H-4), 4.29 *(dd, J =* 12.0, 5.0 Hz, 1 H, H-6b), 4.18 (ddd, *J =* 10.0, 5.0, 2.0 Hz, 1 H, H-5), 4.10 (m, 1 H, H-6b), 2.21 (s, 3H, OAc), 2.07 (s, 3H, OAc), 2.05 (s, 3H, OAc), 2.04 (s, 3H, OAc); ¹³C NMR (CDCl₃): δ 170.39, 169.88, 169.72, 169.64 (4 CO), 150.26, 147.74, 146.25, 133.19, 132.92, 130.19, 126.36, 125.06, 123.46, 120.64, 118.26, 117.32, 102.77, 102.28, 96.76 (C-1), 69.86 (C-5), 69.18 (C-2), 68.63 (C-3), 65.66 (C-4), 61.98 (C-6), 20.79, 20.63, 20.61, 20.60 (CO*C*H₃); ESI-MS calcd. for C₂₈H₂₆ClN₃O₁₀ [M+Na]⁺: 622.12, found 622.10.

### Example 61: 2-Chloro-4-(5-chloro-1H-pyrrolo[2,3-b]pyridin-1-yl)phenyl α-D-manno-pyranoside (42a)

To a solution **of 41a** (74.5 mg, 0.122 mmol) in dry methanol (2 mL) 0.5 M CH₃ONa/MeOH (24 µL) is added at r.t. and then stirred for 2 h. The reaction mixture is neutralized with amberlyst-15, filtered, the filtrate is concentrated and the residue is purified by silica-gel column chromatography (CH₂Cl₂/MeOH 10:1) to afford **42a** (34 mg, 63%) as white solid.
¹H NMR (CD₃OD): δ 8.23 (d, *J* = 2.5 Hz, 1 H), 8.06 (d, *J* = 2.5 Hz, 1 H), 7.90 (d, *J* = 2.5 Hz, 1 H), 7.73 (d, *J* = 3.5 Hz, 1 H), 7.63 (dd, *J* = 8.5, 2.5 Hz, 1 H), 7.52 (d, *J* = 9.0 Hz, 1 H), 6.66 (d, *J =* 3.5 Hz, 1 H), 5.60 (d, *J =* 1.5 Hz, 1 H, H-1), 4.14 (dd, *J =* 3.0, 1.5 Hz, 1 H, H-2), 4.01 (dd, *J* = 9.0, 3.0 Hz, 1 H, H-3), 3.82-3.72 (m, 3H, H-6a, H-4, H-6b), 3.68 ( m, 1 H, H-5); ¹³C NMR (CD₃OD): δ 151.88, 146.85, 142.64, 134.37, 131.20, 129.67, 126.78, 125.81, 125.22, 124.44, 124.08, 118.82, 102.61, 101.02 (C-1), 76.06 (C-5), 72.40 (C-3), 71.84 (C-2), 68.22 (C-4), 62.67 (C-6); ESI-MS calcd. for C₁₉H₁₈Cl₂N₂O₆ [M+Na]⁺: 463.04, found 463.02.

### Example 62: 1-[3-Chloro-4-(α-D-mannopyranosyloxy)phenyl]-1H-pyrrolo[2,3-b]pyridine-5-carbonitrile (42b)

To a solution **of 41 b** (112 mg, 0.187 mmol) in dry methanol (4 mL) 0.5 M CH₃ONa/MeOH (40 µL) is added at r.t. and then stirred for 2 h. The reaction mixture is neutralized with amberlyst-15, filtered, the filtrate is concentrated and the residue is purified by silica-gel column chromatography (CH₂Cl₂/MeOH 10:1) to afford **42b** (73 mg, 91%) as white solid. ¹H NMR (CD₃OD): δ 8.58 (d, *J* = 2.0 Hz, 1 H), 8.45 (d, *J* = 2.0 Hz, 1 H), 7.91 (d, *J* = 2.5 Hz, 1 H), 7.86 (d, *J* = 4.0 Hz, 1 H), 7.65 (dd, *J* = 9.0, 2.5 Hz, 1 H), 7.54 (d, *J* = 9.0 Hz, 1 H), 6.82 (d, *J* = 4.0 Hz, 1 H), 5.62 (d, *J =* 1.5 Hz, 1 H, H-1), 4.14 (dd, *J* =3.5, 2.0 Hz, 1 H, H-2), 4.01 (dd, *J* =9.5, 3.5 Hz, 1H, H-3), 3.82-3.72 (m, 3H, H-6a, H-4, H-6b), 3.66 (ddd, *J* = 10.0, 5.5, 2.5 Hz, 1H, H-5); ¹³C NMR (CD₃OD): δ 152.28, 149.09, 147.03, 134.69, 133.72, 132.29, 127.15, 125.23, 124.83, 122.49, 119.27, 118.72, 103.79, 103.20, 100.97 (C-1), 76.10 (C-5), 72.40 (C-3), 71.81 (C-2), 68.21 (C-4), 62.67 (C-6); ESI-MS calcd. for C₂₀H₁₈ClN₃O₆ [M+Na]⁺: 454.08, found 454.07.

For the preparation of compounds of (**I**) wherein R¹ is 4-(7-aza-5-indol-5-yl) (Examples 63-64) the following procedure is used:

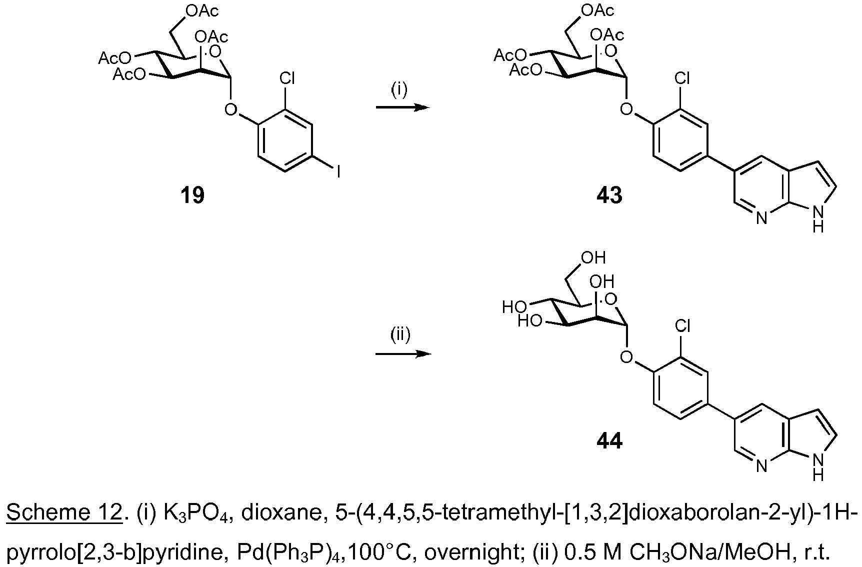

### Example 63: 2-Chloro-4-(1H-pyrrolo[2,3-b]pyridin-5-yl)phenyl 2,3,4,6-tetra-O-acetyl-α-D-mannopyranoside (43)

To a resealable Schlenk tube compound 19 (88 mg, 0.15 mmol), Pd(Ph₃P) (5.2 mg, 0.0045 mmol), 5-(4,4,5,5-tetramethyl-[1,3,2]dioxaborolan-2-yl)-1*H*-pyrolo-[2,3-*b*]pyridine (40.3 mg, 0.165 mmol), K₃PO₄ (49.8 mg, 0.225 mmol), and a stir bar are added, and the reaction vessel is fitted with a rubber septum. The vessel is twice evacuated and flashed with argon. Then dioxane (1.5 mL) is added under a stream of argon. The reaction tube is quickly sealed and the contents are stirred at 100°C overnight. The reaction mixture is cooled to r.t., diluted with ethyl acetate, and filtered through a plug of celite. The filtrate is concentrated and the residue is purified by chromatography on silica gel (petrol ether/ EtOAc 3:1 to 1:1) to afford **43** (48 mg, 56%) as yellow solid.
¹H NMR (CDCl₃): δ 9.74 (s, 1 H, NH), 8.49 (d, *J* = 2.0 Hz, 1 H), 8.07 (d, *J* = 2.0 Hz, 1 H), 7.46 (m, 2H), 7.39 (dd, *J* = 3.5, 2.5 Hz, 1 H), 7.27 (d, *J* = 8.0 Hz, 1 H), 6.64 (d, *J* = 4.0 Hz, 1 H), 6.56 (dd, *J* = 3.5, 2.5 Hz, 1 H), 5.66 (dd, *J =* 10.0, 3.5 Hz, 1 H, H-3), 5.61 (d, *J =* 1.5 Hz, 1H, H-1), 5.57 (dd, *J =* 3.5, 1.5 Hz, 1H, H-2), 5.41 (t, *J =* 10.0 Hz, 1H, H-4), 4.31 (dd, *J* = 12.0, 5.0 Hz, 1H, H-6b), 4.24 (ddd, *J* = 12.0, 5.0, 2.0 Hz, 1 H, H-5), 4.13 (m, 1 H, H-6b), 3.08 (s, 3H, NHAc), 2.22 (s, 3H, OAc), 2.08 (s, 3H, OAc), 2.05 (s, 3H, OAc), 2.04 (s, 3H, OAc); ¹³C NMR (CDCl₃): δ 170.49, 169.97, 169.78 (4 CO), 150.37, 148.15, 141.99, 128.54, 128.44, 127.85, 127.10, 126.51, 125.91, 124.86, 120.19, 117.51, 101.32, 96.77 (C-1), 69.77 (C-5), 69.36 (C-2), 68.79 (C-3), 65.86 (C-4), 62.13 (C-6), 21.03, 20.86, 20.70, 20.67 (COCH₃).

### Example 64: 2-Chloro-4-(1H-pyrrolo[2,3-b]pyridin-5-yl)phenyl α-D-mannopyranoside (44)

To a solution of **43** (50 mg, 0.087 mmol) in dry methanol (2 mL 0.5 M CH₃ONa/MeOH (0.18 mL) is added at r.t. and then stirred for another 2.5 h. The reaction mixture is neutralized with amberlyst-15, filtered, the filtrate is concentrated and the residue is purified by silica-gel column chromatography (CH₂Cl₂/MeOH 10:1 to 7:1) to afford **44** (14.8 mg, 42%) as a white solid.
¹H NMR (CD₃OD): δ 8.39 (d, *J* = 1.5 Hz, 1 H), 8.17 (d, *J* = 1.5 Hz, 1 H), 7.69 (d, *J* = 2.0 Hz, 1 H), 7.55 (dd, *J* = 8.5, 2.0 Hz, 1 H), 7.46 (d, *J* = 8.5 Hz, 1 H), 7.43 (d, *J* = 3.5 Hz, 1 H), 6.55 (d, *J =* 3.0 Hz, 1H), 5.59 (s, 1 H, H-1), 4.13 (m, 1 H, H-2), 4.01 (dd, *J* = 9.5, 3.5 Hz, 1 H, H-3), 3.82-3.72 (m, 3H, H-6a, H-4, H-6b), 3.69 (m, 1H, H-5); ¹³C NMR (CD₃OD): δ 152.52, 148.84, 142.00, 136.19, 129.73, 129.09, 128.33, 127.94, 127.76, 125.39, 122.32, 118.93, 101.79, 100.89 (C-1), 75.97 (C-5), 72.42 (C-3), 71.90 (C-2), 68.25 (C-4), 62.68 (C-6); ESI-MS calcd. for C₁₉H₁₉ClN₂O₆ [M+Na]⁺: 429.08, found 429.04, calcd. for [M+H]⁺407.10, found 407.01.

For the preparation of compounds of (**I**) wherein R¹ is 4-(3*H*-imidazo[4,5-*b*]pyridin-6-yl) (Examples 65-66) the following procedure is used:

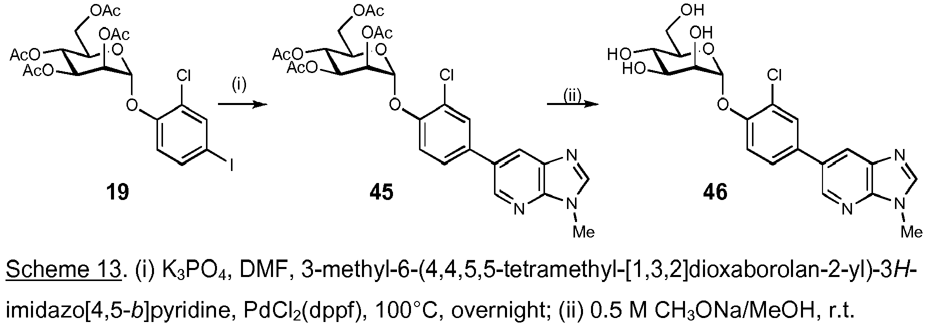

### Example 65: 2-Chloro-4-(3-methyl-3H-imidazo[4,5-b]pyridin-6-yl)phenyl 2,3,4,6-tetra-O-acetyl-α-D-mannopyranoside 45

To a resealable Schlenk tube compound **19** (88 mg, 0.15 mmol), PdCl₂(dppf) (3.67 mg, 0.0045 mmol), 3-methyl-8-(4,4,5,5-tetramethyl-[1,3,2]dioxaborolan-2-yl)-3H-imidazo[4,5-b]pyridine (42.8 mg, 0.165 mmol), K₃PO₄ (49.8 mg, 0.225 mmol), and a stir bar are added, and the reaction vessel is fitted with a rubber septum. The vessel is twice evacuated and flashed with argon. Then DMF (0.9 mL) is added under a stream of argon. The reaction tube is quickly sealed and the content stirred at 100°C overnight. The reaction mixture is cooled to r.t., diluted with ethyl acetate, and filtered through a plug of celite, eluting with additional ethyl acetate. The filtrate is concentrated and the residue is purified with chromatography on silica gel with CH₂Cl₂/MeOH 20:1 to 15:1 to afford **45** (85 mg, 96%) as a brown solid.
¹H NMR (CDCl₃): δ 8.57 (d, *J* = 2.0 Hz, 1 H), 8.16 (d, *J* = 2.0 Hz, 1 H), 8.08 (s, 1 H), 8.01 (s, 1 H), 7.65 (d, *J* = 2.5 Hz, 1 H), 7.44 (dd, *J* = 8.5, 2.5 Hz, 1 H), 7.28 (d, *J* = 8.5 Hz, 1 H), 5.64 (dd, *J* = 10.0, 3.5 Hz, 1H, H-3), 5.61 (d, *J* = 2.0 Hz, 1 H, H-1), 5.56 (dd, *J* = 3.5, 2.0 Hz, 1 H, H-2), 5.40 (t, *J* = 10.0 Hz, 1 H, H-4), 4.29 (dd, *J* = 12.0, 5.0 Hz, 1 H, H-6b), 4.21 (ddd, *J* = 10.0, 5.0, 2.0 Hz, 1 H, H-5), 4.11 (m, 1 H, H-6b), 3.95 (s, 3H, NCH₃), 2.21 (s, 3H, OAc), 2.07 (s, 3H, OAc), 2.04 (s, 3H, OAc), 2.03 (s, 3H, OAc); ¹³C NMR (CDCl₃): δ 170.45, 169.94, 169.76, 169.75 (4 CO), 150.70, 147.11, 145.53, 143.32, 135.41, 135.03, 130.37, 129.47, 126.74, 126.01, 124.96, 117.51, 96.68 (C-1), 69.78 (C-5), 69.30 (C-2), 68.75 (C-3), 65.81 (C-4), 62.09 (C-6), 29.87 (NCH₃), 20.84, 20.68, 20.65, 20.64 (COCH₃); ESI-MS calcd. for C₂₇H₂₈ClN₃O₁₀ [M+Na]⁺: 612.14, found 612.20, calcd. for [M+H]⁺590.15, found 590.15.

### Example 66: 2-Chloro-4-(3-methyl-3H-imidazo[4,5-b]pyridin-6-yl)phenyl α-D-manno-pyranoside (46)

To a solution of **45** (105 mg, 0.178 mmol) in dry methanol (2 mL) 0.5 M CH₃ONa/MeOH (35 µL) is added at r.t. and then stirred for another 2 h. The reaction mixture is neutralized with amberlyst-15, filtered, the filtrate is concentrated and the residue is purified by silica-gel column chromatography (CH₂Cl₂/MeOH 10:1 to 6:1) to afford **46** (28 mg, 37%) as a white solid.
¹H NMR (CD₃OD): δ 8.63 (d, *J* = 1.5 Hz, 1 H), 8.39 (s, 1 H), 8.22 (d, *J* = 2.0 Hz, 1 H), 7.74 (d, *J* = 2.0 Hz, 1 H), 7.59 (dd, *J* = 8.5, 2.0 Hz, 1 H), 7.50 (d, *J* = 8.5 Hz, 1 H), 5.61 (d, *J* = 1.5 Hz, 1H, H-1), 4.13 (dd, *J* = 3.5, 1.5 Hz, 1H, H-2), 4.01 (dd, *J* = 9.5, 3.5 Hz, 1 H, H-3), 3.96 (s, 3H, NC*H*₃), 3.82-3.72 (m, 3H, H-6a, H-4, H-6b), 3.67 ( m, 1H, H-5); ¹³C NMR (CD₃OD): δ 152.96, 147.98, 144.37, 136.09, 134.95, 130.02, 128.14, 126.37, 125.43, 118.96, 100.83 (C-1), 76.12 (C-5), 72.39 (C-3), 71.81 (C-2), 68.20 (C-4), 62.64 (C-6), 30.29 (N*C*H₃); ESI-MS calcd. for C₁₉H₂₀ClN₃O₆ [M+Na]⁺: 444.09, found 444.04, calcd. for [M+H]⁺ 422.11, found 422.08.

For the preparation of compounds of formula (**I**) wherein R¹ is 4-(1-indolyl) (Examples 67 to 71) the following procedure is used:

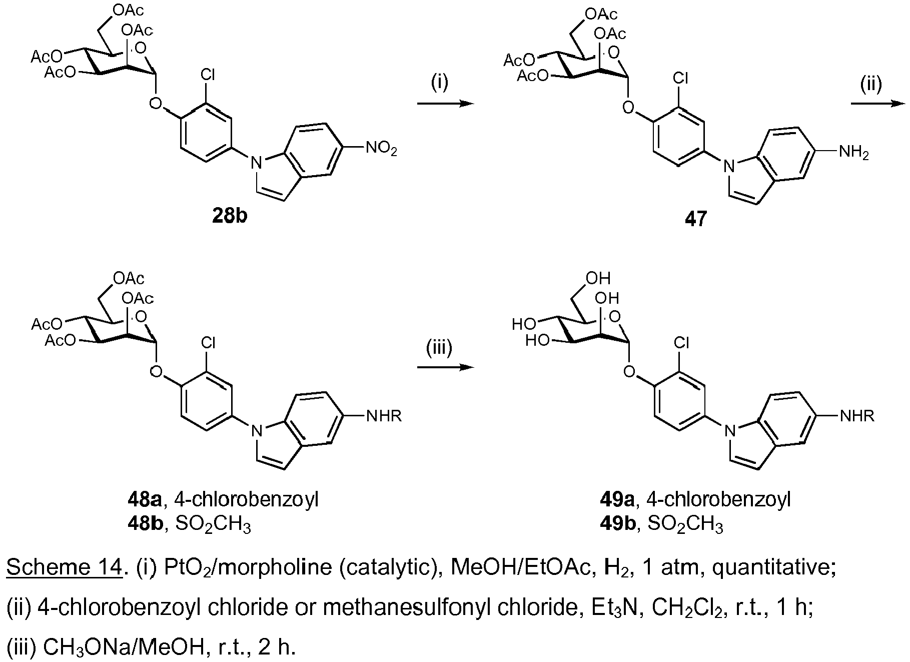

### Example 67: 4-(5-Aminoindol-1-yl)-2-chlorophenyl 2,3,4,6-tetra-O-acetl-α-D-mannopyranoside (47)

To a solution of **28b** (166 mg, 0.268 mmol) in methanol/ethyl acetate (1:1, 4 mL) PtO₂ (16 mg) and morpholine (12 µL, 0.136 mmol) are added. The reaction mixture is stirred at r.t. under 1 atm hydrogen for 1 h, then filtrated through a pad of celite and washed thoroughly with ethyl acetate. The filtrate is concentrated under vacuum to give compound **47** (158 mg, quant.) as off-white foam. It is used for next step without further purification.
¹H NMR (CDCl₃): δ 7.54 (d, *J* = 2.5 Hz, 1 H), 7.33-7.26 (m, 3H), 7.17 (d, *J* = 3.5 Hz, 1 H), 6.96 (d, *J* = 2.0 Hz, 1 H), 6.69 (dd, J = 8.5, 2.0 Hz, 1 H), 6.49 (d, J = 3.5 Hz, 1 H), 5.63 (dd, *J =* 10.0, 3.5 Hz, 1 H, H-3), 5.58 (d, *J* = 2.0 Hz, 1 H, H-1), 5.56 (dd, *J* = 3.5, 2.0 Hz, 1 H, H-2), 5.41 *(t, J =* 10.0 Hz, 1H, H-4), 4.30 (dd, *J* =12.0, 5.0 Hz, 1H, H-6a), 4.23 (ddd, J =10.0, 5.5, 2.0 Hz, 1 H, H-5), 4.13 (dd, J = 12.0, 2.5 Hz, 1 H, H-6b), 2.22 (s, 3H, OAc), 2.08 (s, 3H, OAc), 2.05 (s, 6H, OAc); ESI-MS calcd. for C₂₈H₂₉ClN₂O₁₀ [M+H]⁺: 589.16, found 589.15, calcd. for [M+Na]⁺: 611.14, found 611.23.

### Example 68: 2-Chloro-4-[5-(4-chlorobenzamido)indol-1-yl]phenyl 2,3,4,6-tetra-O-acetyl-α-D-mannopyranoside (48a)

To a solution of **47** (71 mg, 0.12 mmol) in dry CH₂Cl₂ (1.5 mL) triethylamine (33 µL, 0.24 mmol) and 4-chlorobenzoylchloride (17 µL, 0.13 mmol) are added at r.t. The reaction mixture is stirred at r.t. for 1 h. After quenching with methanol (100 µL) the solvent is removed under vacuum and the residue purified by chromatography (petrol ether/EtOAc 3:1 to 2:1) to afford compound **48a** (82 mg, 94%) as a white foam.
[α]_{D}²⁰ +52.91 (c = 0.565, CHCl₃). ¹H NMR (CDCl₃): δ 8.03 (s, 1 H), 7.92 (s, 1 H), 7.84 (d, *J* = 8.5 Hz, 2H), 7.55 (d, *J* = 2.0 Hz, 1 H), 7.44 (m, 3H), 7.36-7.30 (m 2H), 7.27 (d, *J* = 3.5 Hz, 1 H), 6.66 (d, *J =* 3.0 Hz, 1 H), 5.64 (dd, *J* = 10.0, 3.5 Hz, 1 H, H-3), 5.60 (d, *J* = 2.0 Hz, 1 H, H-1), 5.57 (dd, *J* = 3.5, 2.0 Hz, 1 H, H-2), 5.42 (t, *J* = 10.0 Hz, 1 H, H-4), 4.31 (dd, *J* =12.0, 5.5 Hz, 1 H, H-6a), 4.23 (ddd, *J* =10.0, 5.5, 2.0 Hz, 1 H, H-5), 4.14 (dd, *J* = 12.0, 2.0 Hz, 1 H, H-6b), 3.70 (brs, 1 H, N*H*CO), 2.22 (s, 3H, OAc), 2.08 (s, 3H, OAc), 2.06 (s, 3H, OAc), 2.05 (s, 3H, OAc); ¹³C NMR (125 MHz, CDCl₃): δ 170.44, 169.96, 169.79, 169.72, 164.69 (5 CO), 149.82, 137.85, 135.47, 133.58, 133.40, 131.01, 129.52, 128.95, 128.85, 128.66, 128.63, 128.48, 126.35, 125.36, 123.42, 117.84, 116.99, 113.44, 110.43, 104.30, 96.99 (C-1), 69.89 (C-5), 69.29 (C-2), 68.70 (C-3), 65.78 (C-4), 62.11 (C-6), 20.85, 20.68, 20.66 (4C, CH₃CO); ESI-MS calcd. for C₃₅H₃₂Cl₂N₂O₁₁ [M+H]⁺: 727.15, found 727.19.

### Example 69: 2-Chloro-4-[5-(4-chlorobenzamido)indol-1-yl]phenyl α-D-mannopyranoside (49a)

To a solution of **48a** (80 mg, 0.11 mmol) in dry methanol (2 mL) 0.5 M CH₃ONa/MeOH (22 µL) is added at r.t. After stirring for 2 h, the reaction mixture is neutralized with amberlyst-15, filtered, the filtrate concentrated and the residue purified by silica-gel column chromatography (CH₂Cl₂/MeOH 10:1) to afford **49a** (52 mg, 85%) as a white solid. ¹H NMR (CD₃OD+CDCl₃): δ 7.96 (s, 1 H), 7.92 (d, *J* = 8.5 Hz, 2H), 7.54 (d, *J* = 2.5 Hz, 1 H), 7.50-7.38 (m, 6H), 7.35 (d, *J* = 3.0 Hz, 1 H), 6.63 (d, *J* = 3.0 Hz, 1 H), 5.60 (s, 1 H, H-1), 4.16 (dd, J= 2.5, 1.5 Hz, 1H, H-2), 4.02 (dd, *J* = 9.5, 3.0 Hz, 1H, H-2), 3.83-3.76 (m, 3H, H-6a, H-4, H-6b), 3.70 (ddd, *J* = 9.5, 5.0, 2.0 Hz, 1 H, H-5); ¹³C NMR (CD₃OD+CDCl₃): δ 167.40 (CO), 151.53, 138.55, 135.69, 134.65, 134.56, 132.20, 130.45, 129.96, 129.61, 129.48, 126.75, 125.43, 118.81, 118.57, 115.09, 110.99, 104.70, 100.62 (C-1), 75.45 (C-5), 72.07 (C-3), 71.45 (C-2), 67.85 (C-4), 62.33 (C-6); ESI-MS calcd. for C₂₇H₂₄Cl₂N₂O₇ [M+H]⁺: 559.10, found. 559.12.

### Example 70: 2-Chloro-4-(5-methylsulfonamidoindol-1-yl)phenyl 2,3,4,6-tetra-O-acetl-α-D-mannopyranoside (48b)

Compound **48b** is prepared in a similar procedure as compound **48a,** Example 69. Starting from **47** (59 mg, 0.1 mmol), **48b** (55 mg, 82%) is obtained as a white solid.
¹H NMR (CDCl₃): δ 7.60 (d, *J* = 2.0 Hz, 1 H), 7.54 (s, 1 H), 7.43 (d, *J* = 8.5 Hz, 1 H), 7.32 (m, 2H, N*H*SO₂Me, ArH), 7.29 (d, *J* = 3.5 Hz, 1 H),7.13 (dd, *J* = 9.0, 2.0 Hz, 1 H), 6.66 (d, *J =* 3.0 Hz, 1 H), 6.50 (s, 1 H), 5.64 (dd, *J =* 10.0, 3.5 Hz, 1H, H-3), 5.61 (d, *J* = 2.0 Hz, 1 H, H-1), 5.56 (dd, *J* = 3.5, 2.0 Hz, 1H, H-2), 5.43 (t, *J =* 10.0 Hz, 1H, H-4), 4.31 (dd, *J* =12.0, 5.5 Hz, 1H, H-6a), 4.23 (ddd, *J* =10.0, 5.5, 2.0 Hz, 1H, H-5), 4.14 (dd, *J* = 12.0, 2.0 Hz, 1H, H-6b), 2.98 (s, 3H, CH₃SO₂NH), 2.22 (s, 3H, OAc), 2.09 (s, 3H, OAc), 2.06 (s, 3H, OAc), 2.05 (s, 3H, OAc); ¹³C NMR (CDCl₃): δ 170.45, 170.00, 169.83, 169.73 (4 CO), 150.09, 135.16, 134.39, 129.77, 129.44, 129.19, 126.54, 125.42, 123.59, 119.04, 117.81, 115.92, 111.08, 104.11, 96.98 (C-1), 69.91 (C-5), 69.28 (C-2), 68.67 (C-3), 65.76 (C-4), 62.10 (C-6), 38.82 (CH₃SO₂NH), 20.85, 20.69, 20.67 (4C, CH₃CO); ESI-MS calcd. for C₂₉H₃₁ClN₂O₁₂S [M+Na]⁺: 689.12, found 689.18.

### Example 71: 2-Chloro-4-(5-methylsulfonamidoindol-1-yl)phenyl α-D-mannopyranoside (49b)

Compound **49b** is prepared in a similar procedure as compound **49a** (Example 69). Starting from **48b** (55 mg, 0.082 mmol) **49b** (34 mg, 82%) is obtained as a white foam.
¹H NMR (CD₃OD): δ 7.57-7.54 (m, 3H), 7.44-7.41 (m, 3H), 7.13 (dd, *J* = 9.0, 2.0 Hz, 1H), 6.63 (dd, *J* = 3.0, 0.5 Hz, 1 H), 5.62 (d, *J* = 1.5 Hz, 1 H, H-1), 4.14 (dd, *J* = 3.5, 1.5 Hz, 1 H, H-2), 4.01 (dd, *J* = 9.5, 3.5 Hz, 1 H, H-2), 3.82 (dd, *J* = 12.0, 2.5 Hz, 1 H, H-6a), 3.78 (t, *J* = 10.0 Hz, 1H, H-4), 3.74 (dd, *J* = 12.0, 5.5 Hz, 1H, H-6b), 3.68 (ddd, *J* = 10.0, 5.5, 2.0 Hz, 1H, H-5), 2.90 (s, 3H, CH₃SO₂NH); ¹³C NMR (CD₃OD): δ 151.97, 135.90, 135.43, 132.01, 121.23, 130.36, 127.03, 125.67, 124.95, 119.76, 119.26, 116.29, 111.75, 104.73, 101.02 (C-1), 76.10 (C-5), 72.39 (C-3), 71.82 (C-2), 68.24 (C-4), 62.70 (C-6), 38.55 (CH₃SO₂NH); ESI-MS calcd. for C₂₁H₂₃ClN₂O₈S [M+Na]⁺: 521.08, found 521.12.

For the preparation of compounds of formula (**I**) wherein R¹ is 4-(pyridyl-2-carboxylate) or 4-(1*H*-1,2,3-triazolyl-4-carboxylate) (Examples 72-78) the following procedure is used:

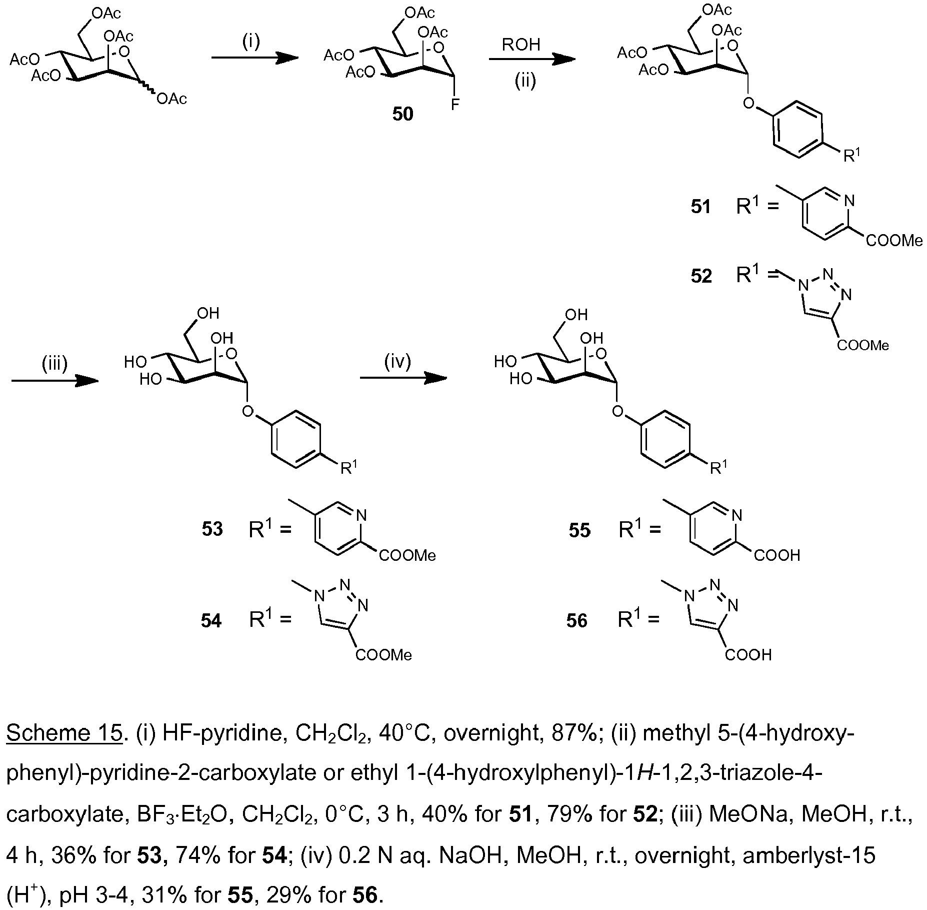

### Reference Example 72: 2,3,4,6-Tetra-O-acetyl-α-D-mannopyranosyl fluoride (50)

Compound **50** is prepared according to the literature *[*Carbohydr. Res. 1999, 317, 210-216].

### Reference Example 73: Methyl 5-[4-(2,3,4,6-tetra-O-acetyl-α-D-mannopyranosyloxy)-phenyl]-picolinate (51)

### Reference Example 74: Methyl 1-[4-(2,3,4,6-tetra-O-acetyl-α-D-mannopyranosyloxy)-phenyl]-1H-1,2,3-triazole-4-carboxylate (52)

### Reference Example 75: Methyl 5-[4-(α-D-mannopyranosyloxy)phenyl]-picolinate (53)

### Reference Example 76: Methyl 1-[4-(α-D-mannopyranosyloxy)phenyl]-1H-1,2,3-triazole-4-carboxylate (54)

### Reference Example 77: 5-[4-(α-D-Mannopyranosyloxy)phenyl]-picolinic acid (55)

### Reference Example 78: 1-[4-(α-D-Mannopyranosyloxy)phenyl]-1H-1,2,3-triazole-4-carboxylic acid (56)

For the preparation of compounds of formula (**I**) wherein R¹ is 4-(pyrimidyl-2-carboxylate), 4-(pyridyl-3-carboxylate) and 4-(pyrazinyl-2-carboxylate) (Examples 79-88) the following procedure is used:

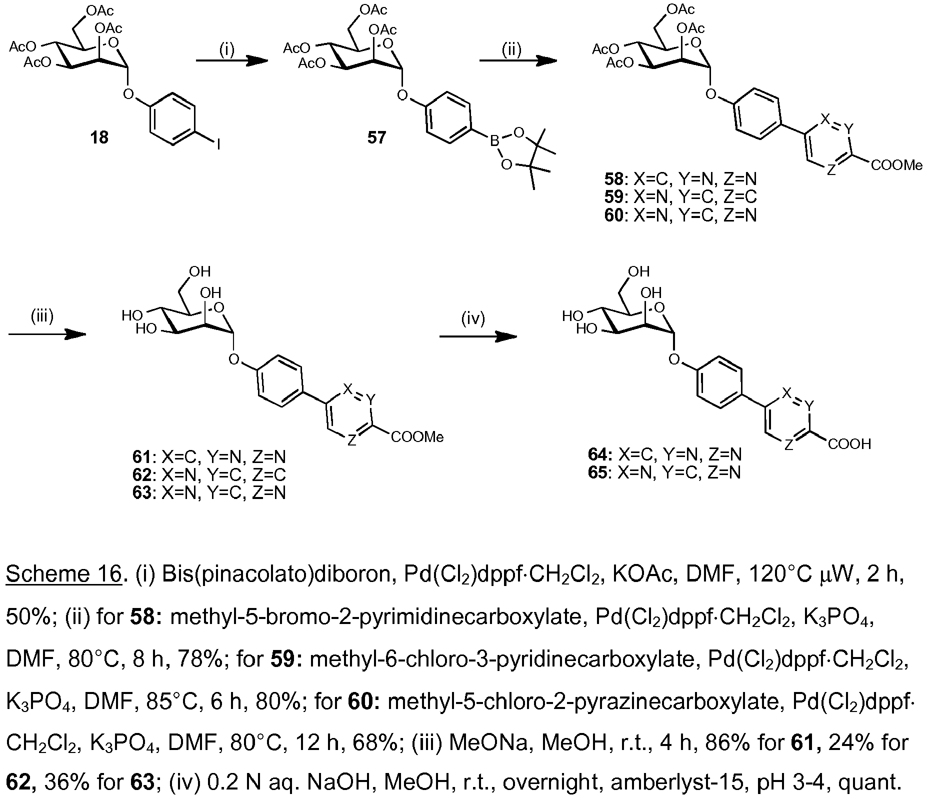

### Reference Example 79: 4-(4,4,5,5-Tetramethyl-1,3,2-dioxaborolan-2-yl)phenyl 2,3,4,6-tetra-O-acetyl-α-D-mannopyranoside (57)

### Reference Example 80: Methyl 5-[4-(2,3,4,6-tetra-O-acetyl-α-D-mannopyranosyloxy)-phenyl]-pyrimidine-2-carboxylate (58)

### Reference Example 81: Methyl 6-[4-(2,3,4,6-tetra-O-acetyl-α-D-mannopyranosyloxy)-phenyl]-nicotinate (59)

### Reference Example 82: Methyl 5-[4-(2,3,4,6-tetra-O-acetyl-α-D-mannopyranosyloxy)-phenyl]-pyrazine-2-carboxylate (60)

### Reference Example 83: Methyl 5-[4-(α-D-mannopyranosyloxy)phenyl]-pyrimidine-2-carboxylate (61)

### Reference Example 84: Methyl 6-[4-(α-D-mannopyranosyloxy)phenyl]-nicotinate (62)

### Reference Example 85: Methyl 5-[4-(α-D-mannopyranosyloxy)phenyl]-pyrazine-2-carboxylate (63)

### Reference Example 86: 5-[4-(α-D-Mannopyranosyloxy)phenyl]-pyrimidine-2-carboxylic acid (64)

### Reference Example 87: 5-[4-(α-D-Mannopyranosyloxy)phenyl]-pyrazine-2-carboxylic acid (65)

### For the preparation of compounds of formula (I) wherein R¹ is 4-(morpholine-4-carbonyl) phenyl, 3,5-difluoro-4-hydroxyphenyl, 4-(N-methylsulfamoyl)phenyl, 4-(methylsulfonyl)-phenyl or 4-cyanophenyl (Examples 88-96) the following procedure is used:

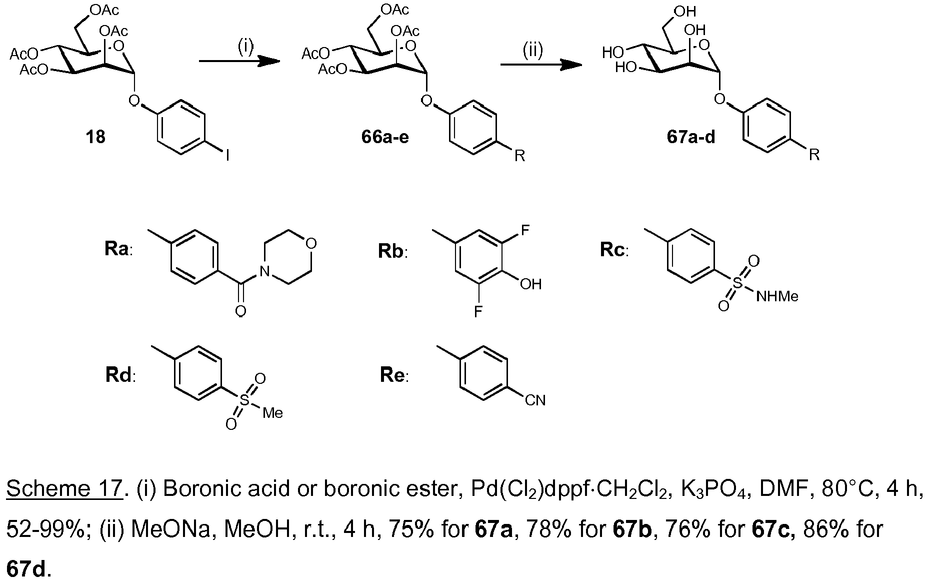

### Reference Example 88: [4'-(2,3,4,6-Tetra-O-acetyl-α-D-mannopyranosyloxy)-biphenyl-4-yl](morpholino)methanone (66a)

### Reference Example 89: 4'-(2,3,4,6-Tetra-O-acetyl-α-D-mannopyranosyloxy)-3,5-difluoro-biphenyl-4-ol (66b)

### Reference Example 90: 4'-(2,3,4,6-Tetra-O-acetyl-α-D-mannopyranosyloxy)-N-methyl-biphenyl-4-sulfonamide (66c)

### Reference Example 91: 4'-(2,3,4,6-Tetra-O-acetyl-α-D-mannopyranosyloxy)-4-(methylsulfonyl)-biphenyl (66d)

### Reference Example 92: 4'-(2,3,4,6-Tetra-O-acetyl-α-D-mannopyranosyloxy)-biphenyl-4-carbonitrile (66e)

### Reference Example 93: [4'-(α-D-Mannopyranosyloxy)-biphenyl-4-yl](morpholino)-methanone (67a)

### Reference Example 94: 3,5-Difluoro-4'-(-D-mannopyranosyloxy)-biphenyl-4-ol (67b)

### Reference Example 95: 4'-(α-D-Mannopyranosyloxy)-N-methyl-biphenyl-4-sulfonamide (67c)

### Reference Example 96: 4'-(α-D-Mannopyranosyloxy)-4-(methylsulfonyl)-biphenyl (67d)

For the preparation of compounds of formula (**I**) wherein R¹ is 4-(1*H*-tetrazol-5-yl)phenyl (Examples 97-98) the following procedure is used:

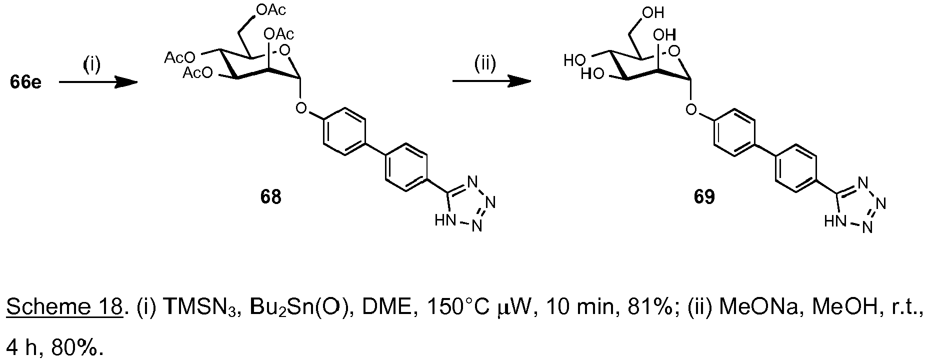

### Reference Example 97: 5-[4'-(2,3,4,6-Tetra-O-acetyl-α-D-mannopyranosyloxy)biphenyl-4-yl]-1H-tetrazole (68)

### Reference Example 98: 5-[4'-(α-D-Mannopyranosyloxy)biphenyl-4-yl]-1H-tetrazole (69)

For the preparation of compounds of formula (**I**) wherein R¹ is 4-(morpholino-4-carbonyl)-phenyl (Examples 99-100) the following procedure is used:

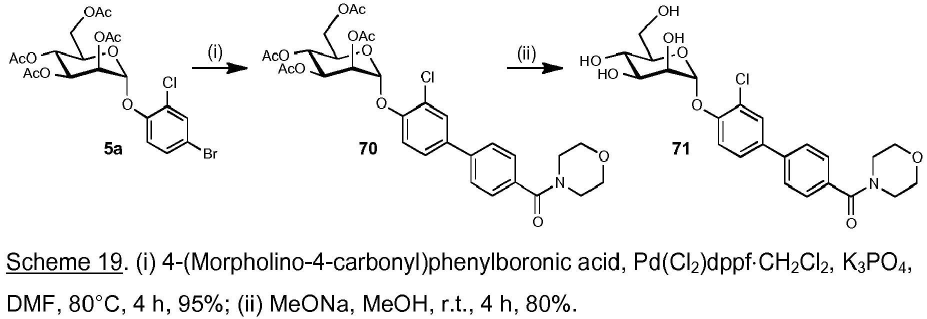

### Reference Example 99: [4'-(2,3,4,6-Tetra-O-acetyl-α-D-mannopyranosyloxy)-3'-chloro-biphenyl-4-yl](morpholino)methanone (70)

### Reference Example 100: [3'-Chloro-4'-(α-D-mannopyranosyloxy)-biphenyl-4-yl]-(morpholino)methanone (71)

### Example 101: Preliminary clotting assay

Overnight cultures of C. *albicans* (strain ATCC 60193) and *E. coli* (strain RS 218) are prepared by suspending colony material from agar plates in 10 ml trypticase soy broth (TSB) each and incubated for 16-18 h at 37°C, the culture of *E. coli* without shaking, but the culture of C. *albicans* with agitation on a horizontal shaker at 200 rpm. The cells of 2 ml of each culture are washed twice with 1 ml phosphate buffered saline (PBS) by centrifugation (10 min at 2500 x g), discarding the supernatant and re-suspension of the cell pellet in fresh PBS. After the last washing step, the optical density at 600 nm is measured and adjusted to 5.0 for C. *albicans* (corresponding to approx. 5 x 10⁷ viable cells/ml) and to 3.0 for *E. coli* (corresponding to approx. 5 x 10⁹ viable cells/ml). Defined solutions of test inhibitor substances and α-D-mannoside are prepared in PBS or, where necessary for reasons of solubility, in PBS containing 5% DMSO. Each test well is supplied with 33 µl PBS or the test solution of the desired concentration, and 33 µl of *E*. *coli* suspension is added and mixed by slightly tapping the plate. After 5 min 33 µl of C. *albicans* suspension is added to give a total volume of 100 µl per well. As a negative clumping control, one well is left without addition of *E*. *coli.* The plate is incubated for 20 min at 37°C on a horizontal shaker at 150 rpm and the cells are allowed to settle for 10 min at r.t. before the wells are inspected with the help of a magnifier for visual clumps. For each substance the minimal concentration that prevents clotting of C. *albicans* cells is noted. Each experiment is performed in triplicate. The following results were obtained:

**Table 1: Activity of FimH antagonists in the clotting assay**

| **Antagonist** | **Average conc. to prevent clotting** |
|---|---|
| **HM** (Heptyl α-D-mannopyranoside) | 42 µM |
| **29a** | 1.111 µM |
| **29b** | 0.556 µM |
| **32** | 5.556 µM |
| **34** | 0.833 µM |

### Example 102: Aggregometer assay

In analogy to N. Firon, I. Ofek, N. Sharon, Biochem. Biophys. Res. Comm. 1982, 105, 1426-1432, the following assay is conducted: *E.coli* strain UTI89 is statically incubated in Luria-Bertani broth for 24 h, washed twice and adjusted to an optical density at 600 nm (OD₆₀₀) of 4.0. The percentage of aggregation of UTI89 to guinea pig erythrocytes (GPE) is quantitatively measured using an APACT 4004 aggregometer (Endotell AG, Allschwil, Switzerland) at 740 nm, 37°C under stirring at 1000 rpm. For calibration, first the protein poor plasma (PPP) is measured using PBS 1% and set as 100% aggregation, followed by the protein rich plasma (PRP) using GPE at an OD₆₀₀ of 4.0, set as 0% aggregation. After calibration, the measurement is initiated using 250 µL GPE and 50 µL bacterial suspension. Following the aggregation phase of 600 s, 25 µL of the antagonists at different concentrations are added and disaggregation is monitored over 1400 s. The FimH-deleted mutant of *E.coli* UTI89 is used to proof FimH-specific GPE agglutination. For evaluation, the area under the disaggregation curve (AUC) is calculated and compared to the reference, heptyl α-D-mannopyranoside (see Table 2). The activities are reported as rel. IC₅₀ (rIC₅₀) with heptyl α-D-mannopyranoside as reference compound (rIC₅₀ = 1). The FimH-deleted mutant shows no agglutination of GPE.

### Example 103: Competitive binding assay

As described by D. Stokmaier, B. Ernst et al., Bioorg. Med. Chem. 2009, 17, 7254-7264, the following competitive binding assay is performed: Flat-bottom 96-well microtiter plates are coated with 100 µL/well of a 10 µg/mL solution of FimH-CRD in 20 mM HEPES, 150 mM NaCl and 1 mM CaCl₂, pH 7.4 (HBS-buffer) overnight at 4 °C. The coating solution is discarded and the wells are blocked with 150 µL/well of 3% BSA in HBS-buffer for 2 h at 4°C. After three washing steps with 150 µL/well of HBS-buffer, 50 µL/well of the test compound solution (0.2 nM to 250 µM in HBS-Buffer containing 5% DMSO) and 50 µL of a 0.5 µg/mL of streptavidin-peroxidase coupled trimannoside-PAA polymer are added. The plates are incubated for 3 h at rt and 350 rpm. The plates are then carefully washed four times with 150 µL/well HBS-buffer. After the addition of 100 µL/well of ABTS-substrate, the colorimetric reaction is allowed to develop for 4 min. The reaction is stopped by the addition of 2% aqueous oxalic acid and the optical density (OD) is measured at 415 nm on a microplate-reader. The IC₅₀ values of the compounds tested in duplicates are calculated with prism software (GraphPad Software, Inc, La Jolla, USA). The IC₅₀ defines the molar concentration of the test compound that reduces the maximal specific binding of trimannoside-PAA polymer to FimH-CRD by 50%. For heptyl α-D-mannopyranoside it is 73.05 ± 7.9 nM (average of five measurements). The relative IC₅₀ (rIC₅₀) is the ratio of the IC₅₀ of the test compound to the IC₅₀ of heptyl α-D-mannopyranoside.

**Table 2: rIC₅₀ for FimH antagonists in the aggregometer assay (Example 102) and the competitive binding assay (Example 103)**

| **Compound** | **Structure** | **MW** | **rIC₅₀ (measured in the aggregometer assay)** | **rIC₅₀ (measured in the polymer binding assay)** |
|---|---|---|---|---|
| **HM** (Heptyl α-D-mannopyranoside, reference) | 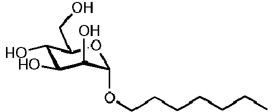 | 278.34 | 1 | 1 |
| *p*-Nitrophenyl α-D-mannopyranoside¹⁾ | 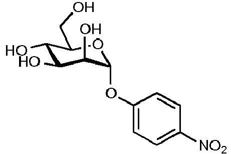 | 301.25 | - | 1.6 |
| **7a** | 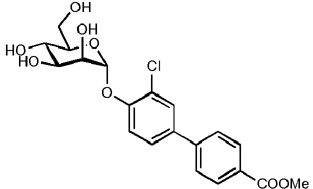 | 424.83 | 0.081 | 0.06 |
| **7b** | 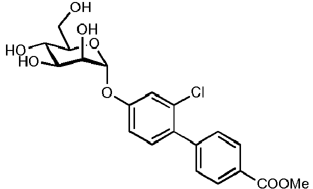 | 424.83 | 0.649 | 0.30 |
| **7c** | 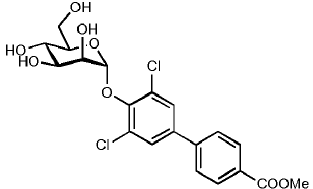 | 459.27 | 0.214 | 0.22 |
| **7d** | 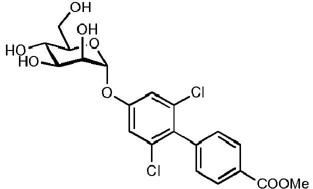 | 459.27 | - | 0.07 |
| **7e** | 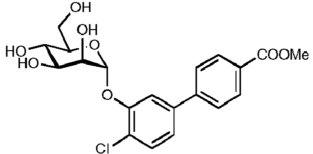 | 424.83 | 0.486 | 0.16 |
| **7f** | 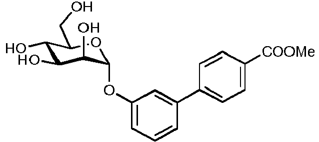 | 390.38 | 0.512 | 0.27 |
| **8a** | 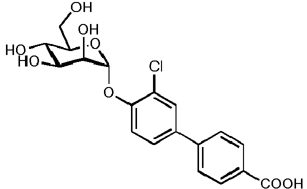 | 410.80 | 0.083 | 0.09 |
| **8b** | 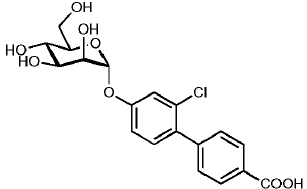 | 410.80 | 0.262 | 0.38 |
| **8f** | 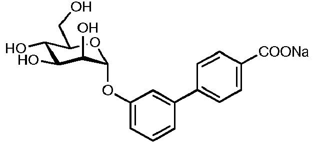 | 398.34 | 0.697 | 0.53 |
| **17** | 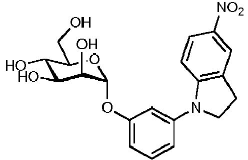 | 418.40 | 0.300 | - |
| **21** | 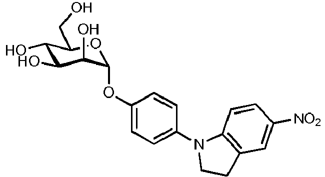 | 418.40 | 0.419 | 0.27 |
| **23** | 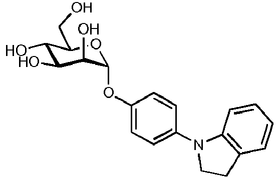 | 373.40 | - | 0.20 |
| **25** | 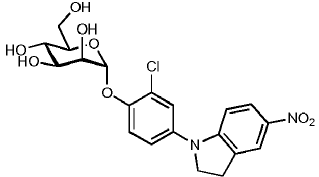 | 452.84 | - | 0.03 |
| **29a** | 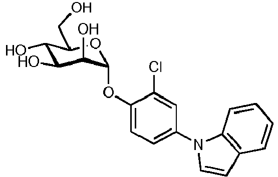 | 405.83 | - | 0.10 |
| **29b** | 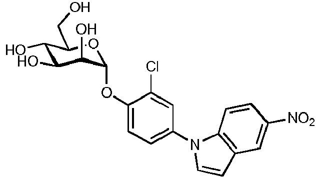 | 450.83 | - | 0.20 |
| **29c** | 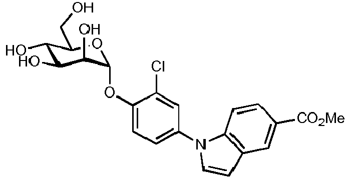 | 463.86 | - | 0.11 |
| **32** | 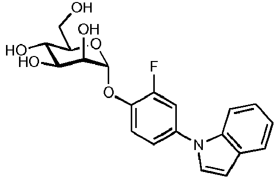 | 389.37 | - | 0.50 |
| **37** | 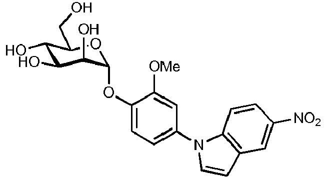 | 446.41 | - | 0.25 |
| **40** | 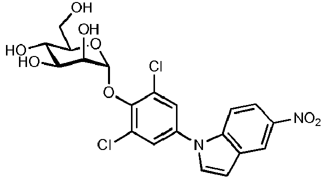 | 485.27 | - | 0.40 |
| **42b** | 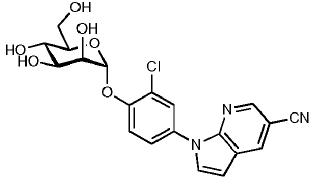 | 431.83 | - | 0.17 |

| | | | | |
|---|---|---|---|---|
| ¹⁾ Reference compound *p*-nitrophenyl α-D-mannopyranoside see WO 2005/089733 (Berglund et al.). | | | | |

### Example 104: Flow cytometry inhibition assay

The GFP tagged *E.coli* strain UTI89 is statically incubated in Luria-Bertani broth for 24 h, washed twice and adjusted to an optical density at 600 nm (OD₆₀₀) between 2.0 and 3.0. The human epithelial bladder carcinoma cell line 5637 was grown in RPMI 1640 medium, supplemented with 10% fetal calf serum (FCS), 100 U/ml penicillin and 100 µg/ml streptomycin at 37 °C, 5% CO₂ in 24-well plates. Cells were infected with 200 µl bacterial suspension (multiplicity of infection of 1:50 (cell:bacteria)), premixed with 25 µl of antagonists at different concentrations. To homogenize the infection, plates were centrifuged at room temperature for 3 min at 600 g. After an incubation of 1.5 h at 37 °C, infected cells were washed four times with RPMI 1640 medium and suspended in ice-cold PBS for 5-20 min. Samples were measured with a CyAn ADP flow cytometer (Becton Dickinson, San Jose, CA) and analyzed by gating on the eukaryotic cells based on forward (FSC) and side scatter (SSC). A total of 10⁴ cells were measured per sample. Data were acquired in a linear mode for the SSC and logarithmic mode for FSC and the green fluorescent channel FL1-H (e.g. GFP). The mean fluorescence intensity (MFI) of FL1-H was counted as a surrogate marker for the adherence of bacteria. Quantification of adhesion was evaluated with the FlowJo software 9.0.1 (Tree Star, Inc., Ashland, OR, USA). IC₅₀ values were determined by plotting the concentration of the antagonist in a logarithmic mode versus the MFI and by fitting the curve with the prism software (GraphPad, inhibition curve, variable slope). The relative IC₅₀ (rIC₅₀) is the ratio of the IC₅₀ of the test compound to the IC₅₀ of the reference compound **HM** (*n*-heptyl α-D-mannopyranoside.

**Table 3: IC₅₀ and rIC₅₀ for FimH antagonists in the flow cytometry inhibition assay**

| **Compound** | **Structure** | **Flow cytometry inhibition assay** | |
|---|---|---|---|
| | | **IC₅₀ [µM]** | **rIC₅₀** |
| **HM** (*n*-Heptyl α-D-mannopyranoside, reference) | 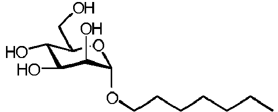 | 3.9 ± 1.6 | 1 |
| **7a** | 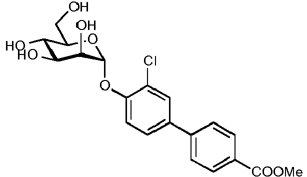 | 0.24 ± 0.043 | 0.06 |
| **8a** | 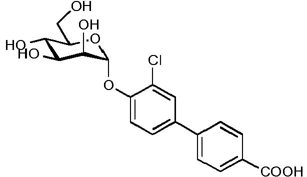 | 0.33 ± 0.05 | 0.085 |
| **8b** | 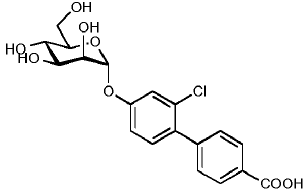 | 0.53 ± 0.06 | 0.135 |
| **8e** | 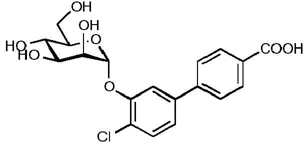 | 0.78 ± 0.16 | 0.2 |
| **8f** | 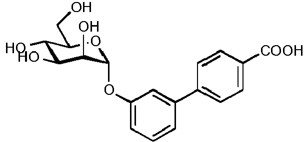 | 4.45 ± 1.9 | 1.14 |
| **29b** | 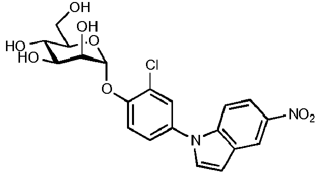 | 0.59 ± 0.045 | 0.15 |

### Example 105: Adherence test to urothelial cells

Human bladder uroethelia from surgical specimens are cultured for 6 passages and expanded *in vitro.* Uropathogenic *E. coli* strain HC14366 is used in an adherence test that corresponds essentially to the test published in K. Gupta, M.Y. Chou, A. Howell, C. Wobbe, R. Grady, A.E. Stapleton, J. Urol. 2007, 177, 2357-2360.

### Example 106: In vivo pharmacokinetic and disease model

The aim is the identification of FimH antagonists suitable for intravenous (i.v.) or preferably peroral (p.o.) applications. Before infection studies in a mouse disease model could be performed, the *in vivo* pharmacokinetic parameters (Cₘₐₓ, AUC) had to be determined, to ensure the antagonists availability in the target organ (bladder). Single-dose pharmacokinetic studies were performed by i.v. and p.o. application of the FimH antagonists at a concentration of 50 mg/kg followed by urine and plasma sampling. For i.v. application, the antagonists **(HM, 8f, 8a)** were diluted in 100 µL PBS and injected into the tail vein. For p.o. application, antagonist **HM** was diluted in 200 µL PBS and antagonists **8a** and **7a** were first dissolved in DMSO (20 x) and then slowly diluted to the final concentration (1 x) in 1% Tween-80/PBS to obtain a suspension. Antagonists were applied i.v. by injection into the tail vein and p.o. using a gavage followed by blood and urine sampling (10 µL) after 6 min, 30 min, 1 h, 2 h, 4 h, 6 h, 8 h and 24 h. Before analysis, proteins in blood and urine samples were precipitated using methanol and centrifuged for 11 min at 13'000 rpm. The supernatant was transferred into a 96-well plate and analyzed by LC-MS.

**Table 4. Determination of antagonist concentration in urine and plasma after a single i.v. and p.o. application of 50 mg/kg of antagonists 7a, 8a, 8f and HM.**

| Antagonist applied | Antagonist detected | Compartment | i.v. application AUC_{0-24 i.v.} (µg x h/mL) | p.o. application AUC₀₋₂₄ p.o. (µg x h/mL) |
|---|---|---|---|---|
| **HM** | **HM** | Plasma | 34.3 ± 33.3 | - |
| | | Urine | 2469.3 ± 636.4 | |
| **8f** | **8f** | Plasma | 19.3 ± 6.2 | - |
| | | Urine | 139.9 ± 118.8 | |
| **8a** | **8a** | Plasma | 20.8 ± 7.3 | n.d. 2.7 ± 3.2 |
| | | Urine | 209.6 ± 72.3 | |
| **7a** | **7a** | Plasma | - | 1.02 ± 0.32 |
| | | Urine | | 1.89 ± 0.37 |
| | **8a** | Plasma | - | 2.1 ± 0.61 |
| | | Urine | | 21.69 ± 3.88 |

The plasma concentration of orally applied **8a** was below the detection level and only a small portion was present in the urine. However, after the p.o. application of the prodrug **7a,** metabolite **8a** was predominantly detected due to fast metabolic hydrolysis of **7a.** However, minor amounts of **7a** are still traceable in plasma as well as urine; n.d. not detectable; (-) not tested.

### Example 107: Mouse model of urinary tract infection

Mice were infected as previously described (J.R. Johnson et al., Infect. Immun. 2005, 73, 965-971; W.J. Hopkins et al., Infec. Dis. 2003, 187, 418-23 and C.K. Garofalo et al., Infect. Immun. 2007, 75, 52-60). Female C3H/HeN mice, aged between 9 and 10 weeks were anesthetized with 1.1 vol% isoflurane/oxygen mixture and placed on their back. Anesthetized mice were inoculated transurethrally with the UPEC strain UTI89 by use of a 2 cm polyethylene catheter, which was placed on a syringe (Hamilton Gastight Syringe 50 µL). The catheter was gently inserted through the urethra until it reached the top of the bladder, followed by slow injection of 50 µL bacterial suspension at a concentration of approximately 10⁹ to 10¹⁰ CFU/mL.

For treatment studies, FimH antagonists were applied i.v. in 100 µL PBS into the tail vein or p.o. as a suspension by the help of a gavage, 10 minutes **(7a, 8a, 8f)** or 1 h before infection **(HM).**
3 h after the onset of infection, urine was collected by gentle pressure on the abdomen and then the mice were sacrificed with CO₂. Organs were removed aseptically and homogenized in 1 mL PBS. Serial dilutions of urine, bladder and kidneys were plated on Levine Eosin Methylene Blue Agar plates. CFU counts were determined after over night incubation at 37 °C and expressed as CFU/mL for the urine and as CFU/bladder and CFU/2 kidneys for the organs (Figure).

## Claims

1. A compound of formula (**I**) 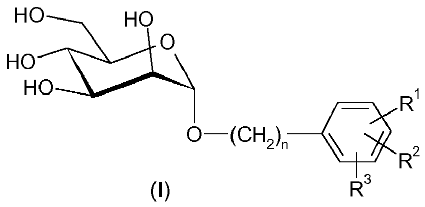 wherein
n is 0, 1 or 2;
R¹ is a residue of formula (B) or (C) 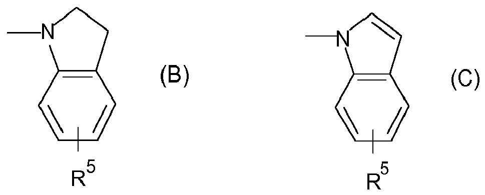 wherein R⁵ is hydrogen, trifluoromethyl, cylcopropyl, lower alkoxy, lower alkoxy-lower alkoxy, phenoxy, hydroxysulfonyl, aminosulfonyl, lower alkylsulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids, carboxy, lower alkoxycarbonyl, aminocarbonyl, hydroxylaminocarbonyl, tetrazolyl, nitro, cyano, or halo;
or of formula (D) 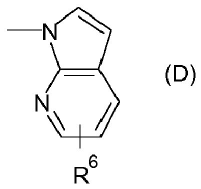 wherein R⁶ is hydrogen, trifluoromethyl, cylcopropyl, lower alkoxy, lower alkoxy-lower alkoxy, phenoxy, hydroxysulfonyl, aminosulfonyl, lower alkylsulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids, carboxy, lower alkoxycarbonyl, aminocarbonyl, hydroxylaminocarbonyl, tetrazolyl, nitro, cyano, or halo;
or of formula (E) 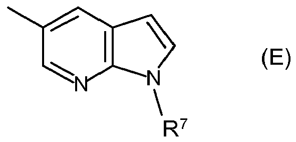 wherein R⁷ is hydrogen, lower alkyl, lower alkoxy-lower alkyl, lower alkylcarbonyl, optionally substituted phenylcarbonyl, or aminomethylcarbonyl substituted at the methyl group such that the resulting acyl group corresponds to one of the 20 naturally occurring standard amino acids;
R² and R³ are, independent of each other, hydrogen, lower alkyl, halo-lower alkyl, hydroxy-lower alkyl, lower alkoxy-lower alkyl, optionally substituted alkenyl, optionally substituted alkinyl, cycloalkyl, hydroxy, lower alkoxy, halo-lower alkoxy, lower alkoxy-lower alkoxy, phenoxy, hydroxysulfonyloxy; mercapto, alkylmercapto, hydroxysulfinyl, alkyl-sulfinyl, halo-lower alkylsulfinyl, hydroxysulfonyl, alkylsulfonyl, arylsulfonyl, heteroaryl-sulfonyl, aminosulfonyl, amino optionally substituted by one or two substitutents selected from lower alkyl, hydroxy-lower alkyl, lower alkoxy-lower alkyl; lower alkylcarbonylamino, alkoxycarbonylamino, benzoylamino, pyridinylcarbonylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; carboxy, lower alkylcarbonyl, benzoyl, pyridinecarbonyl, pyrimidinecarbonyl, lower alkoxycarbonyl, aminocarbonyl, wherein amino is unsubstituted or substituted by one hydroxy or amino group or one or two substitutents selected from lower alkyl, hydroxy-lower alkyl or lower alkoxy-lower alkyl; tetrazolyl, cyano, halogen, or nitro; or wherein two substituents in ortho-position to each other form a 5- or 6-membered heterocyclic ring containing one or two oxygen atoms and/or one or two nitrogen atoms, wherein the nitrogen atoms are optionally substituted by lower alkyl, lower alkoxy-lower alkyl or lower alkylcarbonyl; and prodrugs and salts thereof.

2. The compound according to claim 1 of formula (I) wherein n is 0 or 1; and prodrugs and salts thereof.

3. The compound according to claim 1 of formula (I) wherein n is 0; and prodrugs and salts thereof.

4. The compound according to anyone of claims 1 to 3 wherein R² and R³ are hydrogen, lower alkyl, halo-lower alkyl, cyclopropyl, lower alkoxy, lower alkoxy-lower alkoxy, phenoxy, hydroxysulfonyl, aminosulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids; carboxy, lower alkoxycarbonyl, aminocarbonyl, hydroxylaminocarbonyl, tetrazolyl, halo, cyano or nitro; and
prodrugs and salts thereof.

5. The compound according to anyone of claims 1 to 3 wherein R² and R³ are hydrogen, lower alkoxy, or halo; and prodrugs and salts thereof.

6. The compound according to anyone of claims 1 to 5 wherein R¹ is a residue of formula (B) or (C) 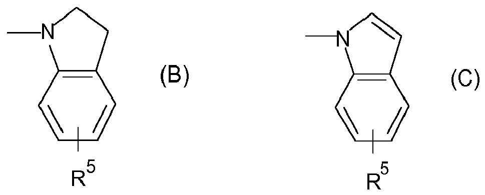 wherein R⁵ is hydrogen, trifluoromethyl, cylcopropyl, lower alkoxy, lower alkoxy-lower alkoxy, phenoxy, hydroxysulfonyl, aminosulfonyl, lower alkylsulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids, carboxy, lower alkoxycarbonyl, aminocarbonyl, hydroxylaminocarbonyl, tetrazolyl, nitro, cyano, or halo;
and prodrugs and salts thereof.

7. The compound according to anyone of claims 1 to 5 wherein R¹ is a residue of formula (D) 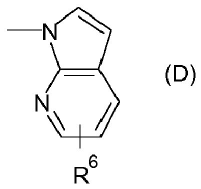 wherein R⁶ is hydrogen, trifluoromethyl, cylcopropyl, lower alkoxy, lower alkoxy-lower alkoxy, phenoxy, hydroxysulfonyl, aminosulfonyl, lower alkylsulfonyl, amino, lower alkylcarbonylamino, benzoylamino, carboxymethylamino or lower alkoxycarbonylmethylamino substituted at the methyl group such that the resulting substituent corresponds to one of the 20 naturally occurring standard amino acids, aminomethylcarbonylamino substituted at the methyl group such that the resulting acyl group corresponds to one of the 20 naturally occurring standard amino acids, carboxy, lower alkoxycarbonyl, aminocarbonyl, hydroxylaminocarbonyl, tetrazolyl, nitro, cyano, or halo;
and prodrugs and salts thereof.

8. The compound according to anyone of claims 1 to 5 wherein R¹ is a residue of formula (E) 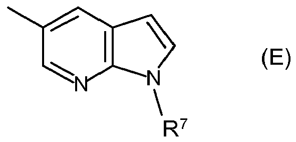 wherein R⁷ is hydrogen, lower alkyl, lower alkoxy-lower alkyl, lower alkylcarbonyl, optionally substituted phenylcarbonyl, or aminomethylcarbonyl substituted at the methyl group such that the resulting acyl group corresponds to one of the 20 naturally occurring standard amino acids;
and prodrugs and salts thereof.

9. The compound according to anyone of claims 1 to 5 of formula (I) wherein R¹ is a residue of formula (B) or (C) 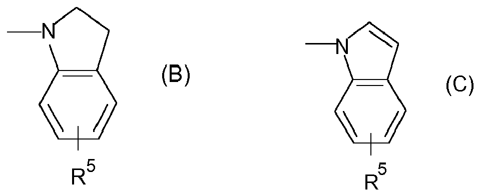 wherein R⁵ is hydrogen, trifluoromethyl, lower alkoxy, benzyloxy, amino, carboxy, lower alkoxycarbonyl, tetrazolyl, nitro, cyano, or halo;
or of formula (D) 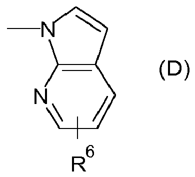 wherein R⁶ is hydrogen, trifluoromethyl, lower alkoxy, carboxy, lower alkoxycarbonyl, tetrazolyl, nitro, cyano, or halo;
or of formula (E) 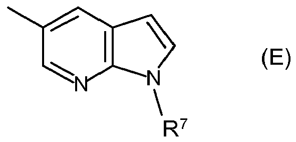 wherein R⁷ is hydrogen or lower alkyl; and
prodrugs and pharmaceutically acceptable salts thereof.

10. The prodrug of a compound according to anyone of claims 1 to 9 which is the tetraacetate.

11. A compound of according to anyone of claims 1 to 10, prodrugs and salts thereof, for use in the prevention and treatment of infectious diseases.

12. The compound according to claim 11, prodrugs and pharmaceutically acceptable salts thereof, for use in the prevention and treatment of urinary tract infections caused by virulent strains of *E.coli.*

13. A process for the manufacture of a compound according to anyone of claims 1 to 10, wherein a compound of formula (I), wherein the hydroxy functions of the α-D-mannopyranoside are protected and wherein R¹ is halogen, is condensed with a reagent replacing halogen by a residue of formula (B), (C), (D) or (E), the protective groups are removed, and, if so desired, an obtainable compound of formula (I) is converted into another compound of formula (I), compound of formula (I) is converted into a prodrug, a free compound of formula (I) is converted into a salt, an obtainable salt of a compound of formula (I) is converted into the free compound or another salt, and/or a mixture of isomeric compounds of formula (I) is separated into the individual isomers.

14. A pharmaceutical composition comprising a compound according to anyone of claims 1 to 10.
